(19) Europäisches Patentamt / European Patent Office / Office européen des brevets

(11) **EP 1 013 759 B1**

(12) **EUROPEAN PATENT SPECIFICATION**

(45) Date of publication and mention
of the grant of the patent:
**24.01.2007 Bulletin 2007/04**

(21) Application number: **98901095.4**

(22) Date of filing: **02.02.1998**

(51) Int Cl.:
*C12N 15/09* (2006.01)     *C12N 9/12* (2006.01)
*G06F 17/00* (2006.01)     *G06F 19/00* (2006.01)

(86) International application number:
**PCT/JP1998/000430**

(87) International publication number:
**WO 1998/033900 (06.08.1998 Gazette 1998/31)**

(54) **METHOD AND APPARATUS FOR PREDICTING PROTEIN FUNCTION SITE, METHOD FOR IMPROVING PROTEIN FUNCTION, AND FUNCTION-IMPROVED PROTEIN**

METHODE UND APPARAT ZUR VORHERSAGE VON FUNKTIONELLEN PROTEINDOMÄNEN, METHODE ZUR VERBESSERUNG DER PROTEINFUNKTION, SOWIE FUNKTIONELL VERBESSERTES PROTEIN

PROCEDE ET DISPOSITIF DE PREDICTION DU SITE DE FONCTION PROTEIQUE, PROCEDE D'AMELIORATION DE LA FONCTION PROTEIQUE ET PROTEINE A FONCTION AMELIOREE

(84) Designated Contracting States:
**CH DE FR GB LI NL**

(30) Priority: **31.01.1997  JP  1924897
31.01.1997  JP  1924997
02.12.1997  JP  33210097
30.01.1998  JP  1869998**

(43) Date of publication of application:
**28.06.2000  Bulletin 2000/26**

(60) Divisional application:
**05005854.4 / 1 564 288**

(73) Proprietor: **Japan Science and Technology
Agency
Kawaguchi-shi,
Saitama 332-0012 (JP)**

(72) Inventors:
• **DOI, Hirofumi
Funabashi-shi,
Chiba 273-0865 (JP)**
• **HIRAKI, Hideaki
Tokyo 113-0022 (JP)**
• **KANAI, Akio
Tsukuba-shi,
Ibaraki 305-0085 (JP)**

(74) Representative: **Owen, Deborah Jane et al
Frank B. Dehn & Co.
St Bride's House
10 Salisbury Square
London EC4Y 8JD (GB)**

(56) References cited:
**JP-A- 6 266 810          JP-A- 8 110 909**

• **DATABASE GENESEQ [Online] AC No: R96733, 15 October 1996 (1996-10-15) MATHUR EJ: "Wild-type P. furiosus DNA polymerase I" XP002164692**
• **ZHANG B ET AL: "FROM FOLD PREDICTIONS TO FUNCTION PREDICTIONS: AUTOMATION OF FUNCTIONAL SITE CONSERVATION ANALYSIS FOR FUNCTIONAL GENOME PREDICTIONS" PROTEIN SCIENCE, CAMBRIDGE UNIVERSITY PRESS, CAMBRIDGE, GB, vol. 8, March 1999 (1999-03), pages 1104-1115, XP002923809 ISSN: 0961-8368**
• **J. LIPID RES., Vol. 35, No. 12, (1994), R.B. WEINBERG, "Identification of Functional Domains in the Plasma Apolipoproteins by Analysis of Interspecies Sequence Variability", p. 2212-2222, XP002912939**
• **NUCLEIC ACIDS RES., Vol. 19, No. 24, (1991), E.J. MATHUR et al., "The DNA Polymerase Gene from the Hyperthermophilic Marine Archaebacterium, Pyrococcus Furiosus, Shows Sequence Homology with alpha-Like DNA Polymerases", p. 6952, XP002912940**

- NUCLEIC ACIDS RES., Vol. 20, No. 7, (1992), P. FORTERRE, "The DNA Polymerase from the Archaebacterium Pyrococcus Furiosus does not Testify for a Specific Relationship Between Archaebacteria and Eucaryotes", p. 1811, XP002912941

- NUCLEIC ACIDS RES., Vol. 21, No. 2, (1993), T. UEMORI et al., "Organization and Nucleotide Sequence of the DNA Polymerase Gene from the Archaeon Pyrococcus Furiosus", p. 259-265, XP002912942

**Description**

TECHNICAL FIELD

The present invention relates to a method for predicting a functional site of a protein, a system for predicting the function thereof, and a method for modifying the function of a protein and a function-modified protein. More specifically, the present invention relates to prediction of a functional site of a protein with no information on function obtained by genome analysis or cDNA analysis, and prediction of a novel function or a novel functional site of a protein with a known function. The present invention further ralates to prediction of a site on a protein to be modified for improving the function of the protein, and a protein with a modified function based on the prediction.

BACKGROUND ART

Following the progress of genome analysis and cDNA analysis of various organisms including pathogenic microorganisms, the number of novel genes whose functions are unknown is rapidly increasing, together with the number of proteins encoded by the genes. So far, the analysis of the nucleotide sequence of the whole genome of a microorganism, for example *Mycoplasma genitalium* (Fraser et al., Science 270, 397-403, 1995), *Haemophilus influenzae* (Fleischman et al., Science 269, 496-512, 1995), and *Methanococcus jannaschii* (Bult et al., Science 273, 1058-1073, 1996), has been completed, so that numerous novel proteins predicted from the genome sequence have been discovered. For humans and mice, the cDNA analysis is under way in combination with the genome analysis, which brings about the discovery of a great number of novel proteins.

In such circumstance, prediction of function of a protein with no information on function or a functional site has been a significant issue. If not only a novel protein but also a novel function or a novel functional site of a protein with a known function is discovered, whether or not these proteins are worth industrial or clinical application is possibly determined. Furthermore, such prediction of function possibly enables to prepare a modified protein with a further improved function.

Whether or not a protein encoded by a gene elucidated by genome analysis or cDNA analysis is novel or has a known function has been determined conventionally by searching the homology through protein databases such as Swiss-Prot. So as to predict a functional site, additionally, functionally identical proteins derived from various organisms are extracted from a protein database and are then subjected to alignment, to identify a region conserved in common to them and predict the conserved region as a functional site.

However, disadvantageously, such alignment method cannot be used if a protein obtained by genome analysis or cDNA analysis is an absolutely novel protein. Even if the protein was homologous with known proteins in a protein database, the conserved region occupies most of the amino acid sequence of the protein in case that the protein is homologous to proteins derived from closely related organisms, so that it is impossible to predict the functional site. As to modification of protein, generally, the function of a protein is potentially deteriorated irrespective of the fact that the function is known or unknown once the conserved region is modified, even if the functional site is predicted by alignment. Accordingly, the amino-acid residues outside the conserved region should be modified to improve the function. In other words, it is required to find a novel functional site in such protein to be modified. Using the conventional alignment method, disadvantageously, a novel functional site cannot be discovered or which amino-acid residue should be modified cannot be predicted.

Taking account of such circumstance, the present invention has been carried out. It is an object of the present invention to provide a novel method for predicting a functional site of a protein with no information on function obtained by genome analysis or cDNA analysis.

It is another object of the present invention to provide a system for predicting function of a protein.

Furthermore, it is an object of the present invention to provide a method for predicting a novel functional site of a protein with an unknown function or with a known function and subjecting the functional site to mutation to prepare a modified protein.

Still furthermore, it is an object of the present invention to provide a protein with a function modified by the method described above.

DISCLOSURE OF INVENTION

A first invention of the present application is a method for predicting a functional site of a protein derived from the entire putative proteins of an organism "*a*" of which genome data or cDNA data is known, which method comprises the steps of:

    (1) calculating the frequency of occurrence of each amino acid and the frequency of occurrence of individual oligopeptides produced by permutations of twenty amino acids in the amino acid sequences of the entire proteins of the organism "*a*", and determining the smallest length (n) of oligopeptides satisfying the following criteria;
among oligopeptides of length (n), the number of oligopeptides which occur once in the entire proteins is smaller than the number of oligopeptides which occur twice in the entire proteins; among oligopeptides of length (n+1), the number of oligopeptides which occur once in the entire proteins is larger than the number of oligopeptides which occur twice in the entire proteins,

(2) calculating in the entire proteins of the organism "*a*", the frequency of occurrence of the following Aj-oligopeptide of length (n+1), which is a part of the amino acid sequence of length (L) of the protein as a subject for predicting a functional site and contains an amino-acid residue Aj (n+1 $\leq$ j $\leq$ L-1) at the j-th position from the N-terminus of the amino acid sequence of the protein;

Aj-oligopeptide: aj1aj2 .... Aji .... ajnaj(n+1) (wherein, 1$\leq$i$\leq$n+1; Aj=Aji; and Aj is the i-th residue of the oligopeptide), and calculating in the entire proteins of the organism "*a*", the frequency of occurrence of the following Xi-oligopeptide of length (n+1);

Xi-oligopeptide: aj1aj2 .... Xi .... ajnaj(n+1) (wherein, the i-th residue Xi is any amino acid),

(3) calculating ratio Yji of the frequency of occurrence of the Aj-oligopeptide to that of the Xi-oligopeptide,

(4) calculating mean Yj of the Yji;

$$Yj = \sum_{i=1}^{n+1} Yji/(n+1),$$

(5) calculating functional value Zj of Yj;

$$Zj = f(Yj)$$

(wherein, function f is a monotonously decreasing function or a monotonously increasing function),
and defining the Zj value as a representative value of the function of the j-th amino-acid residue Aj of the amino acid sequence of length (L), and

(6) repeating the steps (2) to (5) sequentially and determining the Zj value of each Aj of all the amino-acid residues at the positions between n+1$\leq$j$\leq$L-n in the amino acid sequence of length (L), thereby predicting the degree of the involvement of each amino-acid residue in the function of the protein by using the dimension of the Zj value as an indicator.

A second invention is a method for predicting a functional site of a protein derived from the entire putative proteins of an organism "*a*" of which genome data or cDNA data is known, which method comprises the steps of:

(1) calculating the frequency of occurrence of each amino acid and the frequency of occurrence of individual oligopeptides produced by permutations of twenty amino acids in the amino acid sequences of the entire proteins of the organism "*a*",

(2) as to a protein of the organism "*a*",

(2') calculating in the entire proteins of the organism "*a*", the frequency of occurrence of the following Aj-oligopeptide of given length of (n) (1$\leq$n$\leq$M, provided that M is the the smallest length of oligopeptides satisfying the criterion that all the oligopeptides of length M are at frequency 1 of the occurrence), which Aj-oligopeptide is a part of the amino acid sequence of length (L) of the protein and contains an amino-acid residue Aj (n$\leq$j$\leq$L-n+1) at the j-th position from the N-terminus of the amino acid sequence of the protein;

Aj-oligopeptide: aj1aj2....aji....ajn (wherein, 1$\leq$i$\leq$n+1; Aj=aji, and Aj is the i-th residue of the oligopeptide), and calculating in the entire proteins of the organism "*a*", the frequency of occurrence of the following Xi-oligopeptide of length (n) corresponding to the length of Aj-oligopeptide;

Xi-oligopeptide: aj1aj2....Xi....ajn (wherein, the i-th residue Xi is any amino acid),

(3) calculating ratio Yji of the frequency of occurrence of the Aj-oligopeptide to that of the Xi-oligopeptide,

(4) calculating mean Y(j,n) of the Yji;

$$Y(j,n) = \sum_{i=1}^{n} Yji/n,$$

(5) calculating functional value Z(j,n) of Y(j,n);

$$Z(j,n) = -\log(Y(j,n)),$$

(6) repeating the steps (2') to (5) sequentially and determining the Z(j,n) value of each amino-acid residue Aj at the j-th position (n≦j≦L-n+1) in the amino acid sequence of length (L),

(7) sequentially repeating the steps (2) to (6) for the entire proteins of the organism "*a*", thereby determining the distribution of the Z(j,n) value of each amino-acid residue in the entire proteins, and the Z(j,n) values are classified into twenty according to the twenty amino acids, and then calculating mean Av(Aa) of the Z(j,n) values for each amino acid Aa and the standard deviation Sd (Aa) of the distribution thereof, on the basis of the distribution, to calculate a function g to the j-th amino-acid residue Aj of a protein for normalizing the difference in distribution due to the species of amino-acid residues;

$$g = (Z(j,n), Aj) = [Z(j,n) - Av(Aa)]/Sd(Aa)$$

(wherein, Aj = Aa),

(8) calculating a value D(j,n) of the function g of each Aj of all the amino-acid residues at the j-th position (n≦j≦ L-n+1) of a protein in the entire proteins as recovered in the step (7) ;

$$D(j,n) = g(Z(j,n), Aj),$$

and

(9) defining the representative value of the function of the j-th amino-acid residue in the amino acid sequence of length (L) as a functional value Wj of the Z(j,n) and D(j,n);

Wj=h(Z(j,1),Z(j,2),..,Z(j,M),D(j,1),D(j,2),..,D(j,M)) thereby predicting the degree of the involvement of each amino-acid residue in the function of the protein by using the dimension of the Wj value as an indicator.

A third invention is a system for automatically conducting the method according to claim 1, at least comprising the following units (a) to (g);

(a) an outer memory unit memorizing the amino acid sequence data of the entire putative proteins of organism "*a*" of which genome data or cDNA data is known, as well as an existing protein data base,

(b) a calculation/memory unit, composed of CPU calculating the frequency of occurrence of each amino acid and the frequency of occurrence of individual oligopeptides produced by permutations of twenty amino acids, in the amino acid sequences of the entire proteins of the organism "*a*", and a memory unit having the memory of the calculation results,

(c) a calculation/memory unit, composed of CPU calculating the smallest length (n) of oligopeptides satisfying the following criteria among the individual oligopeptides of which the frequencies of the occurrences being memorized in the unit (b) ;

among oligopeptides of length (n), the number of oligopeptides which occur once in the entire proteins is smaller than the number of oligopeptides which occur twice in the entire proteins; among oligopeptides of length (n+1), the number of oligopeptides which occur once in the entire proteins is larger than the number of oligopeptides which occur twice in the entire proteins,

and a memory unit having the memory of the (n),

(d) a calculation/memory unit, composed of CPU calculating in the entire proteins of the organism "*a*", the frequency of occurrence of the following Aj-oligopeptide of length (n+1), which is a part of the amino acid sequence of length of (L) of the protein as a subject for predicting a functional site and contains an amino-acid residue Aj (n+1 $\leq$ j $\leq$ L-n) at the j-th position from the N-terminus of the amino acid sequence of the protein;

Aj-oligopeptide: aj1aj2....Aji....ajnaj(n+1) (wherein, 1 $\leq$ i $\leq$ n+1; Aj = Aji; and Aj is the i-th residue of the oligopeptide), and calculating in the entire proteins of the organism "*a*", the frequency of occurrence of the following Xi-oligopeptide of length (n+1);

Xi-oligopeptide: ajlaj2....Xji....ajnaj(n+1) (wherin, the i-th residue Xji is any amino acid), and a memory unit having the memory of the calculation results,

(e) a calculation/memory unit, composed of CPU calculating ratio Yji of the frequency of occurrence of the Aj-oligopeptide to that of the Xi-oligopeptide, and a memory unit having the memory of Yji,

(f) a calculation/memory unit, composed of CPU calculating mean Yj of the Yji;

$$Yj = \sum_{i=1}^{n+1} Yji/(n+1),$$

and a memory unit having the memory of Yj, and

(g) a calculation/memory unit, composed of CPU calculating functional value Zj of Yj;

$$Zj = f(Yj)$$

(wherein, function f is a monotonously decreasing function or a monotonously increasing function), and a memory unit having the memory of Zj.

A fourth invention is a system for automatically conducting the method according to claim 3, at least comprising the following units (a) to (i);

(a) an outer memory unit memorizing the amino acid sequence data of the entire putative proteins of organism "*a*" of which genome data or cDNA data is known, as well as an existing protein data base,

(b) a calculation/memory unit, composed of CPU calculating the frequency of occurrence of each amino acid and the frequency of occurrence of individual oligopeptides produced by permutations of twenty amino acids in the amino acid sequences of the entire proteins of the organism "*a*", and a memory unit having the memory of the calculation results,

(c) a calculation/memory unit, composed of CPU calculating in the entire proteins of the organism "*a*", the frequency of occurrence of the following Aj-oligopeptide of given length of (n) (1 $\leq$ n $\leq$ M, provided that M is the smallest length of oligopeptides satisfying the criterion that all the oligopeptides of length M are at frequency 1 of the occurrence), which Aj-oligopeptide is a part of the amino acid sequence of length (L) of a given protein of the organism "*a*" and contains an amino-acid residue Aj (n $\leq$ j $\leq$ L-n+1) at the j-th position from N-terminus of the amino acis sequene of the protein;

Aj-oligopeptide: aj1aj2....aji....ajn (wherein, 1 $\leq$ i $\leq$ n+1; Aj=aji, and Aj is the i-th residue of the oligopeptide), and calculating in the entire proteins of the organism "*a*", the frequency of occurrence of the following Xi-oligopeptide of length (n) corresponding to the length of Aj -oligopeptide;

Xi-oligopeptide: aj1aj2....Xji....ajn (wherein, the i-th residue Xji is any amino acid), and a memory unit memorizing the calculation results,

(d) a calculation/memory unit, composed of CPU calculating ratio Yji of the frequency of occurrence of the Aj-oligopeptide to that of the Xi-oligopeptide, and a memory unit having the memory of the Yji,

(e) a calculation/memory unit, composed of CPU calculating mean Y(j,n) of the Yji;

$$Y(j,n) = \sum_{i=1}^{n} Yji/n,$$

and a memory unit having the memory of Y(j,n),

(f) a calculation/memory unit, composed of CPU calculating functional value Z(j,n) of Y(j,n);

$$Z(j,n) = -log(Y(j,n)),$$

and a memory unit having the memory of Z(j,n),

(g) a calculation/memory unit, composed of CPU calculating the Z(j,n) value of each amino-acid residue in the amino acid sequences of the entire proteins of the organism "*a*", calculating the distribution of the Z(j,n) value of each amino-acid residue in the entire proteins, and the Z(j,n) values are classified into twenty according to the twenty amino acids, and then caclulating mean Av(Aa) of the Z(j,n) values for each amino acid Aa and the standard deviation Sd (Aa) of the distribution thereof, on the basis of the distribution, to determine function g for normalizing the difference in distribution due to the species of amino-acid residues;

$$g = (Z(j,n), Aj) = [Z(j,n) - Av(Aa)]/Sd(Aa)$$

(wherein, Aj = Aa)
and a memory unit having the memory of g,

(h) a calculation/memory unit, composed of CPU calculating value D (j,n) of function g memorized in the unit (g) concerning each of all the amino-acid residues Aj at the j-th position (n≦j≦L-n+1) in the amino acid sequence of length (L) ;

$$D(j,n) = g(Z(j,n), Aj)$$

and a memory unit having the memory of the D(j, n) value, and

(i) a calculation/memory unit, composed of a calculation unit calculating an appropriate functional value Wj of the Z (j,n) and D(j,n) of each amino-acid residue in the amino acid sequence;

$$Wj=h(Z(j,1),Z(j,2),..,Z(j,M),D(j,1),D(j,2),..,D(j,M))$$

and a memory unit having the memory of the Wj value.

A fifth invention is a method for modifying the known function of protein "*A*" derived from the entire proteins of organism "*a*" of which genome data or cDNA data has been known, which method comprises the steps of:

(1) extracting a protein closely related to the protein "*A*" from an existing protein data base and subjecting the proteins to alignment,

(2) calculating the frequency of occurrence of each amino acid and the frequency of occurrence of individual oligopeptides produced by permutations of twenty amino acids in the amino acid sequences of the entire proteins of the organism "*a*", and determing the smallest length (n) of oligopeptides satisfying the following criteria;

among oligopeptides of length (n), the number of oligopeptides which occur once in the entire proteins is smaller than the number of oligopeptides which occur twice in the entire proteins; among oligopeptides of length (n+1), the number of oligopeptides which occur once in the entire proteins is larger than the number of oligopeptides which occur twice in the entire proteins,

(3) calculating in the entire proteins of the organism "*a*", the frequency of occurrence of the following Aj-oligopeptide of length (n+1), which is a part of the amino acid sequence of length (L) of the protein "*A*" and contains an amino-acid residue Aj (n+1≦j≦L-n) at the j-th position from the N-terminus of the amino acid sequence of the protein;

Aj-oligopeptide: aj1aj2....Aji....ajnaj(n+1) (wherein, 1≦i≦n+1; Aj=Aji; and Aj is the i-th residue of the oligopeptide), and calculating in the entire proteins of the organism "*a*", the frequency of occurrence of the following Xi-oligopeptide of length (n+1);

Xi-oligopeptide: aj1aj2....Xji....ajnaj(n+1) (wherein, the residue Xji is any amino acid),

(4) calculating ratio Yji of the frequency of occurrence of the Aj-oligopeptide to that of the Xi-oligopeptide,

(5) calculating mean Yj of the Yji;

$$Yj = \sum_{i=1}^{n+1} Yji/(n+1),$$

(6) calculating functional value Zj of Yj;

$$Zj = f(Yj)$$

(wherein, function f is a monotonously decreasing function or a monotonously increasing function),
and defining the Zj value as a representative value of the function of the j-th amino-acid residue of the amino acid sequence of length (L) of the protein "*A*",
(7) sequentially repeating the steps (3) to (6) and determining the Zj value of each of all the amino-acid residues at position between n+1 ≦j≦L-n in the amino acid sequence of length (L),
(8) selecting at least one amino-acid residue to be subjected to mutation on the basis of the alignment data carried out in the step (1) from the amino acid sequence of length (L) of the protein "*A*", sequentially repeating the steps (3) to (6) for variant amino-acid residues in various mutated amino acid sequences where the selected amino-acid residue has been mutated into another amino-acid residue, to determine the Zj value of the variant amino-acid residues,
(9) selecting a mutated amino acid sequence wherein the Zj value of the variant amino-acid residue as determined in the step (8) is larger or smaller than the Zj value of the wild type amino-acid residue as determined in the step (7), and
(10) preparing a modified gene encoding the modified amino acid sequence from the protein "*A*" gene, and producing the modified protein as the expression product of the gene.

A sixth invention is a method for modifying the function of protein "*B*" derived from an organism "*b*" of which genome data or cDNA data has been unknown, which method comprises the steps of:

(1) extracting protein "*A*" most closely related to protein "*B*" from the entire proteins of organism "*a*" of which genome data or cDNA data being known and subjecting the protein to alignment, or extracting a protein closely related to protein "*B*" from an existing protein data base to subject the protein to alignment,
(2) calculating in the amino acid sequences of the entire proteins of the organism "*a*", the frequency of occurrence of each amino acid and the frequency of occurrence of individual oligopeptides produced by permutations of twenty amino acids, and determining the smallest length (n) of oligopeptides satisfying the following criteria;
among oligopeptides of length (n), the number of oligopeptides which occur once in the entire proteins is smaller than the number of oligopeptides which occur twice in the entire proteins; among oligopeptides of length (n+1), the number of oligopeptides which occur once in the entire proteins is larger than the number of oligopeptides which occur twice in the entire proteins,
(3) calculating in the entire proteins of the organism "*a*", the frequency of occurrence of the following Aj-oligopeptide of length (n+1), which is a part of the amino acid sequence of length of (L) of the protein "*A*" and contains an amino-acid residue Aj (n+1≦j≦L-n) at the j-th position from the N-terminus of the amino acid sequence of the protein;
Ai-oligopeptide: aj1aj2....Aji....ajnaj (n+1) (wherein, 1≦i≦n+1; Aj=Aji; and Aj is the i-th residue of the oligopeptide), and calculating in the entire proteins of the organism "*a*", the frequency of occurrence of the following Xi-oligopeptide of length (n+1);
Xi-oligopeptide: aj1aj2....Xji....ajnaj(n+1) (wherein, the i-th residue Xji is any amino acid),
(4) calculating ratio Yji of the frequency of occurrence of the Aj-oligopeptide to that of the Xi-oligopeptide,
(5) calculating mean Yj of the Yji;

$$Yj = \sum_{i=1}^{n+1} Yji/(n+1),$$

(6) calculating functional value Zj of Yj;

$$Zj = f(Yj)$$

(wherein, function f is a monotonously decreasing function or a monotonously increasing function),

and defining the Zj value as a representative value of the function of the j-th amino-acid residue Aj of the amino acid sequence of length (L) of the protein "A",

(7) sequentially repeating the steps (3) to (6) and determining the Zj value of each of all the amino-acid residues at position between $n+1 \leqq j \leqq L-n$ in the amino acid sequence of length (L),

(8) selecting at least one amino-acid residue to be subjected to mutation on the basis of the alignment data carried out in the step (1) from the amino acid sequence of length (L) of the protein "A", sequentially repeating the steps (3) to (6) for variant amino-acid residues in various mutated amino acid sequences where the selected amino-acid residue has been mutated into another amino-acid residues, to determine the Zj value of the variant amino-acid residues,

(9) selecting the mutation position and the mutated amino-acid residue wherein the Zj value of the variant amino-acid residue as determined in the step (8) is larger or smaller than the Zj value of the wild type amino-acid residue as determined in the step (7), and

(10) preparing a modified gene encoding the modified amino acid sequence having the mutated amino-acid residue at the position from the protein "B" gene, and producing the modified protein as the expression product of the gene.

A seventh invention is a method for modifying the known function of protein "A" derived from the entire proteins of organism "a" of which genome data or cDNA data has been known, which method comprises the steps of:

(1) extracting proteins closely related to the protein "A" from an existing protein data base and subjecting the proteins to alignment,

(2) calculating the frequency of occurrence of each amino acid and the frequency of occurrence of individual oligopeptides produced by permutations of twenty amino acids, in the amino acid sequences of the entire proteins of the organism "a",

(3) as to a protein of the organism "a",

(3') calculating in the entire proteins of the organism "a", the frequency of occurrence of the following Aj-oligopeptide of given length of (n) ($1 \leqq n \leqq M$, provided that M is the smallest length of oligopeptides satisfying the criterion that all the oligopeptides of length M are at frequency 1 of the occurrence), which Aj-oligopeptide is a part of the amino acid sequence of the protein and contains an amino-acid residue Aj ($n \leqq j \leqq L-n+1$) at the j-th position from the N-terminus of the protein;

Aj-oligopeptide: aj1aj2....aji....ajn (wherein, $1 \leqq i \leqq n$; Aj=aji, and Aj is the i-th residue of the oligopeptide),

and calculating in the entire proteins of the organism "a", the frequency of occurrence of the following Xi-oligopeptide of length (n) corresponding to the length of Aj-oligopeptide;

Xi-oligopeptide: aj1aj2....Xji....ajn (wherein, the i-th residue Xji is any amino acid),

(4) calculating ratio Yji of the frequency of occurrence of the Aj-oligopeptide to that of the Xi-oligopeptide,

(5) calculating mean Y(j,n) of the Yji;

$$Y(j,n) = \sum_{i=1}^{n} Yji/n,$$

(6) calculating functional value Z(j,n) of Y(j,n);

$$Z(j,n) = -\log(Y(j,n)),$$

(7) repeating the steps (3') to (6) sequentially and determining the Z(j,n) value of each amino-acid residue Aj at position j ($n \leqq j \leqq L-n+1$) in the amino acid sequence of length (L),

(8) sequentially repeating the steps (3) to (7) for the entire proteins of the organism "a", thereby determining the distribution of the Z(j,n) value of each amino-acid residue in the entire proteins, and the Z(j,n) values are classified into twenty according to the twenty amino acids, and then calculating mean Av(Aa) of the Z(j,n) values for each amino acid Aa and the standard deviation Sd (Aa) of the distribution thereof, on the basis of the distribution, to determine function g to the j-th amino-acid residue Aj of a protein for normalizing the difference in distribution due to the species of amino-acid residues;

$$g = (Z(j,n), Aj) = [Z(j,n) - Av(Aa)]/Sd(Aa)$$

(wherein, Aj = Aa),
(9) calculating value D(j,n) of the function g of each Aj of all the amino-acid residues at the j-th position (n≦j≦L-n+1) of a protein in the entire proteins as recovered in the step (8) ;

$$D(j,n) = g(Z(j,n), Aj),$$

(10) defining the representative value of the function of the j-th amino-acid residue in the amino acid sequence of length (L) as functional value Wj of the Z(j,n) and D(j,n);

$$Wj=h(Z(j,1),Z(j,2),..,Z(j,M),D(j,1),D(j,2),..,D(j,M))$$

(11) sequentially repeating the steps (3) to (10), to determine the individual Wj values of all the amino-acid residues at the position (n+1 ≦ j ≦L-n) in the amino acid sequence of length (L) ,
(12) selecting at least one amino-acid residue to be subjected to mutation on the basis of the alignment data carried out in the step (1) from the amino acid sequence of length (L) of the protein "*A*", and sequentially repeating the steps (3) to (10) for variant amino-acid residues in various mutated amino acid sequences where the selected amino-acid residue has been mutated into another amino-acid residue, to determine the Wj value of the variant amino-acid residue,
(13) selecting a mutated amino acid sequence wherein the Wj value of the variant amino-acid residue as determined in the step (12) is larger or smaller than the Wj value of the wild type amino-acid residue as determined in the step (10), and
(14) preparing a modified gene encoding the modified amino acid sequence from the protein "*A*" gene, and producing the modified protein as the expression product of the gene.

An eighth invention is a thermophilic DNA polymerase, prepared by artificially modifying the amino acid sequence of *Pfu* DNA polymerase so that the elongation of synthesized DNA chain might not be terminated intermediately during the catalysis for the synthesis of a DNA chain complimentary to a single-stranded DNA, and more specifically, the thermophilic DNA polymerase is one comprising the amino acid sequence of SQ ID No.1.
In association with the eighth invention, the present application provides a DNA sequence encoding the amino acid sequence of SQ ID No.1 and a recombinant vector carrying the DNA sequence. Such recombinant vector includes recombinant plasmid pDP320 carried on *Escherichia coli* HMS174 (DE3) /pDP320 (FERM P-16052).
Still furthermore, in accordance with the present invention, it is provided a method for preparing a thermophilic DNA polymerase, comprising culturing a cell transformed with an expression vector carrying the DNA sequence and isolating and purifying the objective enzyme generated in a culture medium.
A ninth invention is a thermophilic DNA polymerase, prepared by artificially modifying the amino acid sequence of *Pfu* DNA polymerase so that the synthesized DNA chain might be more elongated during the catalysis for the synthesis of a DNA chain complimentary to a single-stranded DNA, and more specifically, the thermophilic DNA polymerase is one comprising the amino acid sequence of SQ ID No.6 or a DNA polymerase comprising the amino acid sequence of SQ ID No.7.
In association with the ninth aspect of the present invention, the present application provides a DNA sequence encoding the amino acid sequence of SQ ID No.6 or 7, and a recombinant vectors carrying such DNA sequences, respectively. As such vectors, there are provided recombinant plasmid pDP5b17 carried on *Escherichia coli* HMS174 (DE3)/pDP5b17 (FERM BP-6189) (vector carrying the DNA sequence encoding the amino acid sequence of SQ ID No. 1), and recombinant plasmid pDP5C4 carried on *Escherichia coli* HMS174 (DE3)/pDP5C4 (FERM BP-6190) (vector carrying the DNA sequence encoding the amino acid sequence of SQ ID No.1).

Still furthermore, it is provided a method for producing a DNA polymerase, comprising culturing a cell transformed with an expression vector carrying the DNA sequence and isolating and purifying the objective enzyme produced in a culture medium.

The method for predicting a protein functional site in accordance with the first invention has been established on a priceple as follows. A protein is composed of a sequence of twenty amino acids, but the sequence is not random. Hence, the frequency of occurrence of a specific oligopeptide as a partial amino acid sequence in the entire proteins encoded by genome derived from an appropriate organism species is not constant, but some oligopeptides occur at high frequencies in various proteins while other oligopeptides rarely occur therein. It is recognized that among them, oligopeptides highly frequently occurring in common to various proteins do not have any potency to determine the uniqueness (specificity) of individual proteins, namely any potency to determine the functions, while oligopeptides occurring at low frequencies adversely determine the uniqueness and functions of individual proteins.

It is suggested that the functional site of protein is composed of oligopeptides occurring at low frequencies. Additionally, longer oligopeptides, more rarely occurring, increase in number. In other words, oligopeptide of length (n+1) as shown in the step (3) according to the method of the first invention is mostly the shortest oligopeptide occurring at a low frequency, and the calculated functional value $Z_j$ of amino-acid residue $A_j$ at an appropriate position j in the oligopeptide is the coefficient of the occurrence (namely, the representative value of the function) of the amino-acid residue $A_j$ at the position.

According to the method for predicting the protein functional site in accordance with the second invention, the contribution degree of the amino-acid residue $A_j$ to the frequency of the occurrence of $A_j$-oligopeptide can be evaluated on the basis of the ratio $Y_{ji}$ of the frequency of the occurrence of the $A_j$-oligopeptide to that of the $X_i$-oligopeptide as shown in the step (3), and thus, the calculated functional value $Z(j,n)$ of the amino-acid residue $A_j$ at an appropriate position of a protein serves as the coefficient of the occurrence of the amino-acid residue $A_j$ at the position (namely, the representative value of the function).

Furthermore, the value $Z(j, n)$ varies, depending on the species of amino-acid residue $A_j$. In the step (7) according to the inventive method, the distribution of the $Z(j, n)$ value of each of twenty amino acids is determined in the entire proteins of organism "*a*", to determine $D(j, n)$ value by normalizing the $Z(j, n)$ value on the basis of the mean and standard deviation of $Z(j,n)$ value of each amino acid, as determined on the basis of the distribution, which serves as the representative value of the function, after correction of the bias due to each amino-acid residue species.

Furthermore, longer oligopeptides, more rarely occurring, increase in number. Because the $Z(j, n)$ and $D(j, n)$ values generally vary, depending on the length (n), accordingly, the functional value $W_j$ of the $Z(j, n)$ and $D(j, n)$ as determined on a variety of length (n) is defined as the representative value of the function.

The systems for predicting a protein functional site in accordance with the third and fourth inventions are individually systems for automatically carrying out the methods in accordance with the first and second inventions; the methods for modifying protein in accordance with the fifth and sixth inventions are methods for preparing mutant proteins by substituting the amino-acid residue at the functional site predicted by the method of the first invention with another amino-acid residue. Still further, the method for modifying a protein in accordance with the seventh invention is a method for preparing a mutant protein by substituting the amino-acid residue at the functional site predicted by the method in accordance with the second invention with another amino-acid residue. In accordance with the present invention, a novel thermophilic DNA polymerase (the eighth and ninth inventions) is provided as a modified protein.

The term "DNA polymerase" is the generic name of enzymes catalyzing the synthesis of a DNA chain complimentary to a single-stranded DNA. DNA polymerase is an essential enzyme for DNA sequencing and *in vitro* DNA amplification, and "thermophilic DNA polymerase" is inevitable for PCR (polymerase chain reaction) in terms of the automation of a series of the reaction cycles.

Such thermophilic DNA polymerase includes known ones, for example *Taq, Pfu, KOD*, which are separately used, depending on the characteristic performance. *Pfu* DNA polymerase in particular has been known as an enzyme with an extremely low frequency of erroneous reading during the synthesis of DNA strands (at a high fidelity). However, the *Pfu* DNA polymerase is inappropriate for the amplification of polymeric DNAs such as genome DNA, because the synthetic DNA yielded by the *Pfu* DNA polymerase is low and the activity thereof to elongate a synthetic chain is insufficient. Thus, the present application provides a novel *Pfu* DNA polymerase prepared according to the method of the fifth invention.

BRIEF DESCRIPTION OF DRAWINGS

Fig.1 depicts graphically the individual frequencies of the occurrences of oligopeptides of lengths 3, 4 and 5, and the distributions of the individual frequencies thereof according to the method of the first invention;

Fig.2 depicts an example of an amino acid sequence of a length of 20, and examples of Aj-oligopeptide of a length of 4, containing the amino-acid residue Met at position 5 in the sequence and examples of Xi-oligopeptides;

Fig.3 depicts graphically the individual frequencies of the occurrences of oligopeptides of lengths of 2, 3, 4 and 5, and the distributions of the individual frequencies thereof according to the method of the second invention;

Fig.4 depicts an example of the flow chart for conducting the step (1) of the method of the second invention;

Fig.5 depicts an example of the flow chart for conducting the steps (2') to (3) of the method of the second invention;

Fig.6 depicts an example of the flow chart for conducting the steps (4) to (5) of the method of the second invention;

Fig.7 depicts a distribution of the frequency of Z (j, 3) of each of three amino acids, see below, according to the method of the second invention, wherein the solid line expresses the distribution of that of isoleucine (Ile); the dotted line expresses the distribution of that of alanine (Ala); and the alternate long and short dash line expresses the distribution of that of methionine (Met);

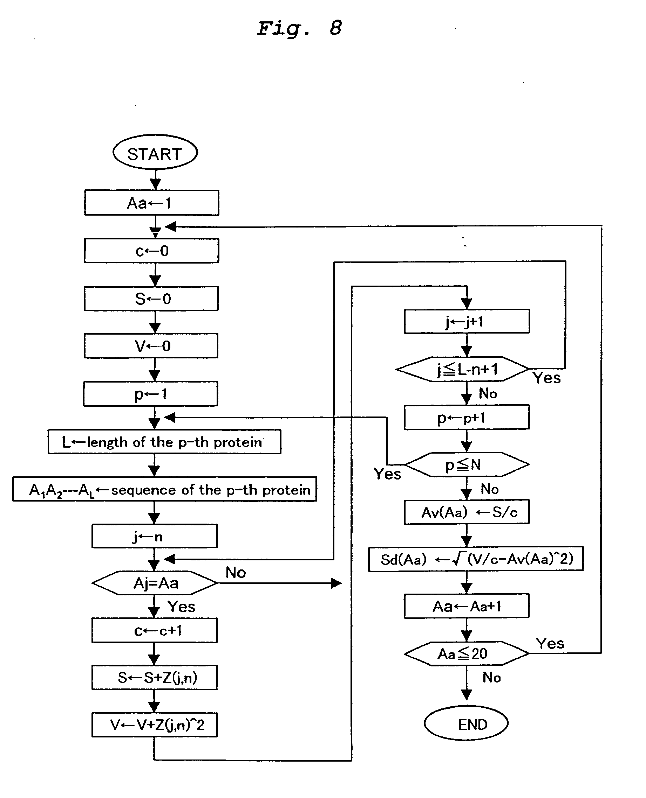

Fig.8 depicts an example of the flow chart for conducting the step (7) of the method of the second invention;

Fig.9 depicts an example of the flow chart for conducting the step (8) of the method of the second invention;

Fig.10 depicts an example of the flow chart for conducting the step (9) of the method of the second invention;

Fig 11 depicts a block diagram illustrating the system of the third invention;

Fig.12 depicts a block diagram illustrating the system of the third invention;

Fig.13 depicts the electrophoresis results of conventional *Pfu* DNA polymerase and *KOD* DNA polymerase, indicating the primer elongating activities thereof;

Fig.14 depicts a distribution chart of the plotted Zj = -logYj value for the whole amino acid sequence of the $\alpha$-type DNA polymerase encoded by MJ0885, which value is calculated by the first invention;

Fig.15 depicts a distribution chart of the plotted Zj = -logYj value for partial sequences (motif A and motif C) of the amino acid sequence of which the distribution chart is shown in Fig.14;

Fig.16 depicts a frequency distribution chart of the Zj = -logYj value calculated on the basis of the amino acid sequence of the $\alpha$-type DNA polymerase encoded by MJ0885;

Fig.17 depicts alignment charts of the amino acid sequences of the individual motif Cs from the $\alpha$-type DNA polymerases *Pfu, KOD* and *MJ;*

Fig.18 depicts distribution charts of the plotted Zj = -logYj values for the individual motif Cs of the $\alpha$-type DNA polymerases *Pfu, KOD* and *MJ;*

Fig.19 depicts distribution charts of the plotted values of Wj = Z(j, 3) - Z(j,1) (in solid line), Wj = Z(j, 4) - Z(j, 3) (in dotted line) and Wj = Z(j, 5) - Z (j, 3) (in alternate long and short dash line) of the 100 residues from the N-terminus of the whole amino acid sequence of the $\alpha$-type DNA polymerase encoded by MJ0885, and these values are calculated by the method of the second aspect of the present invention;

Fig.20 depicts distribution charts of the plotted value Wj = Z (j, 5) - Z (j, 3) for partial sequences (regions comprising exoI, exoII, motif A, motif B and motif C) of the amino acid sequence of the $\alpha$-type DNA polymerase encoded by MJ0885;

Fig.21 depicts distribution charts of the plotted values of Wj = D(j, 3) (in dark color) and Wj = D (j, 5) (in pale color) for partial sequences (regions comprising exoI, exoII, motif A, motif B and motif C) of the amino acid sequence of the $\alpha$-type DNA polymerase encoded by MJ0885;

Fig.22 depicts distribution charts in dark color of the positions of amino-acid residues with Wj = D (j, 3) of 2 or more or of 2 or less in the three-dimensional structure of the amino acid sequence of enolase encoded by MJ0232 on a three-dimensional structure model;

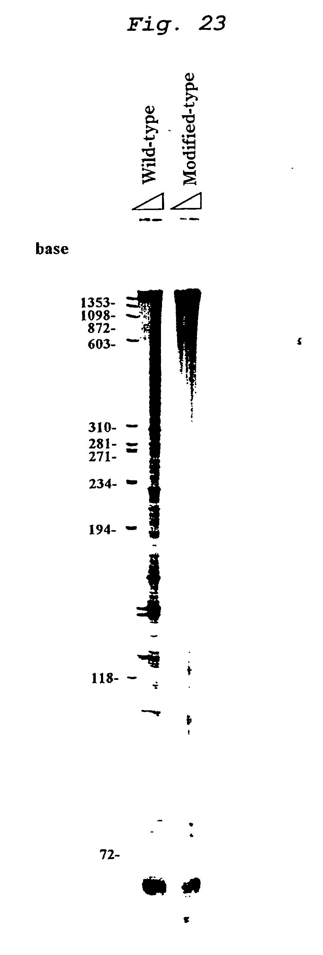

Fig.23 depicts the results of electrophoresis, indicating the primer elongating activities of the conventional *Pfu* DNA polymerase (wild type) and the modified *Pfu* DNA polymerase I of the present invention; and

Fig.24 depicts the results of electrophoresis, indicating the primer elongating activities of the conventional *Pfu* DNA polymerase (wild type) and the modified *Pfu* DNA polymerases II and III of the present invention.

BEST MODE FOR CARRYING OUT THE INVENTION

The method for predicting a functional site of a protein in accordance with the first invention is a method for predicting the functional site of a protein of organism "*a*" with a known genome data or cDNA analysis data, in the entire putative proteins of the organism "*a*", essentially comprising the following steps (1) to (6).

Step (1):

By calculating the frequency of the occurrence of each amino acid and the frequencies of the occurrences of individual oligopeptides produced by permutations of twenty amino acids in the amino acid sequences of the entire proteins of the organism "*a*", the oligopeptide length (n) is determined.

The length (n) is determined, then, as the smallest integer satisfying the following criteria:

"Among oligopeptides of length (n), the number of oligopeptides that occur once in the entire proteins is smaller than the number of oligopeptides that occur twice in the entire proteins; among oligopeptides of length (n+1), the number of oligopeptides that occur once in the entire proteins is larger than the number of oligopeptides that occur twice in the entire proteins."

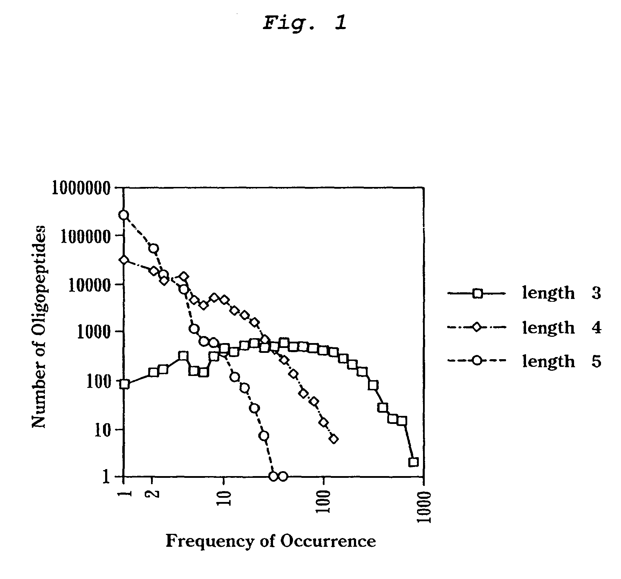

For example, Fig.1 depicts distribution charts of the frequencies of the occurrences of oligopeptides of a length of 3, oligopeptides of a length of 4 and oligopeptides of a length of 5, in the entire proteins encoded by the genome of a microorganism *Methanococcus jannaschii* (Bult et al.,Science 273, 1058-1073, 1996). In the case of the three types of length of the oligopeptides shown in Fig. 1, the smallest n in the step (1) is 3.

Step (2):

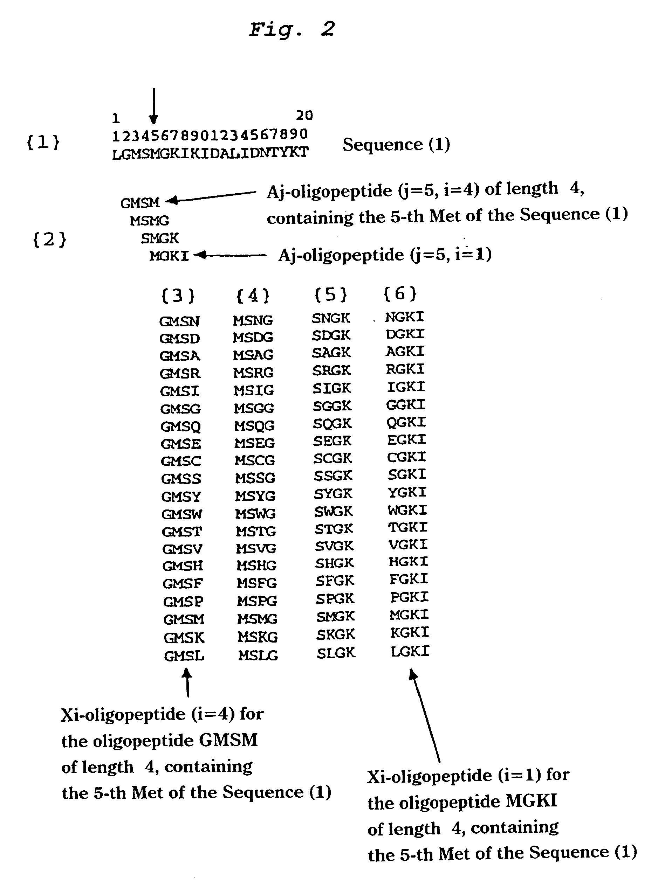

Given that the j-th amino-acid residue from the N-terminus of the amino acid sequence of length (L) of the protein as a subject for predicting a functional site is described here as Aj ($n+1 \leqq j \leqq L-n$), the frequency of occurrence of a partial sequence of the amino acid sequence of the protein, which sequence corresponds to the following Aj-oligopeptide of length (n+1), containing the j-th amino-acid residue Aj;
Aj-oligopeptide: aj1aj2....Aji....ajnaj(n+1) (wherein, $1 \leqq i \leqq n+1$; Aj = Aji; and Aj is the i-th residue of the oligopeptide),
and the frequency of the occurrence of the following Xi-oligopeptide of length (n+1);
Xi-oligopeptide: ajlaj2....Xji....ajnaj(n+1) (wherein, the i-th residue Xji is any amino acid)
should be determined in the entire proteins of the organism *"a"*.
Such Aj-oligopeptide and Xi-oligopeptide can be illustrated for example in Fig.2. The upper row (1) in Fig.2 expresses in single letter code the partial sequence from the N-terminus to the 20-th amino-acid residue of the putative amino acid sequence speculated from the gene MJ0885, which is believed to encode the $\alpha$-type DNA polymerase of *Methanococcus jannaschii* (Bult et al., Science 273, 1058-1073, 1996); the middle row (2) expresses examples of Aj-oligopeptide of a length of 4, containing the 5-th amino-acid residue Met(M) in the amino acid sequence; and the rows (3) to (6) further below express examples of Xi-oligopeptide containing the 5-th amino-acid residue M.

Step (3):

Calculating ratio Yji of the frequency of the occurrence of the Aj-oligopeptide to that of the Xi-oligopeptide.

Step (4):

The mean Yj of the Yji is calculated as follows.

$$Yj = \sum_{i=1}^{n+1} Yji \ / (n+1),$$

Step (5):

Monotonously decreasing functional value or monotonously increasing functional value Zj of Yj is determined as follows;

$$Zj = f(Yj).$$

The Zj value is defined as a representative value of the function of the j-th amino-acid residue of the amino acid sequence of length (L).

Step (6):

By subsequently repeating the steps (2) to (5) sequentially and determining the Zj value of each of all the amino-acid residues at position $n+1 \leqq j \leqq L-n$, the degree of the involvement of each amino-acid residue in the function of the protein is predicted by using the dimension of the Zj value as an indicator. More specifically, because the manner of occurring of each amino-acid residue in the context is expressed as the functional value Zj of Yj, a larger Zj value indicates a lower frequency of occurrence of the amino-acid residue if Zj is a monotonously decreasing functional value, which suggests that the amino-acid residue has higher responsibility over the performance of the function. If Zj is a monotonously increasing function, additionally, it is suggested that an amino-acid residue with a smaller Zj value has greater responsibility over the function.

By expressing the Zj value of each amino-acid residue for example in a distribution chart wherein the Zj value is plotted on the vertical axis while the amino acid sequence is shown on the horizontal axis, furthermore, the functional site can be confirmed at a glance, which is preferable as an embodiment for carrying out the present invention.

The second invention is a method for predicting a functional site of an appropriate protein in the entire putative proteins of the organism "*a*" with a known genome data or cDNA analysis data, essentially comprising the following steps (1) to (9).

Step (1):

The frequency of the occurrence of each amino acid and the frequencies of the occurrences of individual oligopeptides produced by permutations of twenty amino acids, in the amino acid sequences of the entire proteins of the organism "*a*", are calculated.

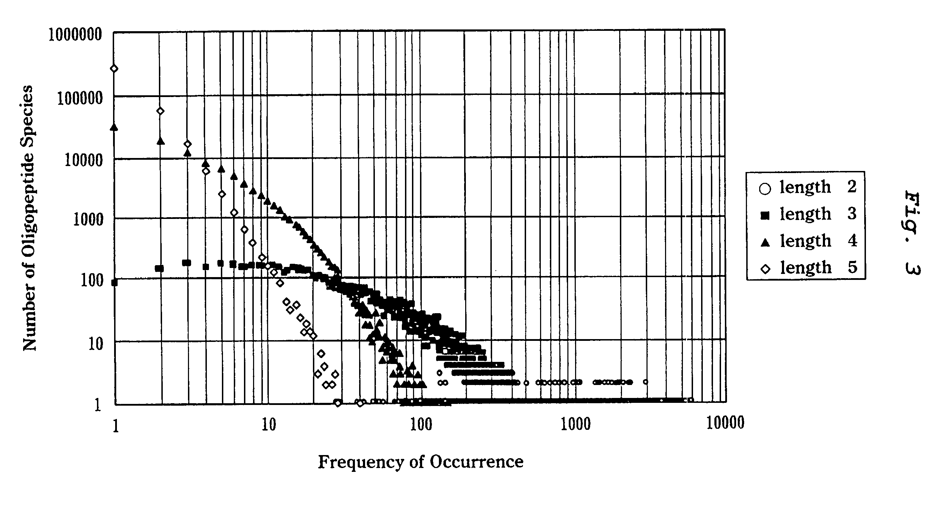

For example, Fig.3 shows a distribution chart of the frequencies of the occurrences of oligopeptides of a length of 3, oligopeptides of a length of 4 and oligopeptides of a length of 5, which are determined in the entire proteins encoded by the genome of a microorganism *Methanococcus jannaschii* (Bult et al., Science 273, 1058-1073, 1996) on the basis of the genome data of the microorganism.

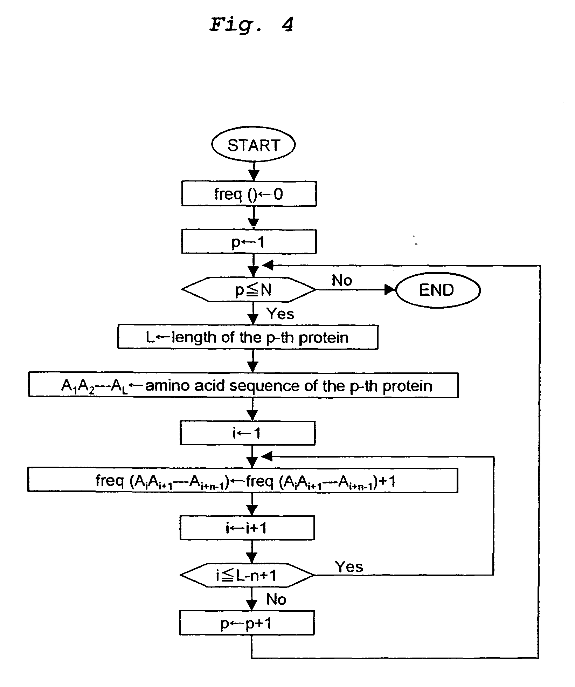

Fig.4 depicts an example of the flow chart for carrying out the step (1).

Step (2):

As to a protein of organism "*a*",

Step (2'):

given that the j-th amino-acid residue from the N-terminus of the amino acid sequence of length (L) of the protein is described here as Aj, the frequency of the occurrence of a partial sequence of the amino acid sequence of the protein, which sequence corresponds to the following Aj-oligopeptide of an appropriate length n ($1 \leqq n \leqq M$, provided that "M" is the smallest length of oligopeptides satisfying the following criterion; all the oligopeptides of length M are at frequency 1 of the occurrence), containing the j-th amino-acid residue Aj ($n \leqq j \leqq L-n+1$) ;

Aj-oligopeptide: ajlaj2....Aji....ajn (wherein, $1 \leqq i \leqq n$; Aj = Aji and Aji is the i-th residue of the oligopeptide),

and the frequency of the occurrence of the following Xi-oligopeptide of the length n corresponding to the legnth of the Aj-oligopeptide;

Xi-oligopeptide: aj1aj2....Xji....ajn (wherein, the i-th residue Xji is any amino acid),

are calculated in the entire proteins of the organism "*a*".

In the same manner as by the method of the first invention, such Aj-oligopeptide and Xi-oligopeptide are for example illustrated as in Fig.2.

Step (3):

The ratio Yji of the frequency of the occurrence of the Aj-oligopeptide to that of the Xi-oligopeptide is calculated.

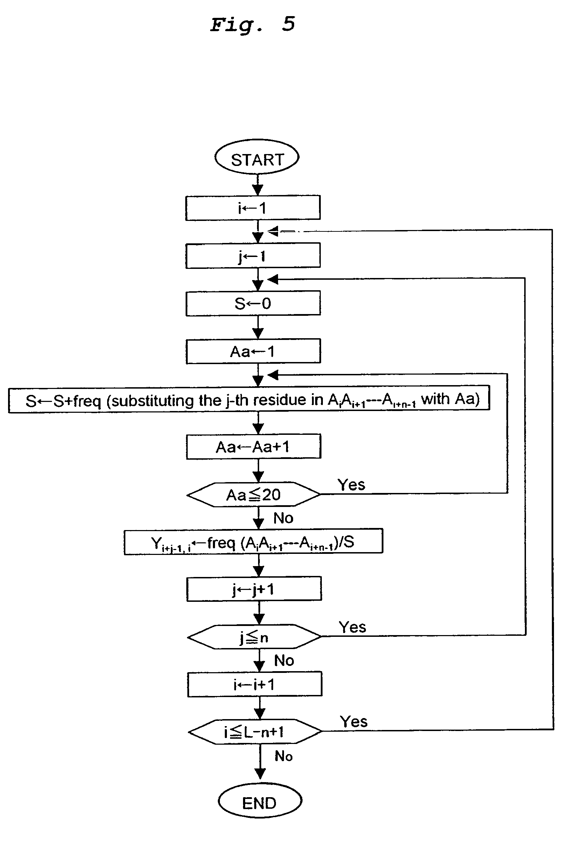

Fig.5 depicts an example of the flow chart for carrying out the aforementioned steps (2') to (3).

Step (4):

The mean Y(j, n) of the Yji is calculated as described below:

$$Y(j, n) = \sum_{i=1}^{n} Yji/n.$$

Step (5):

The logarithmic value Z(j, n) of Y(j, n) is determined as follows.

$$Z(j, n) = -\log(Y(j, n))$$

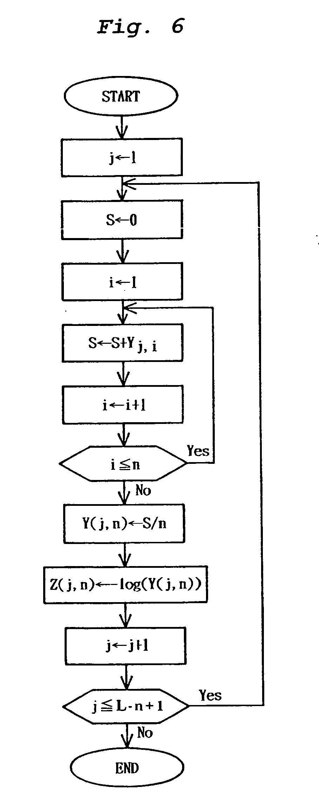

Fig.6 depicts an example of the flow chart for carrying out the aforementioned steps (4) to (5).

Step (6):

By subsequently repeating the steps (2') to (5) sequentially, the Z(j, n) value of each of all the amino-acid residues at position $n \leqq j \leqq L-n+1$ in the amino acid sequence of length (L) is determined.

Step (7):

By sequentially repeating the steps (2) to (6) over the entire proteins of the organism "*a*", thereby determining the distribution of the Z(j,n) value of each amino-acid residue in the entire proteins, and the Z(j,n) values are classified into twenty according to the twenty amino acids, and then calculating mean Av(Aa) of the Z(j,n) values for each amino acid Aa and the standard deviation Sd (Aa) of the distribution thereof, on the basis of the distribution, to determine function g to the j-th amino-acid residue Aj of a protein for normalizing the difference in distribution due to the species of amino-acid residues is determined;

$$g = (Z(j, n), Aj) = [Z(j, n) - Ad(Aa)]/Sd(Aa)$$

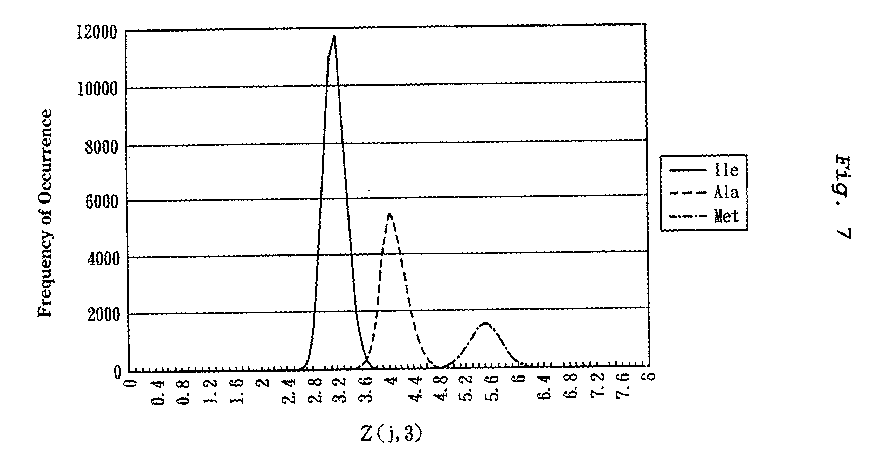

(whrerein, Aj = Aa).
For example, Fig. 7 depicts a distribution of the frequency of Z (j, n) for three species of amino acids, namely isoleucine (Ile), alanine (Ala) and methionine (Met), in the entire proteins encoded by the genome of *Methanococcus jannaschii* (Bult et al., Science 273, 1058-1073, 1996). Based on the distribution, the mean and standard deviation of the Z(j, n) values for an amino acid isoleucine (Ile), namely Ad (Ile) and Sd(Ile), respectively, are determined as Ad(Ile) = 3.16 and Sd(Ile) = 0.17, and the function g for Aj = Ile is determined as follows.

$$g= (Z(j, n), Aj) = (Z(j, n) - 3.16)/0.17$$

Fir. 8 depicts an example of the flow chart for carrying out the step (7).

Step (8):

The value D(j, n) of the function g of each of all the amino-acid residues Aj at position $n \leqq j \leqq L-n+1$ in the amino acid sequence of length (L) as recovered in the step (7) is determined;

$$D(j, n) = g (Z(j, n), Aj).$$

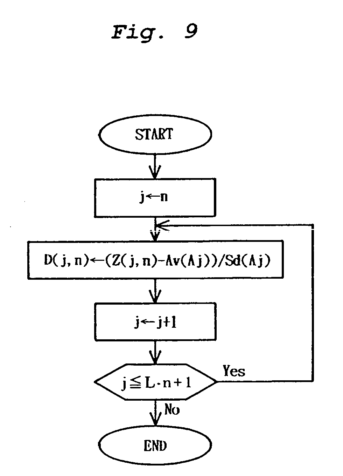

Fig.9 depicts an example of the flow chart for carrying out the step (8).

Step (9):

The functional value Wj of the Z (j, n) value and the D (j, n) value is determined as follows.

$$Wj = h(Z(j, 1), Z(j, 2),..., Z(j, M), D(j,1), D(j, 2),..., D(j, M))$$

By defining the value of the Wj as the representative value of the function of the j-th amino-acid residue in the amino acid sequence of length (L), the degree of the responsibility of each amino-acid residue over the function of the protein is estimated by using the dimension of the Wj value as an indicator.

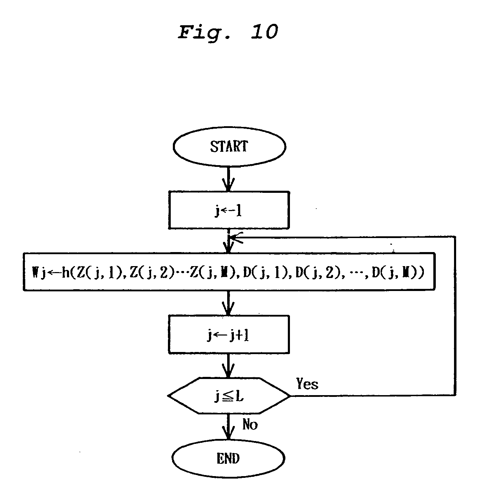

Fig.10 depicts an example of the flow chart for carrying out the step (9). By expressing the Wj value of each amino-acid residue for example in a distribution chart wherein the Wj value is plotted on the vertical axis while the amino acid sequence is shown on the horizontal axis, furthermore, the functional site can be confirmed at a glance, which is preferable as an embodiment for carrying out the present invention.

If the three-dimensional structure of the protein as a subject for predicting the functional site is known or if a three-dimensional structure model thereof can be prepared by known methods (for example, homology modeling method, Peitsch, Proceedings of the Fifth International Conference on Intelligent Systems for Molecular Biology, 1997, 5, 234-236), the distribution is expressed on the three-dimensional structure, whereby a spatial arrangement of an amino-acid residue as a candidate of a novel functional site can be confirmed, which is preferable as an embodiment for carrying out the invention.

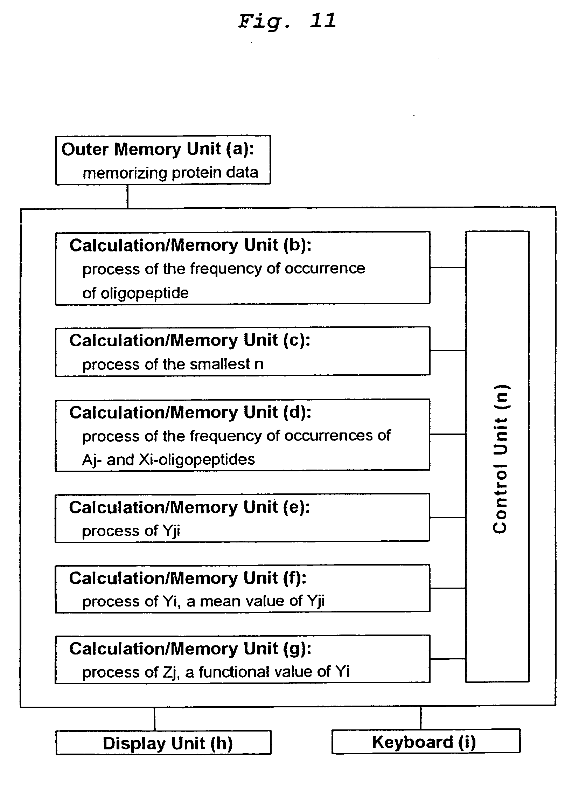

The third invention is a system for automatically carrying out the method for predicting a functional site in accordance with the method of the first invention, at least comprising the following units (a) to (g) for conducting the steps (1) to (6) of the method of the first invention, as shown for example in the composition example in Fig.11.

Outer memory unit (a):

Unit memorizing the amino acid sequence data of a protein or an existing protein data base for use in the step (1) the first invention.

Calculation/memory unit (b):

Unit, composed of CPU calculating the frequencies of the occurrences of individual oligopeptides as determined in the step (1), and a memory unit having the memory of the calculation results.

Calculation/memory unit (c):

Unit, composed of CPU calculating the smallest length (n) of oligopeptides as determined in the step (1) and a memory unit having the memory of the length n.

Calculation/memory unit (d):

Unit, composed of CPU calculating the the frequencies of occurrence of each amino acid and the frequencies of occurrence of Aj-oligopeptide and Xi-oligopeptide in the entire proteins as determined in the step (2) and a memory unit having the memory of the calculation results.

Calculation/memory unit (e):

Unit, composed of CPU calculating the Yji value as determined in the step (3) and a memory unit having the memory of the Yji value.

Calculation/memory unit (f):

Unit, composed of CPU calculating the Yj value as determined in the step (4) and a memory unit having the memory of the Yj value.

Calculation/memory unit (g):

Unit, composed of CPU calculating the Zj value as determined in the step (5) and a memory unit having the memory of Zj.

Additionally, the system for predicting a functional site is provided with the following display unit (h) in a preferable embodiment.

Display unit (h):

Unit displaying the Zj value of each amino-acid residue recovered in the calculation/memory unit (g) in a distribution chart.

The system of the present invention may be equipped with keyboard (i) and control unit (j) and the like as illustrated in Fig.11, in addition to these units (a) to (h).

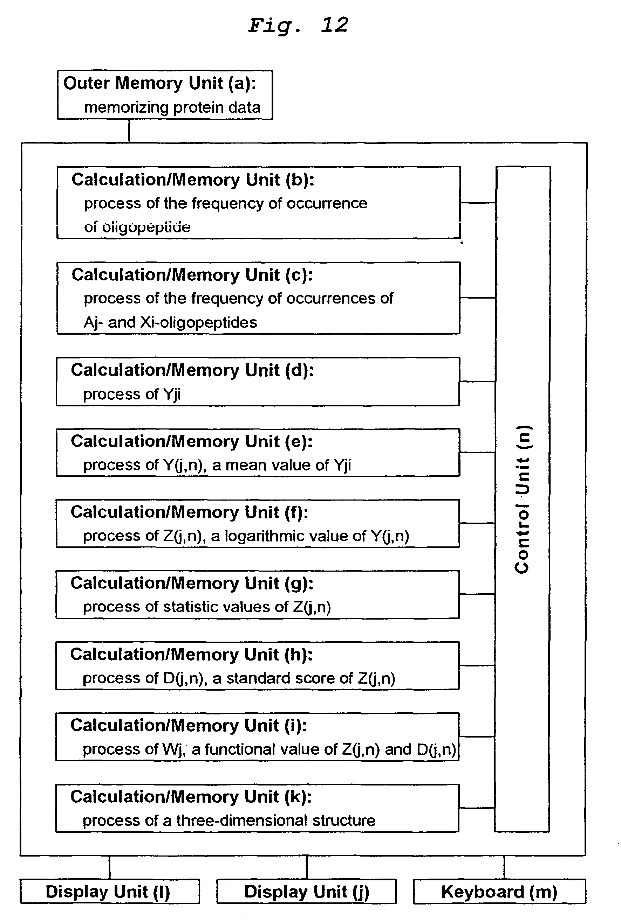

According to the fourth invention, the system for predicting a protein functional site is a system for automatically conducting the method of the second invention, at least comprising the following units (a) to (i) for carrying out the steps (1) to (9) according to the method of the second invention, as shown in the composition example in Fig.12.

Outer memory unit (a):

Unit memorizing the amino acid sequence data and an existing protein data base for use in the step (1).

Calculation/memory unit (b):

Unit, composed of CPU calculating the frequencies of the occurrences of individual oligopeptides as determined in the step (1) and a memory unit having the memory of the calculation results.

Calculation/memory unit (c):

Unit, composed of CPU calculating the frequencies of occurrence of each amino acid and the individual frequencies of the occurrences of Aj-oligopeptide and Xi-oligopeptide in the entire proteins as determined in the step (2') and a memory unit having the memory of the calculation results.

Calculation/memory unit (d):

Unit, composed of CPU calculating Yji as determined in the step (3) and a memory unit having the memory of the Yji value.

Calculation/memory unit (e):

Unit, composed of CPU calculating the Y(j, n) value as determined in the step (4) and a memory unit having the memory of the Y(j, n) value.

Calculation/memory unit (f):

Unit, composed of CPU calculating the Z(j, n) value as determined in the steps (5) and (6) and a memory unit having the memory of the Z(j, n) value.

Calculation/memory unit (g):

Unit, composed of CPU calculating the g value as determined in the step (7) and a memory unit having the memory of the g value.

Calculation/memory unit (h):

Unit, composed of CPU calculating the D(j, n) value as determined in the step (8) and a memory unit having the memory of the D(j, n) value.

Calculation/memory unit (i):

Unit, composed of CPU calculating the Wj value as determined in the step (9) and a memory unit having the memory of the Wj value.

Additionally, the system for predicting a functional site in accordance with the fourth invention may be equipped with an appropriate combination of the following units (j) to (1).

Display unit (j):

Unit displaying the Wj value of each amino-acid residue as recovered with the unit (i) in a distribution chart.

Calculation/memory unit (k):

Unit memorizing an existing database of protein three-dimensional structures or unit preparing a three-dimensional structure model based on an amino acid sequence according to a known method and memorizing the three-dimensional structure.

Display unit (l):

Unit displaying the Wj value of each amino-acid residue in a distribution chart on the three-dimensional structure stored in the database or three-dimensional structure model recorded on the unit (k).
The system of the present invention may satisfactorily be equipped with keyboard (m) and control unit (n) and the like as illustrated in Fig.12, in addition to these units (a) to (1).
The fifth invention is a method for modifying the function of protein "*A*", which function has been known, derived from the entire putative proteins of organism "*a*" with a known genome data or cDNA analysis data, essentially comprising the following steps (1) to (10).

Step (1):

Extracting proteins closely related to the protein "*A*" from an existing protein data base and subjecting the proteins to alignment.

Steps (2) to (7):

Subjecting the amino acid sequences of the entire proteins of the organism "*a*" to the steps (1) to (6) according to the method of the first invention.

Step (8):

Selecting at least one amino-acid residue to be subjected to mutation from the amino acid sequence of length (L) of the protein "*A*" on the basis of the alignment data recovered in the step (1), sequentially repeating the steps (3) to (6) for a variant amino-acid residue of various mutated amino acid sequences where the selected amino-acid residue has been mutated into another amino-acid residue, to determine the Zj value of the variant amino-acid residue.

Step (9):

Selecting a mutated amino acid sequence wherein the Zj value of the variant amino-acid residue as determined in the step (8) is larger or smaller than the Zj value of the intact amino-acid residue as determined in the step (7).

Step (10):

Preparing a modified gene of the protein "*A*", which gene encodes the mutant amino acid sequence selected in the step (9), and expressing the modified gene in an appropriate host-vector system to prepare modified protein "*A*".
The sixth invention is a method for modifying the function of protein "*B*" derived from organism "b" with an unknown genome data or cDNA analysis data, essentially comprising the following steps (1) to (10).

Step (1):

Extracting protein "*A*" most closely related to protein "*B*" from the entire putative proteins of organism "*a*" with a known genome data or cDNA analysis data and subjecting the protein "*A*" to alignment, or extracting proteins closely related to protein "*B*" from an existing protein data base to subject the proteins to alignment.

Steps (2) to (8):

Conducting the steps (2) to (8) of the method of the third invention over the amino acid sequences of the entire proteins of the organism "*a*".

Step (9):

Selecting a position that should be mutated and an amino acid residue for which should be substituted wherein the Zj value of the substitued amino-acid residue as determined in the step (8) is larger or smaller than the Zj value of the intact amino-acid residue as determined in the step (7).

Step (10):

Preparing a modified gene of the protein "*B*" according to a known method, which gene encodes the amino acid sequence mutated at the position to another amino acid residue as selected in the step (9), and expressing the modified gene in an appropriate host-vector system to prepare modified protein *"B".*

As has been described above, the methods for modifying the protein function in the fifth and sixth invention, comprising the method for predicting a functional site of the first invention, are characterized in that an unknown functional site of protein is newly found and is subjected to mutation.

Furthermore, the method for modifying a protein function according to the seventh invention also utilizes the method for predicting the function in accordance with the second invention, whereby the method can be carried out in the same manner as in the case of the fifth invention.

The thermophilic DNA polymerase of the eighth and ninth invention is more specifically an enzyme prepared by modifying, in accodance with the sixth invention, a thermophilic *Pfu* DNA polymerase derived from *Pyrococcus furiosus* in a genetic engineering manner by the known method for preparing mutant gene (Strategies, Vol.9, p.3-4, 1996) (the thermophilic DNA polymerase of the present invention is sometimes referred to as "modified *Pfu* DNA polymerase"). The enzyme can be prepared as follows.

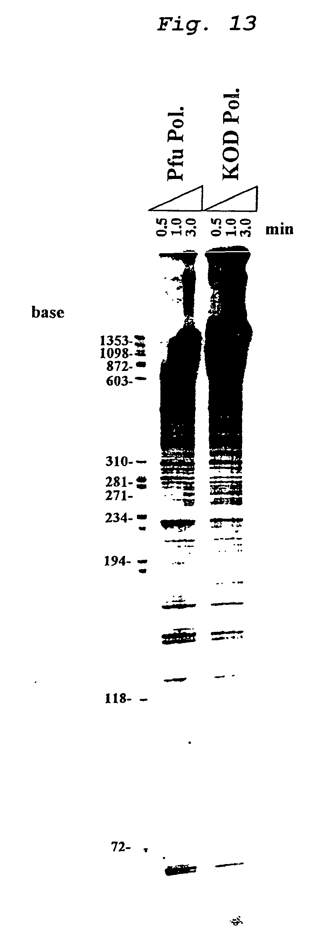

Since the nucleotide sequence of the gene of *Pfu* DNA polymerase is known (Nucleic Acids Research, Vol.21, p.259-265, 1993), the gene of *Pfu* DNA polymerase is prepared by PCR comprising synthetically preparing an oligopeptide complementary to both the ends by using the genome DNA of the *archaebacterium* as template and using the oligopeptide as primer. The DNA fragment of the gene is cloned into a vector, and the gene is subsequently subjected to mutation by the method described in the reference mentioned above. In accordance with the present invention, in particular, the mutation of the gene was executed by nucleotide substitution, so that a part of the amino acid sequence of *Pfu* DNA polymerase might be substituted with the amino-acid residues from *KOD* DNA polymerase. In terms of amino acid sequence, *Pfu* DNA polymerase has about 80 % homology with *KOD* DNA polymerase, and therefore, similar synthetic termination occurs during PCR (Fig.13), but the elongation rate with *KOD* DNA polymerase is about 6-fold the rate with *Pfu* DNA polymerase. By substituting some amino-acid residues of *Pfu* DNA polymerase with some amino-acid residues of *KOD* DNA polymerase, the synthetic termination of the chain elongation might be improved or the elongation rate turns rapid, which possibly enables the recovery of an enzyme capable of elongating a DNA chain under way of synthesis more longer. By expressing in *Escherichia coli* the mutant gene that was mutated in such a manner and recovering and purifying the expression product, the modified *Pfu* DNA polymerase of the present invention was recovered.

The thermophilic DNA polymerase (modified *Pfu* DNA polymerase I) of the eighth invention is more specifically an enzyme of the amino acid sequence of SQ ID No.1. The amino acid sequence is a novel sequence prepared by identifying potentially function-modifiable amino-acid residues of the amino acid sequence of the conventionally known *Pfu* DNA polymerase, according to the inventive method for predicting a functional site, and substituting the amino-acid residues as shown in Table 1. By using the novel enzyme then for DNA synthesis by PCR, for example, the synthetic termination occurring when using the conventional DNApolymerases is almost totally overcome, as shown in the following examples. It is needless to say that template DNA chains to be highly efficiently amplified with the conventional polymerase can be amplified at high efficiency in the same manner.

Furthermore, the thermophilic DNA polymerases (modified *Pfu* DNA polymerases II and III) of the ninth invention are more specifically enzymes of amino acid sequences of SQ ID Nos.6 and 7, which are novel sequences prepared by identifying potentially function-modifiable amino-acid residues of the amino acid sequence of the *Pfu* DNA polymerase, according to the inventive method for predicting a functional site, and substituting the amino-acid residues as shown in Table 1. By using the novel enzymes then for DNA synthesis by PCR, for example, synthetic polymeric products can be recovered at large scales, as shown in the following examples.

Table 1

| Modified DNA polymerases | Positions | Wild-type amino acid | Modified amino acid |
|---|---|---|---|
| I | 2 | Ile | Val |
| | 533 | Phe | Tyr |
| | 538 | Leu | Ile |
| | 540 | Ile | Ser |
| | 545 | Leu | Phe |
| | 546 | Tyr | Phe |
| II | 2 | Ile | Val |
| | 710 | Pro | Arg |
| | 712 | Ser | Arg |
| | 713 | Asn | Asp |
| | 717 | Leu | Pro |
| III | 2 | Ile | Val |
| | 717 | Leu | Pro |

The DNA sequences encoding these modified *Pfu* DNA polymerases include for example the mutant genes of the *Pfu* DNA polymerase gene, as recovered during the process of enzyme preparation. As to these mutant genes, the DNA sequences encoding the amino acid sequences of SQ ID Nos.1, 6 and 7 for example have been cloned in recombinant plasmids p320, pDP5b17 and pDP5C4, respectively, and these recombinant plasmids have been integrated in *Escherichia coli* HMS174 (DE3) and deposited at the National Institute of Bioscience and Human-Technology, the Agency of Industrial Science and Technology, Japan (Deposit Nos. FERM P-16052, FERM BP-6189 and FERM BP-6190, respectively).

Additionally, the DNA sequences of the present invention may appropriately be designed as DNA sequences with conjugated nucleotide codons corresponding to the individual amino-acid residues of SQ ID No.1, 6 or 7.

The thermophilic DNA polymerases of the present invention may be expressed in microorganisms such as *Escherichia coli*, which may thereafter be recovered. By inserting and integrating the DNA sequence into an expression vector with an origin of replication in a microorganism, a promoter, a ribosome-binding site, a cDNA cloning site, and a terminator and the like to prepare an expression vector, transforming a host cell with the expression vector and thereafter culturing the resulting transformant, an enzyme encoded by the DNA sequence can be produced from the microorganism at a large scale.

EXAMPLES

The present invention will now be described more specifically in more detail with reference to examples, but the invention is not limited to the following examples.

Example 1

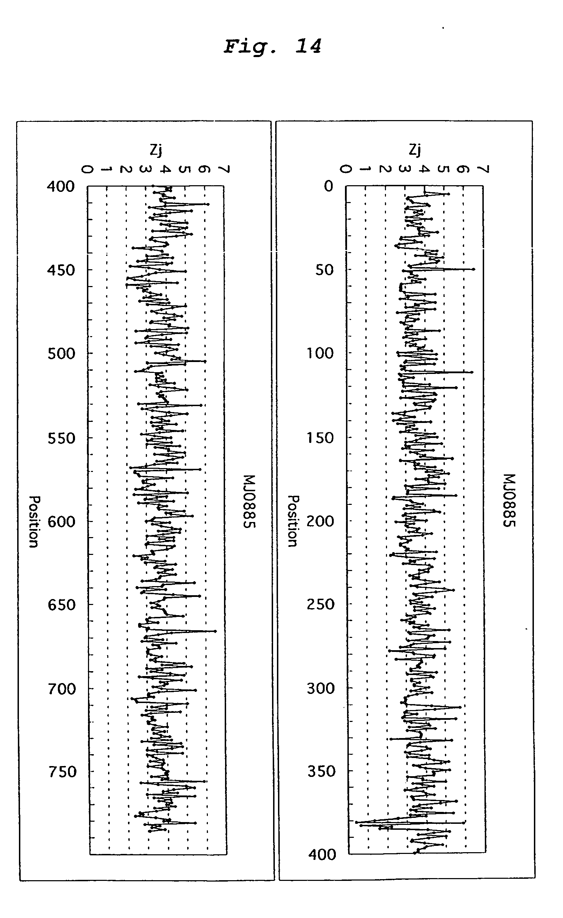

According to the method of the first invention and based on the genome data of *Methanococcus jannaschii* (Bult et al., Science 273, 1058-1073, 1996), $Zj = -\log Yj$ (f = -log) was calculated, concerning each amino-acid residue in the amino acid sequence (from the N-terminus to the C terminus) of a DNA polymerase speculated from the microbial gene MJ0885 which is thought to encode the $\alpha$-type DNA polymerase. The results are plotted in a distribution chart in Fig.14.

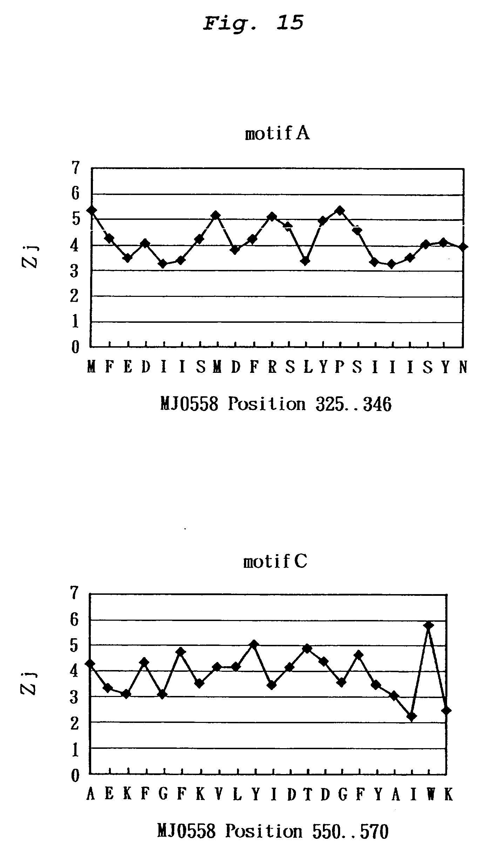

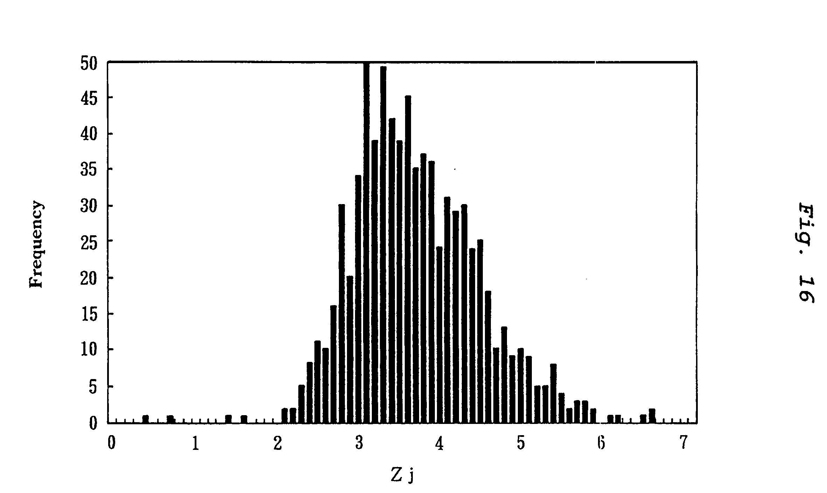

Among the motifs known as the functional sites of the $\alpha$-type DNA polymerase, furthermore, motif A and motif C were extracted, and the Zj values of the individual amino-acid residues were plotted in Fig.15. Fig.15 and Fig.16 below suggest that the $Zj = -\log Yj$ values of amino-acid residues responsible for the function are larger than those of the remaining amino-acid residues.

Fig.16 depicts a distribution chart of the frequency of the value $Zj = -\log Yj$ for the amino acid sequence of the $\alpha$-type DNA polymerase encoded by MJ0885. It is confirmed in the figure that amino-acid residues with value $Zj = -\log Yj$ of 4.8 or more are highly possibly amino-acid residues responsible for the protein function.

Example 2

Following the chart on Fig.15 in Example 1, the characteristic properties of $\alpha$-type DNA polymerase *Pfu* (DDBJ Accession No. D12983) which is derived from *Pyrococcus furious* were modified, on the basis of the amino acid sequence of an $\alpha$-type DNA polymerase KOD (DDBJ Accession No. D29671) which is derived from *Pyrococcus sp.*, and the genome data of *Methanococcus jannaschii* (Bult et al., Science 273, 1058-1073, 1996), and the amino acid sequence of the $\alpha$-type DNA polymerase (named here as *MJ*) encoded by MJ0885.

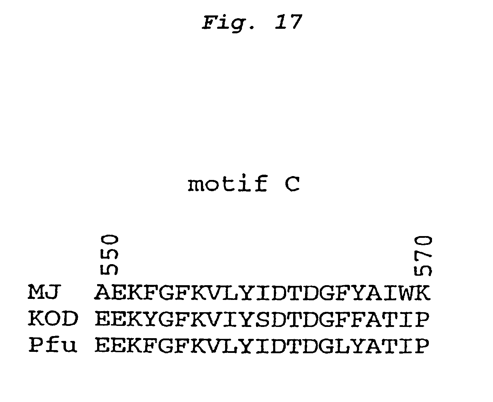

Fig.17 depicts alignment charts of the amino acid sequences of the motif Cs from *Pfu*, *KOD* and *MJ*, with no difference between the region 531 to 544 from *Pfu* and *MJ*.

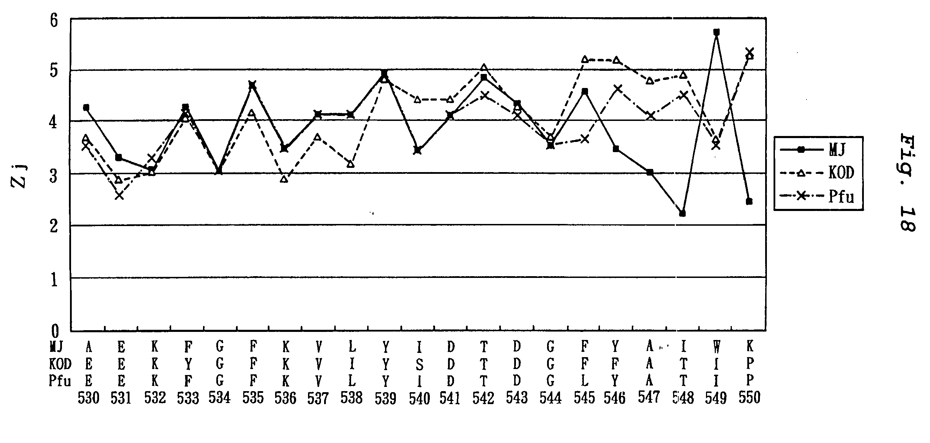

Fig.18 depicts the results of the prediction of functional sites in the amino acid sequences of the motif Cs of *Pfu, KOD* and *MJ* according to the method of the invention, on the basis of the genome data of *Methanococcus jannaschii.* The results indicate that mutations Ile540Ser, Leu545Phe, Tyr546Phe, and Ile568Thr increase the Zj = -logYj values of the amino-acid residues. Furthermore, the values Zj = -logYj of Asp541 and Ala547 are increased. By subjecting the $\alpha$-type DNA polymerase *MJ* of *Methanococcus jannaschii* to such mutation, the resulting sequence turns more unique (specific) which possibly brings about the improvement of some function.

Example 3

Based on the genome data of *Methanococcus jannaschii* (Bult et al., Science 273, 1058-1073, 1996), the values Z (j, 1) = -logY(j, 1), Z (j, 3) = -logY(j, 3), Z (j, 4) = -logY(j, 4), and Z (j, 5) = -logY(j, 5) were calculated, concerning the individual amino-acid residues of the amino acid sequence (from the N to C termini) of a DNA polymerase speculated on the basis of the microbial gene MJ0885 which is believed to encode the $\alpha$-type DNA polymerase, according to the method of the second invention, so that Wj = Z (j, 3) - Z(j, 1) (h = Z(j, 3) - Z (j, 1)) was calculated. Similarly, Wj = Z (j, 4) - Z(j, 3) (h = Z (j, 4) - Z(j, 3)) and Wj = Z (j, 5) - Z(j, 3) (h = Z (j, 5) - Z(j, 3)) were also calculated.

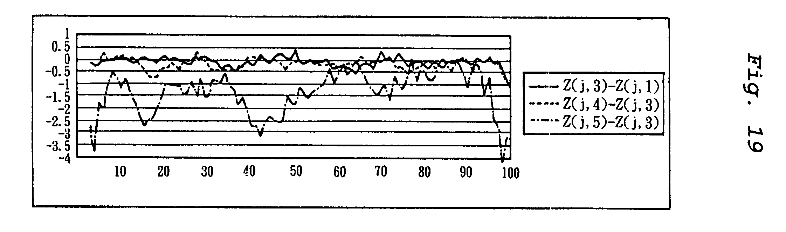

Fig.19 depicts the results of the 100 residues from the N-terminus in a plotted distribution chart. Given h = Z (j, 5) - Z(j, 3), regions with significantly different distributions from those of the remaining two cases are present in the region from the 35-th to 60-th residues and the like. The distributions indicate that smaller Wj = Z (j, 5) - Z(j, 3) more specifically characterizes the amino acid sequence.

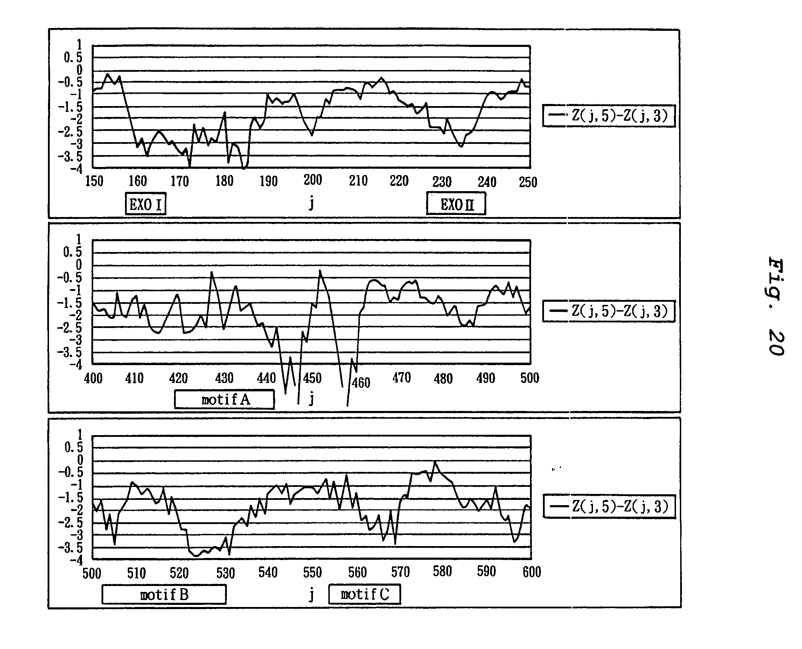

Among the motifs known as the functional sites of the $\alpha$-type DNA polymerases, furthermore, regions containing exoI, exoII, motif A, motif B and motif C were extracted, and subsequently, the Wj values of the individual amino-acid residues are plotted in Fig. 20. As shown in Fig.20, the regions with the characteristic reduction of Wj are consistent with the functional sites.

Example 4

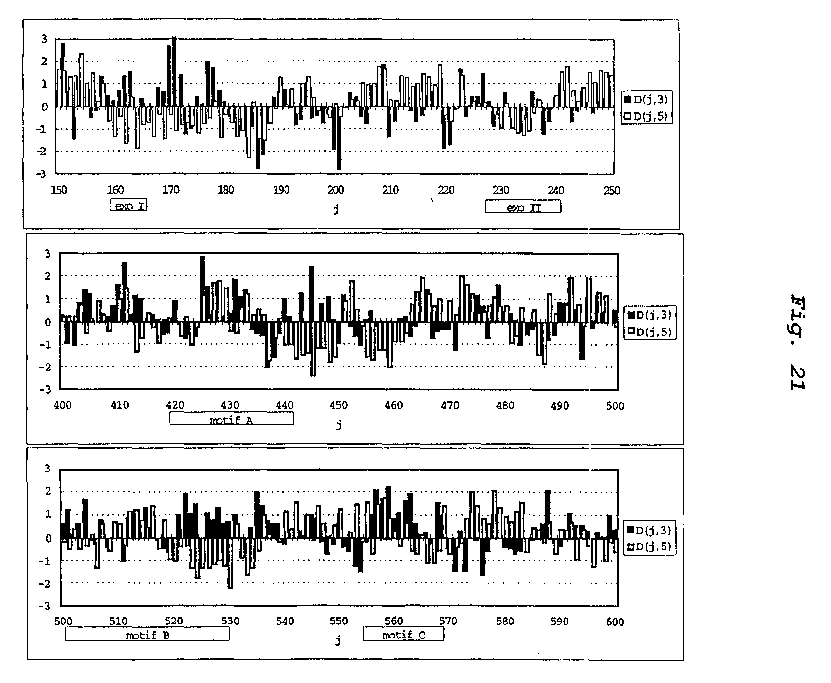

Fig.21 depicts the values Wj = D(j, 3) and Wj = D(j, 5) of individual amino-acid residues in the regions containing the exoI, exoII, motif A, motif B and motif C extracted among the motifs known as the functional sites in the $\alpha$-type DNA polymerases (h = D (j, 3) and h = D (j, 5)). Amino-acid residues with Wj = D (j , n) of 2 or more or of 2 or less are present outside the motifs, and these amino-acid residues are candidates of new functional sites.

Example 5

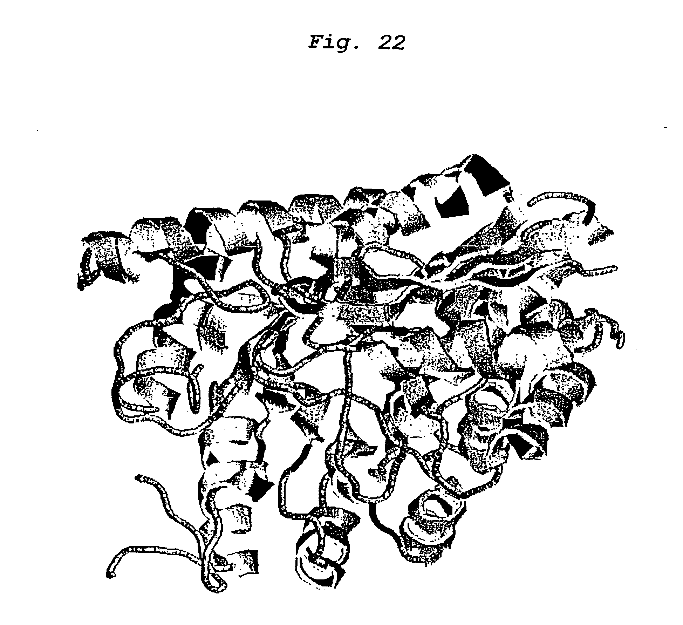

Fig.22 depicts in a dark color the positions of amino-acid residues having Wj = D (j, 3) of 2 or more or of 2 or less in the amino acid sequence of MJ0232, which is speculated as enolase of *Methanococcus jannaschii*, on a three-dimensional structure model prepared on the basis of the enolase of budding yeast. It is indicated that residues positioned apart on the amino acid sequence are closely positioned on the three-dimensional structure.

Example 6

Modified Pfu DNA polymerase I was prepared, by applying the mutation of putative amino-acid residues for improving the function of the DNA polymerase *MJ* in Example 2 to the *Pfu* DNA polymerase.

(1) Preparation of modified *Pfu* DNA polymerase gene Cloning of *Pfu* DNA polymerase gene:

Following the nucleotide sequence of *Pfu* DNA polymerase gene (Nucleic Acids Research, Vol.21, p.259-265, 1993), a PCR primer was prepared, for amplifying the objective gene by PCR by using the genome DNA of *P. furiosus* as template, which was then cloned in an expression vector for *Escherichia coli*. The detail is described below.

*P. furiosus* DSM3638 was cultured according to the method described in the reference described above. First, the culture medium described in the reference was prepared, followed by sterilization at a high temperature under pressure, and subsequently, nitrogen gas was purged into the resulting culture medium. The bacterium was inoculated into the culture medium, for stationary culturing at 95°C for 15 hours. From 200 ml of the culture broth were recovered the bacteria of about 0.5 mg by centrifugation. The collected bacteria were suspended in buffer A (10 mM Tris/HCl (pH 8.0), 1 mM EDTA, 100 mM NaCl), followed by addition of 1 ml of 10 % SDS and subsequent agitation, and to the resulting suspension was added 0.5 mg of proteinase K for reaction at 55 °C for 60 minutes. The reaction solution was extracted sequentially in phenol, phenol/chloroform, and chloroform, and to the extract was added ethanol to make the DNA insoluble, which was then recovered. The resulting DNA was dissolved in 1 ml of TE buffer (10 mM Tris/HCl(pH 8.0), 1 mM EDTA), followed by addition of 0.5 mg RNase A for reaction at 37°C for 60 minutes and re-extraction sequentially in phenol, phenol/chloroform, and chloroform, and subsequent ethanol precipitation, to recover the DNA, which was then dissolved in the TE buffer, to recover the DNA at about 0.3 mg.

For PCR amplification of the objective DNA polymerase gene, then, two primer DNAs of SQ ID Nos.2 and 3 were synthesized on the basis of the known sequence data. More specifically, it was designed that the initiation codon ATG of the objective gene and a restriction nuclease NcoI sequence (5'-CCATGG-3') might be introduced in the forward primer sequence, while the reverse primer might be conjugated at an appropriate position downstream the termination codon. PCR was conducted in a reaction system of 50 $\mu$l, by using 2 $\mu$g of P. *furiosus* DNA and 10 pmol each of the primers under conditions for LA *Taq* (manufactured by TaKaRa Brewery) and attached buffers. The cycle conditions were as follows; 93°C/3 minutes prior to the addition of the enzyme, and 30 cycles of each cycle composed of 94°C for 0.5 minute, 55°C for 0.5 minute and 72°C for 1.0 minute. The amplified DNA fragment was purified, followed by treatment with NcoI, and the resulting DNA fragment was similarly cleaved with NcoI and subsequently blunt ended, and the resulting fragment was then integrated downstream the T7 promoter of an NcoI-treated expression vector pET-15b. The expression vector was defined as pDPWT100, to confirm the nucleotide sequence of the inserted gene.

Modification of *Pfu* DNA polymerase gene:

According to the known method (Strategies, Vol.9, p.3-4, 1996) and for the expression vector pDPWT100 with the cloned *Pfu* DNA polymerase gene integrated therein, a modified *Pfu* DNA polymerase gene was prepared on the expression vector pDPWT100 by using oligopeptides containing desired mutations (SQ ID Nos.4 and 5) and the mutation induction kit manufactured by Promega Corporation, whereby an expression vector pDP320 was constructed. By determining the nucleotide sequence of the modified gene, furthermore, the amino acid sequence of the modified *Pfu* DNA polymerase (SQ ID No.1) was verified.

(2) Expression and purification of the modified *Pfu* DNA polymerase in *Escherichia coli*

The gene of the modified *Pfu* DNA polymerase I was expressed in *Escherichia coli* as follows, which was then purified.

The expression vector pDP320 with the modified *Pfu* DNA polymerase gene was inserted in *Escherichia coli* HMS174 (DE3) and cultured in an LB culture medium supplemented with IPTG to a final concentration of 0.1 mM for 14 hours, to induce the expression of the enzyme in the bacteria of the *Escherichia coli*. After recovering the bacteria by centrifugation, a modified *Pfu* DNA polymerase was extracted under ultrasonic treatment in a buffer containing 150 mM Tris/HCl (pH 7.5), 2 mM EDTA, 0.24 mM APMSF and 0.2 % Tween 20. The crude extract solution was thermally treated at 80 °C for 15 minutes, to inactivate the DNA polymerase derived from *Escherichia coli* and partially purify the DNA polymerase of the present invention. The partially purified fraction was dialyzed against a buffer composed of 50 mM Tris/HCl (pH 7.5), 1 mM EDTA, 0.2 % Tween 20, 7 mM 2-mercaptoethanol and 10 % glycerol. At the stage was detected a DNA polymerizing activity specific to the modified *Pfu* DNA polymerase I.

Example 7

By using the modified *Pfu* DNA polymerase I partially purified in Example 6, the primer elongation reaction of a DNA chain complimentary to the template DNA was tested.

One $\mu$g of the partially purified enzyme fraction described above was placed in 20 $\mu$l of a reaction solution containing 20 m Tris/HCl (pH 8.0), 2 mM MgCl$_2$, 50 $\mu$g/ml BSA, 0.1 % Triton X-100, 1 mM each of cold dNTPs (0.1 mM for dCTP), 0.63 $\mu$g of pBLUESCRIPT plasmid prepared by annealing together 10 $\mu$ Ci of [$\alpha$-$^{32}$P]dCTP and a primer of M13 (-21), for reaction at 75°C for one minute and 3 minutes. The elongated DNA chain was separated by electrophoresis on a polyacrylamide gel containing 8M urea, and the resulting pattern was analyzed with an image analyzer. As a control, additionally, the conventional wild-typ*e Pfu* DNA polymerase was used for the same DNA synthesis.

The results are shown in Fig .23. When the conventional wild-type *Pfu* DNA polymerase was used, at least 10 bands indicating the presence of incomplete DNA fragments due to synthetic termination were observed. However, these bands disappeared during the DNA synthesis with the modified *Pfu* DNA polymerase I of the present invention. Alternatively, no difference in the accumulation of highly elongated DNA fragments around 1000 bases was observed.

Example 8

The *Pfu* DNA polymerases II and III of this invention were prepared.

(1) Preparation of modified *Pfu* DNA polymerase gene

In the same manner as in Example 6(1), the *Pfu* DNA polymerase gene was cloned, to prepare modified genes II and III as follows.

Preparation of modified *Pfu* DNA polymerase II:

According to the known method (Strategies, Vol.9, p.3-4, 1996) and for the expression vector pDPWT100 with the cloned *Pfu* DNA polymerase gene integrated therein, the gene of modified *Pfu* DNA polymerase II was prepared on the expression vector pDPWT100 by using oligopeptides containing desired mutations (SQ ID Nos.8 and 9) and the mutation induction kit manufactured by Promega Corporation, whereby an expression vector pDP5b17 was constructed. By determining the nucleotide sequence of the modified gene, furthermore, the amino acid sequence of the modified *Pfu* DNA polymerase II (SQ ID No.6) was confirmed.

Preparation of the gene of modified *Pfu* DNA polymerase III:

By the same method as described above except for the use of the oligonucleotides of SQ ID Nos.10 and 11, the gene of modified *Pfu* DNA polymerase III was prepared, to construct an expression vector pDP5C4. By determining the nucleotide sequence of the modified gene, the amino acid sequence (SQ ID No.7) of the modified *Pfu* DNA polymerase III was confirmed.

(2) Expression in *Escherichia coli* and purification of the modified *Pfu* DNA polymerases II and III

The genes of the modified *Pfu* DNA polymerases II and III, thus prepared, were expressed in *Escherichia coli* as follows, which were then purified.
The expression vectors pDP5b17 and pDP5C4 were independently inserted in *Escherichia coli* HMS174 (DE3) and cultured in an LB culture medium supplemented with IPTG to a final concentration of 0.1 mM for 14 hours, to induce the expression of the enzymes in the *Escherichia coli*. After recovering the bacteria by centrifugation, modified *Pfu* DNA polymerases II and III were extracted, with ultrasonic treatment, in a buffer containing 150 mM Tris/HCl (pH 7.5), 2 mM EDTA, 0.24 mM APMSF and 0.2 % Tween 20. The crude extract solution was thermally treated at 80°C for 15 minutes, to inactivate the DNA polymerases derived from *Escherichia coli* and partially purify the modified DNA polymerases II and III. The partially purified fractions were dialyzed against a buffer composed of 50 mM Tris/HCl (pH 7.5), 1 mM EDTA, 0.2% Tween 20, 7 mM 2-mercaptoethanol and 10 % glycerol. At the stage were detected DNA polymerizing activities specific to the modified *Pfu* DNA polymerases II and III.

Example 9

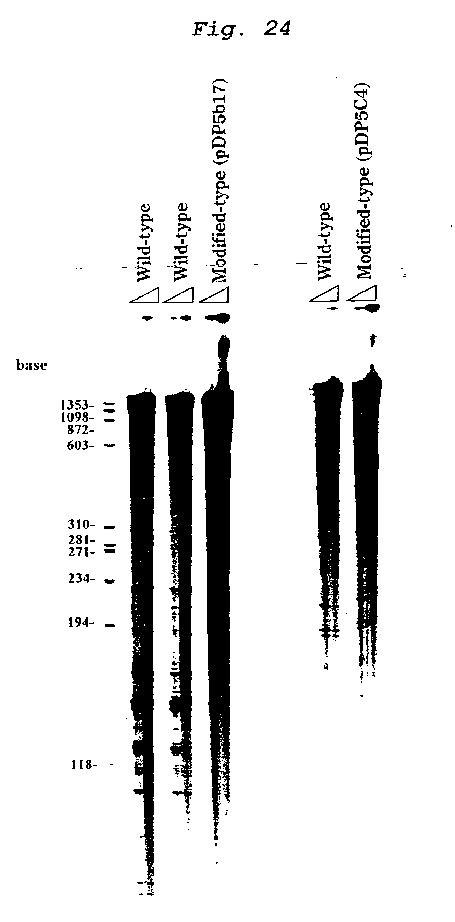

By using the modified *Pfu* DNA polymerases II and III partially purified in Example 8, the primer elongation reaction of a DNA chain complimentary to the template DNA was tested.
One $\mu$g of each of the partially purified enzyme fractions described above was placed in 20 $\mu$l of a reaction solution containing 20 mM Tris/HCl(pH 8.0), 2 mM MgCl$_2$, 50 $\mu$g/ml BSA, 0.1 % Triton X-100, 1 mM each of cold dNTPs (0.1 mM for dCTP), 0.63 $\mu$g of pBLUESCRIPT plasmid prepared by annealing together 10 $\mu$Ci of [$\alpha$-$^{32}$P]dCTP and a primer M13(-21), for reaction at 75 °C for one minute and 3 minutes. The elongated DNA chain was separated by electrophoresis on a polyacrylamide gel containing 8M urea, and the resulting pattern was analyzed with an image analyzer. As a control, additionally, the conventional wild-type *Pfu* DNA polymerase was used for the same DNA synthesis.
The results are shown in Fig.24. When the conventional wild-type *Pfu* DNA polymerase was used, bands indicating the presence of incomplete DNA fragments were observed, because of the presence of a large region at about 1000 bases where synthetic termination occurred. However, the yield of synthesized products including those of bands of about 1000 bases was elevated during the DNA synthesis with the modified *Pfu* DNA polymerases II and III of the present invention, together with bands indicating the presence of more polymeric (more elongated) PCR products under observation.

The results described above indicate that the DNA polymerases of the present invention can more markedly elongate DNA fragments in the course of synthesis during the DNA synthesis by PCR.

INDUSTRIAL APPLICABILITY

In accordance with the present invention, the functional site of a protein with no information of function which obtained bu genome analysis or cDNA analysis can be predicted. A novel functional site of a protein with a known function can also be predicted.

The thermophilic DNA polymerases provided by the present invention can highly efficiently synthesize and amplify the full length of a DNA fragment by PCR, whereby in vitro synthesis and amplification of a DNA fragment and the nucleotide sequencing thereof can be attained at a high precision in a simple manner.

SEQUENCE LISTING

SEQ ID NO.: 1
LENGTH: 775
TYPE: amino acid
MOLECULE TYPE: protein
SEQUENCE

Met Val Leu Asp Val Asp Tyr Ile Thr Glu Glu Gly Lys Pro Val Ile
1               5                   10                  15

Arg Leu Phe Lys Lys Glu Asn Gly Lys Phe Lys Ile Glu His Asp Arg
                20                  25                  30

Thr Phe Arg Pro Tyr Ile Tyr Ala Leu Leu Arg Asp Asp Ser Lys Ile
                35                  40                  45

Glu Glu Val Lys Lys Ile Thr Gly Glu Arg His Gly Lys Ile Val Arg
                50                  55                  60

Ile Val Asp Val Glu Lys Val Glu Lys Lys Phe Leu Gly Lys Pro Ile
65                  70                  75                  80

Thr Val Trp Lys Leu Tyr Leu Glu His Pro Gln Asp Val Pro Thr Ile
                85                  90                  95

Arg Glu Lys Val Arg Glu His Pro Ala Val Val Asp Ile Phe Glu Tyr
                100                 105                 110

Asp Ile Pro Phe Ala Lys Arg Tyr Leu Ile Asp Lys Gly Leu Ile Pro
                115                 120                 125

Met Glu Gly Glu Glu Glu Leu Lys Ile Leu Ala Phe Asp Ile Glu Thr
                130                 135                 140

Leu Tyr His Glu Gly Glu Glu Phe Gly Lys Gly Pro Ile Ile Met Ile

                145                          150                          155                          160

Ser Tyr Ala Asp Glu Asn Glu Ala Lys Val Ile Thr Trp Lys Asn Ile
                          165                          170                          175

Asp Leu Pro Tyr Val Glu Val Val Ser Ser Glu Arg Glu Met Ile Lys
                          180                          185                          190

Arg Phe Leu Arg Ile Ile Arg Glu Lys Asp Pro Asp Ile Ile Val Thr
                     195                          200                          205

Tyr Asn Gly Asp Ser Phe Asp Phe Pro Tyr Leu Ala Lys Arg Ala Glu
                210                          215                          220

Lys Leu Gly Ile Lys Leu Thr Ile Gly Arg Asp Gly Ser Glu Pro Lys
225                          230                          235                          240

Met Gln Arg Ile Gly Asp Met Thr Ala Val Glu Val Lys Gly Arg Ile
                          245                          250                          255

His Phe Asp Leu Tyr His Val Ile Thr Arg Thr Ile Asn Leu Pro Thr
                     260                          265                          270

Tyr Thr Leu Glu Ala Val Tyr Glu Ala Ile Phe Gly Lys Pro Lys Glu
                     275                          280                          285

Lys Val Tyr Ala Asp Glu Ile Ala Lys Ala Trp Glu Ser Gly Glu Asn
                290                          295                          300

Leu Glu Arg Val Ala Lys Tyr Ser Met Glu Asp Ala Lys Ala Thr Tyr
305                          310                          315                          320

Glu Leu Gly Lys Glu Phe Leu Pro Met Glu Ile Gln Leu Ser Arg Leu
                          325                          330                          335

Val Gly Gln Pro Leu Trp Asp Val Ser Arg Ser Ser Thr Gly Asn Leu
                     340                          345                          350

Val Glu Trp Phe Leu Leu Arg Lys Ala Tyr Glu Arg Asn Glu Val Ala
                355                 360                 365

Pro Asn Lys Pro Ser Glu Glu Glu Tyr Gln Arg Arg Leu Arg Glu Ser
        370                 375                 380

Tyr Thr Gly Gly Phe Val Lys Glu Pro Glu Lys Gly Leu Trp Glu Asn
385                 390                 395                 400

Ile Val Tyr Leu Asp Phe Arg Ala Leu Tyr Pro Ser Ile Ile Ile Thr
                405                 410                 415

His Asn Val Ser Pro Asp Thr Leu Asn Leu Glu Gly Cys Lys Asn Tyr
                420                 425                 430

Asp Ile Ala Pro Gln Val Gly His Lys Phe Cys Lys Asp Ile Pro Gly
        435                 440                 445

Phe Ile Pro Ser Leu Leu Gly His Leu Leu Glu Glu Arg Gln Lys Ile
        450                 455                 460

Lys Thr Lys Met Lys Glu Thr Gln Asp Pro Ile Glu Lys Ile Leu Leu
465                 470                 475                 480

Asp Tyr Arg Gln Lys Ala Ile Lys Leu Leu Ala Asn Ser Phe Tyr Gly
                485                 490                 495

Tyr Tyr Gly Tyr Ala Lys Ala Arg Trp Tyr Cys Lys Glu Cys Ala Glu
                500                 505                 510

Ser Val Thr Ala Trp Gly Arg Lys Tyr Ile Glu Leu Val Trp Lys Glu
        515                 520                 525

Leu Glu Glu Lys Tyr Gly Phe Lys Val Ile Tyr Ser Asp Thr Asp Gly
        530                 535                 540

Phe Phe Ala Thr Ile Pro Gly Gly Glu Ser Glu Glu Ile Lys Lys Lys

545          550          555          560

Ala Leu Glu Phe Val Lys Tyr Ile Asn Ser Lys Leu Pro Gly Leu Leu

565          570          575

Glu Leu Glu Tyr Glu Gly Phe Tyr Lys Arg Gly Phe Phe Val Thr Lys

580          585          590

Lys Arg Tyr Ala Val Ile Asp Glu Glu Gly Lys Val Ile Thr Arg Gly

595          600          605

Leu Glu Ile Val Arg Arg Asp Trp Ser Glu Ile Ala Lys Glu Thr Gln

610          615          620

Ala Arg Val Leu Glu Thr Ile Leu Lys His Gly Asp Val Glu Glu Ala

625          630          635          640

Val Arg Ile Val Lys Glu Val Ile Gln Lys Leu Ala Asn Tyr Glu Ile

645          650          655

Pro Pro Glu Lys Leu Ala Ile Tyr Glu Gln Ile Thr Arg Pro Leu His

660          665          670

Glu Tyr Lys Ala Ile Gly Pro His Val Ala Val Ala Lys Lys Leu Ala

675          680          685

Ala Lys Gly Val Lys Ile Lys Pro Gly Met Val Ile Gly Tyr Ile Val

690          695          700

Leu Arg Gly Asp Gly Pro Ile Ser Asn Arg Ala Ile Leu Ala Glu Glu

705          710          715          720

Tyr Asp Pro Lys Lys His Lys Tyr Asp Ala Glu Tyr Tyr Ile Glu Asn

725          730          735

Gln Val Leu Pro Ala Val Leu Arg Ile Leu Glu Gly Phe Gly Tyr Arg

740          745          750

Lys Glu Asp Leu Arg Tyr Gln Lys Thr Arg Gln Val Gly Leu Thr Ser

755     760     765

Trp Leu Asn Ile Lys Lys Ser

770    775

SEQ ID NO.: 2
LENGTH: 35
TYPE: nucleic acid
STRANDEDNESS: single
TOPOLOGY: linear
MOLECULE TYPE: synthetic DNA
SEQUENCE
GTGGGGAGCA CCATGGTTTT AGATGTGGAT TACAT  35

SEQ ID NO.: 3
LENGTH: 35
TYPE: nucleic acid
STRANDEDNESS: single
TOPOLOGY: linear
MOLECULE TYPE: synthetic DNA
SEQUENCE
GCATGCAGAT AGACCATTTC TAACGAAGGC GTTTG  35

SEQ ID NO.: 4
LENGTH: 66
TYPE: nucleic acid
STRANDEDNESS: single
TOPOLOGY: linear
MOLECULE TYPE: synthetic DNA
SEQUENCE

CTCGAAGAAA AGTATGGATT TAAAGTCATC TACAGTGACA CTGATGGTTT CTTTGCAACT 60

ATCCCA                66

SEQ ID NO.: 5
LENGTH: 66
TYPE: nucleic acid
STRANDEDNESS: single
TOPOLOGY: linear
MOLECULE TYPE: synthetic DNA
SEQUENCE

TGGGATAGTT GCAAAGAAAC CATCAGTGTC ACTGTAGATG ACTTTAAATC CATACTTTTC 60

TTCGAG                66

SEQ ID NO.: 6
LENGTH: 775
TYPE: amino acid
MOLECULE TYPE: protein
SEQUENCE

Met Val Leu Asp Val Asp Tyr Ile Thr Glu Glu Gly Lys Pro Val Ile

1               5                     10                    15

Arg Leu Phe Lys Lys Glu Asn Gly Lys Phe Lys Ile Glu His Asp Arg
                20                  25                  30

Thr Phe Arg Pro Tyr Ile Tyr Ala Leu Leu Arg Asp Asp Ser Lys Ile
            35                  40                  45

Glu Glu Val Lys Lys Ile Thr Gly Glu Arg His Gly Lys Ile Val Arg
        50                  55                  60

Ile Val Asp Val Glu Lys Val Glu Lys Lys Phe Leu Gly Lys Pro Ile
65                  70                  75                  80

Thr Val Trp Lys Leu Tyr Leu Glu His Pro Gln Asp Val Pro Thr Ile
                85                  90                  95

Arg Glu Lys Val Arg Glu His Pro Ala Val Val Asp Ile Phe Glu Tyr
                100                 105                 110

Asp Ile Pro Phe Ala Lys Arg Tyr Leu Ile Asp Lys Gly Leu Ile Pro
                115                 120                 125

Met Glu Gly Glu Glu Glu Leu Lys Ile Leu Ala Phe Asp Ile Glu Thr
                130                 135                 140

Leu Tyr His Glu Gly Glu Glu Phe Gly Lys Gly Pro Ile Ile Met Ile
145                 150                 155                 160

Ser Tyr Ala Asp Glu Asn Glu Ala Lys Val Ile Thr Trp Lys Asn Ile
                165                 170                 175

Asp Leu Pro Tyr Val Glu Val Val Ser Ser Glu Arg Glu Met Ile Lys
                180                 185                 190

Arg Phe Leu Arg Ile Ile Arg Glu Lys Asp Pro Asp Ile Ile Val Thr
                195                 200                 205

Tyr Asn Gly Asp Ser Phe Asp Phe Pro Tyr Leu Ala Lys Arg Ala Glu

$$\begin{array}{ccccc}
210 & & 215 & & 220
\end{array}$$

Lys Leu Gly Ile Lys Leu Thr Ile Gly Arg Asp Gly Ser Glu Pro Lys

$$\begin{array}{cccc}
225 & 230 & 235 & 240
\end{array}$$

Met Gln Arg Ile Gly Asp Met Thr Ala Val Glu Val Lys Gly Arg Ile

$$\begin{array}{ccc}
245 & 250 & 255
\end{array}$$

His Phe Asp Leu Tyr His Val Ile Thr Arg Thr Ile Asn Leu Pro Thr

$$\begin{array}{ccc}
260 & 265 & 270
\end{array}$$

Tyr Thr Leu Glu Ala Val Tyr Glu Ala Ile Phe Gly Lys Pro Lys Glu

$$\begin{array}{ccc}
275 & 280 & 285
\end{array}$$

Lys Val Tyr Ala Asp Glu Ile Ala Lys Ala Trp Glu Ser Gly Glu Asn

$$\begin{array}{ccc}
290 & 295 & 300
\end{array}$$

Leu Glu Arg Val Ala Lys Tyr Ser Met Glu Asp Ala Lys Ala Thr Tyr

$$\begin{array}{cccc}
305 & 310 & 315 & 320
\end{array}$$

Glu Leu Gly Lys Glu Phe Leu Pro Met Glu Ile Gln Leu Ser Arg Leu

$$\begin{array}{ccc}
325 & 330 & 335
\end{array}$$

Val Gly Gln Pro Leu Trp Asp Val Ser Arg Ser Ser Thr Gly Asn Leu

$$\begin{array}{ccc}
340 & 345 & 350
\end{array}$$

Val Glu Trp Phe Leu Leu Arg Lys Ala Tyr Glu Arg Asn Glu Val Ala

$$\begin{array}{ccc}
355 & 360 & 365
\end{array}$$

Pro Asn Lys Pro Ser Glu Glu Glu Tyr Gln Arg Arg Leu Arg Glu Ser

$$\begin{array}{ccc}
370 & 375 & 380
\end{array}$$

Tyr Thr Gly Gly Phe Val Lys Glu Pro Glu Lys Gly Leu Trp Glu Asn

$$\begin{array}{cccc}
385 & 390 & 395 & 400
\end{array}$$

Ile Val Tyr Leu Asp Phe Arg Ala Leu Tyr Pro Ser Ile Ile Ile Thr

$$\begin{array}{ccc}
405 & 410 & 415
\end{array}$$

His Asn Val Ser Pro Asp Thr Leu Asn Leu Glu Gly Cys Lys Asn Tyr
                420                 425                 430

Asp Ile Ala Pro Gln Val Gly His Lys Phe Cys Lys Asp Ile Pro Gly
            435                 440                 445

Phe Ile Pro Ser Leu Leu Gly His Leu Leu Glu Glu Arg Gln Lys Ile
        450                 455                 460

Lys Thr Lys Met Lys Glu Thr Gln Asp Pro Ile Glu Lys Ile Leu Leu
465                 470                 475                 480

Asp Tyr Arg Gln Lys Ala Ile Lys Leu Leu Ala Asn Ser Phe Tyr Gly
            485                 490                 495

Tyr Tyr Gly Tyr Ala Lys Ala Arg Trp Tyr Cys Lys Glu Cys Ala Glu
            500                 505                 510

Ser Val Thr Ala Trp Gly Arg Lys Tyr Ile Glu Leu Val Trp Lys Glu
            515                 520                 525

Leu Glu Glu Lys Phe Gly Phe Lys Val Leu Tyr Ile Asp Thr Asp Gly
        530                 535                 540

Leu Tyr Ala Thr Ile Pro Gly Gly Glu Ser Glu Glu Ile Lys Lys Lys
545                 550                 ·555                560

Ala Leu Glu Phe Val Lys Tyr Ile Asn Ser Lys Leu Pro Gly Leu Leu
            565                 570                 575

Glu Leu Glu Tyr Glu Gly Phe Tyr Lys Arg Gly Phe Phe Val Thr Lys
            580                 585                 590

Lys Arg Tyr Ala Val Ile Asp Glu Glu Gly Lys Val Ile Thr Arg Gly
            595                 600                 605

Leu Glu Ile Val Arg Arg Asp Trp Ser Glu Ile Ala Lys Glu Thr Gln

610                    615                      620

Ala Arg Val Leu Glu Thr Ile Leu Lys His Gly Asp Val Glu Glu Ala

625                 630                635                     640

Val Arg Ile Val Lys Glu Val Ile Gln Lys Leu Ala Asn Tyr Glu Ile

645                 650                655

Pro Pro Glu Lys Leu Ala Ile Tyr Glu Gln Ile Thr Arg Pro Leu His

660                 665                670

Glu Tyr Lys Ala Ile Gly Pro His Val Ala Val Ala Lys Lys Leu Ala

675                 680                685

Ala Lys Gly Val Lys Ile Lys Pro Gly Met Val Ile Gly Tyr Ile Val

690                 695                700

Leu Arg Gly Asp Gly Arg Ile Arg Asp Arg Ala Ile Pro Ala Glu Glu

705                 710                715                     720

Tyr Asp Pro Lys Lys His Lys Tyr Asp Ala Glu Tyr Tyr Ile Glu Asn

725                 730                735

Gln Val Leu Pro Ala Val Leu Arg Ile Leu Glu Gly Phe Gly Tyr Arg

740                 745                750

Lys Glu Asp Leu Arg Tyr Gln Lys Thr Arg Gln Val Gly Leu Thr Ser

755                 760                765

Trp Leu Asn Ile Lys Lys Ser

770                 775

SEQ ID NO.: 7
LENGTH: 775
TYPE: amino acid
MOLECULE TYPE: protein
SEQUENCE

34

Met Val Leu Asp Val Asp Tyr Ile Thr Glu Glu Gly Lys Pro Val Ile
1 5 10 15

Arg Leu Phe Lys Lys Glu Asn Gly Lys Phe Lys Ile Glu His Asp Arg
20 25 30

Thr Phe Arg Pro Tyr Ile Tyr Ala Leu Leu Arg Asp Asp Ser Lys Ile
35 40 45

Glu Glu Val Lys Lys Ile Thr Gly Glu Arg His Gly Lys Ile Val Arg
50 55 60

Ile Val Asp Val Glu Lys Val Glu Lys Lys Phe Leu Gly Lys Pro Ile
65 70 75 80

Thr Val Trp Lys Leu Tyr Leu Glu His Pro Gln Asp Val Pro Thr Ile
85 90 95

Arg Glu Lys Val Arg Glu His Pro Ala Val Val Asp Ile Phe Glu Tyr
100 105 110

Asp Ile Pro Phe Ala Lys Arg Tyr Leu Ile Asp Lys Gly Leu Ile Pro
115 120 125

Met Glu Gly Glu Glu Glu Leu Lys Ile Leu Ala Phe Asp Ile Glu Thr
130 135 140

Leu Tyr His Glu Gly Glu Glu Phe Gly Lys Gly Pro Ile Ile Met Ile
145 150 155 160

Ser Tyr Ala Asp Glu Asn Glu Ala Lys Val Ile Thr Trp Lys Asn Ile
165 170 175

Asp Leu Pro Tyr Val Glu Val Val Ser Ser Glu Arg Glu Met Ile Lys

                    180                      185                      190

Arg Phe Leu Arg Ile Ile Arg Glu Lys Asp Pro Asp Ile Ile Val Thr
         195                      200                      205

Tyr Asn Gly Asp Ser Phe Asp Phe Pro Tyr Leu Ala Lys Arg Ala Glu
         210                      215                      220

Lys Leu Gly Ile Lys Leu Thr Ile Gly Arg Asp Gly Ser Glu Pro Lys
225                      230                      235                      240

Met Gln Arg Ile Gly Asp Met Thr Ala Val Glu Val Lys Gly Arg Ile
                  245                      250                      255

His Phe Asp Leu Tyr His Val Ile Thr Arg Thr Ile Asn Leu Pro Thr
                  260                      265                      270

Tyr Thr Leu Glu Ala Val Tyr Glu Ala Ile Phe Gly Lys Pro Lys Glu
                  275                      280                      285

Lys Val Tyr Ala Asp Glu Ile Ala Lys Ala Trp Glu Ser Gly Glu Asn
         290                      295                      300

Leu Glu Arg Val Ala Lys Tyr Ser Met Glu Asp Ala Lys Ala Thr Tyr
305                      310                      315                      320

Glu Leu Gly Lys Glu Phe Leu Pro Met Glu Ile Gln Leu Ser Arg Leu
                  325                      330                      335

Val Gly Gln Pro Leu Trp Asp Val Ser Arg Ser Ser Thr Gly Asn Leu
                  340                      345                      350

Val Glu Trp Phe Leu Leu Arg Lys Ala Tyr Glu Arg Asn Glu Val Ala
         355                      360                      365

Pro Asn Lys Pro Ser Glu Glu Glu Tyr Gln Arg Arg Leu Arg Glu Ser
         370                      375                      380

36

Tyr Thr Gly Gly Phe Val Lys Glu Pro Glu Lys Gly Leu Trp Glu Asn
385                390                395                400

Ile Val Tyr Leu Asp Phe Arg Ala Leu Tyr Pro Ser Ile Ile Ile Thr
              405                410                415

His Asn Val Ser Pro Asp Thr Leu Asn Leu Glu Gly Cys Lys Asn Tyr
          420                425                430

Asp Ile Ala Pro Gln Val Gly His Lys Phe Cys Lys Asp Ile Pro Gly
          435                440                445

Phe Ile Pro Ser Leu Leu Gly His Leu Leu Glu Glu Arg Gln Lys Ile
      450                455                460

Lys Thr Lys Met Lys Glu Thr Gln Asp Pro Ile Glu Lys Ile Leu Leu
465                470                475                480

Asp Tyr Arg Gln Lys Ala Ile Lys Leu Leu Ala Asn Ser Phe Tyr Gly
              485                490                495

Tyr Tyr Gly Tyr Ala Lys Ala Arg Trp Tyr Cys Lys Glu Cys Ala Glu
          500                505                510

Ser Val Thr Ala Trp Gly Arg Lys Tyr Ile Glu Leu Val Trp Lys Glu
          515                520                525

Leu Glu Glu Lys Phe Gly Phe Lys Val Leu Tyr Ile Asp Thr Asp Gly
      530                535                540

Leu Tyr Ala Thr Ile Pro Gly Gly Glu Ser Glu Glu Ile Lys Lys Lys
545                550                555                560

Ala Leu Glu Phe Val Lys Tyr Ile Asn Ser Lys Leu Pro Gly Leu Leu
              565                570                575

Glu Leu Glu Tyr Glu Gly Phe Tyr Lys Arg Gly Phe Phe Val Thr Lys

37

580                          585                          590

Lys Arg Tyr Ala Val Ile Asp Glu Glu Gly Lys Val Ile Thr Arg Gly

595                          600                          605

Leu Glu Ile Val Arg Arg Asp Trp Ser Glu Ile Ala Lys Glu Thr Gln

610                          615                          620

Ala Arg Val Leu Glu Thr Ile Leu Lys His Gly Asp Val Glu Glu Ala

625                 630                          635                 640

Val Arg Ile Val Lys Glu Val Ile Gln Lys Leu Ala Asn Tyr Glu Ile

645                          650                          655

Pro Pro Glu Lys Leu Ala Ile Tyr Glu Gln Ile Thr Arg Pro Leu His

660                          665                          670

Glu Tyr Lys Ala Ile Gly Pro His Val Ala Val Ala Lys Lys Leu Ala

675                          680                          685

Ala Lys Gly Val Lys Ile Lys Pro Gly Met Val Ile Gly Tyr Ile Val

690                          695                          700

Leu Arg Gly Asp Gly Pro Ile Ser Asn Arg Ala Ile Pro Ala Glu Glu

705                 710                          715                 720

Tyr Asp Pro Lys Lys His Lys Tyr Asp Ala Glu Tyr Tyr Ile Glu Asn

725                          730                          735

Gln Val Leu Pro Ala Val Leu Arg Ile Leu Glu Gly Phe Gly Tyr Arg

740                          745                          750

Lys Glu Asp Leu Arg Tyr Gln Lys Thr Arg Gln Val Gly Leu Thr Ser

755                          760                          765

Trp Leu Asn Ile Lys Lys Ser

770                          775

SEQ ID NO.: 8

LENGTH: 49
TYPE: nucleic acid
STRANDEDNESS: single
TOPOLOGY: linear
MOLECULE TYPE: synthetic DNA
SEQUENCE
AGAGGCGATG GTCGAATTCG CGATAGGGCA ATTCCAGCTG AGGAATACG       49

SEQ ID NO.: 9
LENGTH: 49
TYPE: nucleic acid
STRANDEDNESS: single
TOPOLOGY: linear
MOLECULE TYPE: synthetic DNA
SEQUENCE
CGTATTCCTC AGCTGGAATT GCCCTATCGC GAATTCGACC ATCGCCTCT       49

SEQ ID NO.: 10
LENGTH: 40
**TYPE:** nucleic acid
STRANDEDNESS: single
TOPOLOGY: linear
MOLECULE TYPE: synthetic DNA
SEQUENCE
CCAATTAGCA ATAGGGCAAT TCCAGCTGAG GAATACGATC       40

SEQ ID NO.: 11
LENGTH: 40
TYPE: nucleic acid
STRANDEDNESS: single
TOPOLOGY: linear
MOLECULE TYPE: synthetic DNA
SEQUENCE
GATCGTATTC CTCAGCTGGA ATTGCCCTAT TGCTAATTGG       40

**Claims**

1.  A system for automatically conducting a method for predicting a functional site of a protein derived from the entire putative proteins of an organism "*a*" of which genome data or cDNA data is known, which method comprises the steps of:

    (1) calculating the frequency of occurrence of each amino acid and the frequency of occurrence of individual oligopeptides produced by permutations of twenty amino acids in the amino acid sequences of the entire proteins of the organism "*a*", and determining the smallest length (n) of oligopeptides satisfying the following criteria; among oligopeptides of length (n), the number of oligopeptides which occur once in the entire proteins is smaller than the number of oligopeptides which occur twice in the entire proteins; among oligopeptides of length (n+1), the number of oligopeptides which occur once in the entire proteins is larger than the number of oligopeptides which occur twice in the entire proteins,
    (2) calculating in the entire proteins of the organism "*a*", the frequency of occurrence of the following Aj-oligopeptide of length (n+1), which is a part of the amino acid sequence of length (L) of the protein as a subject for predicting a functional site and contains an amino-acid residue Aj (n+1 $\leq$j$\leq$L-1) at the j-th position from the N-terminus of the amino acid sequence of the protein;
    Aj-oligopeptide: aj1aj2....Aji....ajnaj(n+1) (wherein, 1$\leq$n$\leq$n+1; Aj=Aji; and Aj is the i-th residue of the oligopeptide), and calculating in the entire proteins of the organism "*a*", the frequency of occurrence of the following Xi-oligopeptide of length (n+1);
    Xi-oligopeptide: ajlaj2....Xi....ajnaj(n+1) (wherein, the i-th residue Xi is any amino acid),
    (3) calculating ratio Yji of the frequency of occurrence of the Aj-oligopeptide to that of the Xi-oligopeptide,
    (4) calculating mean Yj of the Yji;

$$Yj = \sum_{i=1}^{n+1} Yji/(n+1),$$

    (5) calculating functional value Zj of Yj;

$$Zj = f(Yj)$$

    (wherein, function f is a monotonously decreasing function or a monotonously increasing function), and defining the Zj value as a representative value of the function of the j-th amino-acid residue Aj of the amino acid sequence of length (L), and
    (6) repeating the steps (2) to (5) sequentially and determining the Zj value of each Aj of all the amino-acid residues at the positions between n+1$\leq$j$\leq$L-n in the amino acid sequence of length (L), thereby predicting the degree of the involvement of each amino-acid residue in the function of the protein by using the dimension of the Zj value as an indicator, said sytem at least comprising the following units (a) to (g);

    (a) an outer memory unit memorizing the amino acid sequence data of the entire putative proteins of organism "$\alpha$" of which genome data or cDNA data is known, as well as an existing protein data base,

(b) a calculation/memory unit, composed of CPU calculating the frequency of occurrence of each amino acid and the frequency of occurrence of individual oligopeptides produced by permutations of twenty amino acids, in the amino acid sequences of the entire proteins of the organism "*a*", and a memory unit having the memory of the calculation results,

(c) a calculation/memory unit, composed of CPU calculating the smallest length (n) of oligopeptides satisfying the following criteria among the individual oligopeptides of which the frequencies of the occurrences being memorized in the unit (b) ;

among oligopeptides of length (n), the number of oligopeptides which occur once in the entire proteins is smaller than the number of oligopeptides which occur twice in the entire proteins; among oligopeptides of length (n+1), the number of oligopeptides which occur once in the entire proteins is larger than the number of oligopeptides which occur twice in the entire proteins,

and a memory unit having the memory of the (n),

(d) a calculation/memory unit, composed of CPU calculating in the entire proteins of the organism "*a*", the frequency of occurrence of the following Aj-oligopeptide of length (n+1), which is a part of the amino acid sequence of length of (L) of the protein as a subject for predicting a functional site and contains an amino-acid residue Aj (n+1≤j≤L-n) at the j-th position from the N-terminus of the amino acid sequence of the protein;

Aj-oligopeptide: aj1aj2....Aji....ajnaj(n+1)

(wherein, 1≤i≤n+1 ; Aj = Aji; and Aj is the i-th residue of the oligopeptide),

and calculating in the entire proteins of the organism "*a*", the frequency of occurrence of the following Xi-oligopeptide of length (n+1);

Xi-oligopeptide: aj1aj2....Xji....ajnaj(n+1)

(wherein, the i-th residue Xji is any amino acid),

and a memory unit having the memory of the calculation results,

(e) a calculation/memory unit, composed of CPU calculating ratio Yji of the frequency of occurrence of the Aj-oligopeptide to that of the Xi-oligopeptide, and a memory unit having the memory of Yji,

(f) a calculation/memory unit, composed of CPU calculating mean Yj of the Yji;

$$Yj = \sum_{i=1}^{n+1} Yji/(n+1),$$

and a memory unit having the memory of Yj, and

(g) a calculation/memory unit, composed of CPU calculating functional value Zj of Yj;

$$Zj = f(Yj)$$

(wherein, function f is a monotonously decreasing function or a monotonously increasing function),

and a memory unit having the memory of Zj.

2. The system according to claim 1 wherein in the method conducted on the system the Z j value (n+ 1≤j≤L-n) of each amino-acid residue in the amino acid sequence of length (L) is expressed in a distribution chart.

3. The system according to claim 1, which further comprises a display unit displaying the Zj value (n+1≤j≤L-n) of each amino-acid residue in the amino acid sequence of length (L) in a distribution chart.

4. A system for automatically conducting a method for predicting a functional site of a protein derived from the entire putative proteins of an organism "*a*" of which genome data or cDNA data is known, which method comprises the steps of:

(1) calculating the frequency of occurrence of each amino acid and the frequency of occurrence of individual oligopeptides produced by permutations of twenty amino acids in the amino acid sequences of the entire proteins of the organism *"a"*,

(2) as to a protein of the organism *"a"*,

(2') calculating in the entire proteins of the organism "*a*", the frequency of occurrence of the following Aj-oligopeptide of given length of (n) (1≤n≤M, provided that M is the smallest length of oligopeptides satisfying the criterion that all the oligopeptides of length M are at frequency 1 of the occurrence), which Aj-oligopeptide is a part of the amino acid sequence of length (L) of the protein and contains an amino-acid residue Aj (n≤j≤L-n+1) at the j-th position from the N-terminus of the amino acid sequence of the protein;

Aj-oligopeptide: ajlaj2....aji....ajn (wherein, i≤i≤n+1; Aj=aji, and Aj is the i-th residue of the oligopeptide), and calculating in the entire proteins of the organism "a", the frequency of occurrence of the following Xi-oligopeptide of length (n) corresponding to the length of Aj-oligopeptide;

Xi-oligopeptide: aj1aj2....Xi.... ajn

(wherein, the i-th residue Xi is any amino acid),

(3) calculating ratio Yji of the frequency of occurrence of the Aj-oligopeptide to that of the Xi-oligopeptide,

(4) calculating mean Y(j,n) of the Yji;

$$Y(j,n) = \sum_{i=1}^{n} Yji/n,$$

(5) calculating functional value Z(j,n) of Y(j,n);

(6) repeating the steps (2') to (5) sequentially and determining the Z(j,n) value of each amino-acid residue Aj at the j-th position (n≤j≤L-n+1) in the amino acid sequence of length (L),

(7) sequentially repeating the steps (2) to (6) for the entire proteins of the organism "a", thereby determining the distribution of the Z(j,n) value of each amino-acid residue in the entire proteins, and the Z(j,n) values are classified into twenty according to the twenty amino acids, and then calculating mean Av(Aa) of the Z(j,n) values for each amino acid Aa and the standard deviation Sd (Aa) of the distribution thereof, on the basis of the distribution, to calculate a function g to the j-th amino-acid residue Aj of a protein for normalizing the difference in distribution due to the species of amino-acid residues;

$$g = (Z(j,n), Aj) = [Z(j,n) - Av(Aa)]/Sd(Aa)$$

(wherein, Aj = Aa),

(8) calculating a value D(j,n) of the function g of each Aj of all the amino-acid residues at the j-th position (n≤j≤L-n+1) of a protein in the entire proteins as recovered in the step (7);

$$D(j,n) = g(Z(j,n), Aj),$$

and

(9) defining the representative value of the function of the j-th amino-acid residue in the amino acid sequence of length (L) as a functional value Wj of the Z(j,n) and D(j,n);

$$Wj=h(Z(j,l),Z(j,2),..,Z(j,M),D(j,l),D(j,2),...,D(j,M))$$

thereby predicting the degree of the involvement of each amino-acid residue in the function of the protein by using the dimension of the Wj value as an indicator, said system at least comprising the following units (a) to (i) ;

(a) an outer memory unit memorizing the amino acid sequence data of the entire putative proteins of organism "a" of which genome data or cDNA data is known, as well as an existing protein data base,

(b) a calculation/memory unit, composed of CPU calculating the frequency of occurrence of each amino acid and the frequency of occurrence of individual oligopeptides produced by permutations of twenty amino acids in the amino acid sequences of the entire proteins of the organism *"a",* and a memory unit having the memory of the calculation results,

(c) a calculation/memory unit, composed of CPU calculating in the entire proteins of the organism *"a",* the frequency of occurrence of the following Aj-oligopeptide of given length of (n) (1≤n≤M, provided that M is the smallest length of oligopeptides satisfying the criterion that all the oligopeptides of length M are at frequency 1 of the occurrence), which Aj-oligopeptide is a part of the amino acid sequence of length (L) of a given protein of the organism *"a"* and contains an amino-acid residue Aj (n≤j≤L-n+1) at the j-th position from N-terminus of the amino acid sequence of the protein;

Aj-oligopeptide: aj1aj2....aji....ajn

(wherein, 1≤i≤n+1; Aj=aji, and Aj is the i-th residue of the oligopeptide),

and calculating in the entire proteins of the organism *"a"*, the frequency of occurrence of the following Xi-oligopeptide of length (n) corresponding to the length of Aj-oligopeptide;

Xi-oligopeptide: aj1aj2....Xji....ajn

(wherein, the i-th residue Xji is any amino acid),

and a memory unit memorizing the calculation results,

(d) a calculation/memory unit, composed of CPU calculating ratio Yji of the frequency of occurrence of the Aj-oligopeptide to that of the Xi-oligopeptide, and a memory unit having the memory of the Yji,

(e) a calculation/memory unit, composed of CPU calculating mean Y(j,n) of the Yji;

$$Y(j,n) = \sum_{i=1}^{n} Yji/n,$$

and a memory unit having the memory of Y(j,n),

(f) a calculation/memory unit, composed of CPU calculating functional value Z(j,n) of Y(j,n);

$$Z(j,n) = -\log(Y(j,n)),$$

and a memory unit having the memory of Z(j,n),

(g) a calculation/memory unit, composed of CPU calculating the Z(j,n) value of each amino-acid residue in the amino acid sequences of the entire proteins of the organism "*a*", calculating the distribution of the Z(j,n) value of each amino-acid residue in the entire proteins, and the Z(j,n) values are classified into twenty according to the twenty amino acids, and then calculating mean Av(Aa) of the Z(j,n) values for each amino acid Aa and the standard deviation Sd (Aa) of the distribution thereof, on the basis of the distribution, to determine function g for normalizing the difference in distribution due to the species of amino-acid residues;

$$g = (Z(j,n), Aj) = [Z(j,n) - Av(Aa)]/Sd(Aa)$$

(wherein, Aj = Aa)

and a memory unit having the memory of g,

(h) a calculation/memory unit, composed of CPU calculating value D (j,n) of function g memorized in the unit (g) concerning each of all the amino-acid residues Aj at the j-th position (n≤j≤L-n+1) in the amino acid sequence of length (L);

$$D(j,n) = g(Z(j,n), Aj)$$

and a memory unit having the memory of the D(j , n) value, and

(i) a calculation/memory unit, composed of a calculation unit calculating an appropriate functional value Wj of the Z(j,n) and D(j,n) of each amino-acid residue in the amino acid sequence;

$$Wj = h(Z(j,l),Z(j,2),..,Z(j,M),D(j,l),D(j,2),..,D(j,M))$$

and a memory unit having the memory of the Wj value.

5. The system according to claim 4 wherein in the method conducted on the system the Wj value of each amino-acid residue is expressed in a two-dimensional distribution chart.

6. The system according to claim 4 wherein in the method conducted on the system the Wj value of each amino-acid residue is expressed on a three-dimensional structure model of the protein.

7. The system according to claim 4, which further comprises a display unit displaying the Wj value of each amino-acid residue in a two-dimensional distribution chart.

8. The system according to claim 4, which further comprises a calculation/memory unit memorizing an existing database of three-dimensional structure of proteins or preparing a three-dimensional structure model based on an amino acid sequence according to a known method and memorizing the three-dimensional structure model, and a display unit displaying the Wj value of each amino-acid residue in the amino acid sequence in a distribution chart on the three-dimensional structure stored in the data base or three-dimensional structure model memorized in the calculation/memory unit.

9. A method for modifying the known function of protein *"A"* derived from the entire proteins of organism "*a*" of which genome data or cDNA data has been known, which method comprises the steps of:

(1) extracting a protein closely related to the protein "*A*" from an existing protein data base and subjecting the proteins to alignment,
(2) calculating the frequency of occurrence of each amino acid and the frequency of occurrence of individual oligopeptides produced by permutations of twenty amino acids in the amino acid sequences of the entire proteins of the organism *"a"*, and determining the smallest length (n) of oligopeptides satisfying the following criteria; among oligopeptides of length (n), the number of oligopeptides which occur once in the entire proteins is smaller than the number of oligopeptides which occur twice in the entire proteins; among oligopeptides of length (n+1), the number of oligopeptides which occur once in the entire proteins is larger than the number of oligopeptides which occur twice in the entire proteins,
(3) calculating in the entire proteins of the organism "*a*", the frequency of occurrence of the following Aj -oligopeptide of length (n+1), which is a part of the amino acid sequence of length (L) of the protein "*A*" and contains an amino-acid residue Aj (n+1≤j≤L-n) at the j-th position from the N-terminus of the amino acid sequence of the protein;
Aj-oligopeptide: aj1aj2....Aji....ajnaj(n+1)
(wherein, 1≤i≤n+1; Aj=Aji; and Aj is the i-th residue of the oligopeptide),
and calculating in the entire proteins of the organism "*a*", the frequency of occurrence of the following Xi-oligopeptide of length (n+1);
Xi-oligopeptide: ajlaj2....Xji....ajnaj(n+1)
(wherein, the residue Xji is any amino acid) ,
(4) calculating ratio Yji of the frequency of occurrence of the Aj-oligopeptide to that of the Xi-oligopeptide,
(5) calculating mean Yj of the Yji;

$$Yj = \sum_{i=1}^{n+1} Yji/(n+1),$$

(6) calculating functional value Zj of Yj;

$$Zj = f(Yj)$$

(Whetein, function f is a monotonously decreasing function or a monotonously increasing function), and defining the Zj value as a representative value of the function of the j-th amino-acid residue of the amino acid sequence of length (L) of the protein "*A*",

(7) sequentially repeating the steps (3) to (6) and determining the Zj value of each of all the amino-acid residues at position between n+1≤j≤L-n in the amino acid sequence of length (L),

(8) selecting at least one amino-acid residue to be subjected to mutation on the basis of the alignment data carried out in the step (1) from the amino acid sequence of length (L) of the protein "A", sequentially repeating the steps (3) to (6) for variant amino-acid residues in various mutated amino acid sequences where the selected amino-acid residue has been mutated into another amino-acid residue, to determine the Zj value of the variant amino-acid residues,

(9) selecting a mutated amino acid sequence wherein the Zj value of the variant amino-acid residue as determined in the step (8) is larger or smaller than the Zj value of the wild type amino-acid residue as determined in the step (7), and

(10) preparing a modified gene encoding the modified amino acid sequence from the protein "A" gene, and producing the modified protein as the expression product of the gene.

10. A method for modifying the function of protein "B" derived from an organism "b" of which genome data or cDNA data has been unknown, which method comprises the steps of:

(1) extracting protein "A" most closely related to protein "B" from the entire proteins of organism "a" of which genome data or cDNA data being known and subjecting the protein to alignment, or extracting a protein closely related to protein "B" from an existing protein data base to subject the protein to alignment,

(2) calculating in the amino acid sequences of the entire proteins of the organism "a", the frequency of occurrence of each amino acid and the frequency of occurrence of individual oligopeptides produced by permutations of twenty amino acids, and determining the smallest length (n) of oligopeptides satisfying the following criteria; among oligopeptides of length (n), the number of oligopeptides which occur once in the entire proteins is smaller than the number of oligopeptides which occur twice in the entire proteins; among oligopeptides of length (n+1), the number of oligopeptides which occur once in the entire proteins is larger than the number of oligopeptides which occur twice in the entire proteins,

(3) calculating in the entire proteins of the organism "a", the frequency of occurrence of the following Aj-oligopeptide of length (n+1), which is a part of the amino acid sequence of length of (L) of the protein "A" and contains an amino-acid residue Aj (n+1≤j≤L-n) at the j-th position from the N-terminus of the amino acid sequence of the protein;

Ai-oligopeptide: aj1aj2....Aji....ajnaj(n+1)

(wherein, 1≤i≤n+1; Aj=Aji; and Aj is the i-th residue of the oligopeptide),

and calculating in the entire proteins of the organism "a", the frequency of occurrence of the following Xi-oligopeptide of length (n+1);

Xi-oligopeptide: ajlaj2....Xji....ajnaj(n+1)

(wherein, the i-th residue Xji is any amino acid),

(4) calculating ratio Yji of the frequency of occurrence of the Aj-oligopeptide to that of the Xi-oligopeptide,

(5) calculating mean Yj of the Yji;

$$Yj = \sum_{i=1}^{n+1} Yji/(n+1),$$

(6) calculating functional value Zj of Yj;

$$Zj = f(Yj)$$

(wherein, function f is a monotonously decreasing function or a monotonously increasing function),

and defining the Zj value as a representative value of the function of the j-th amino-acid residue Aj of the amino acid sequence of length (L) of the protein "A",

(7) sequentially repeating the steps (3) to (6) and determining the Zj value of each of all the amino-acid residues at position between n+1≤j≤L-n in the amino acid sequence of length (L),

(8) selecting at least one amino-acid residue to be subjected to mutation on the basis of the alignment data carried out in the step (1) from the amino acid sequence of length (L) of the protein "A", sequentially repeating the steps (3) to (6) for variant amino-acid residues in various mutated amino acid sequences where the selected amino-acid residue has been mutated into another amino-acid residues, to determine the Zj value of the variant amino-acid residues,

(9) selecting the mutation position and the mutated amino-acid residue wherein the Zj value of the variant amino-acid residue as determined in the step (8) is larger or smaller than the Zj value of the wild type amino-acid residue as determined in the step (7), and

(10) preparing a modified gene encoding the modified amino acid sequence having the mutated amino-acid residue at the position from the protein *"B"* gene, and producing the modified protein as the expression product of the gene.

11. A method for modifying the known function of protein *"A"* derived from the entire proteins of organism *"a"* of which genome data or cDNA data has been known, which method comprises the steps of:

(1) extracting proteins closely related to the protein "*A*" from an existing protein data base and subjecting the proteins to alignment,

(2) calculating the frequency of occurrence of each amino acid and the frequency of occurrence of individual oligopeptides produced by permutations of twenty amino acids, in the amino acid sequences of the entire proteins of the organism "*a*",

(3) as to a protein of the organism "*a*",

(3') calculating in the entire proteins of the organism "*a*", the frequency of occurrence of the following Aj-oligopeptide of given length of (n) ($1 \leq n \leq M$, provided that M is the smallest length of oligopeptides satisfying the criterion that all the oligopeptides of length M are at frequency 1 of the occurrence), which Aj-oligopeptide is a part of the amino acid sequence of the protein and contains an amino-acid residue Aj ($n \leq j \leq L-n+1$) at the j-th position from the N-terminus of the protein;

Aj -oligopeptide: aj1aj2....aji....ajn

(wherein, $1 \leq i \leq n$; Aj=aji, and Aj is the i-th residue of the oligopeptide),

and calculating in the entire proteins of the organism "*a*", the frequency of occurrence of the following Xi-oligopeptide of length (n) corresponding to the length of Aj-oligopeptide;

Xi-oligopeptide: aj1aj2....Xji....ajn

(wherein, the i-th residue Xji is any amino acid),

(4) calculating ratio Yji of the frequency of occurrence of the Aj-oligopeptide to that of the Xi-oligopeptide,

(5) calculating mean Y(j,n) of the Yji;

$$Y(j,n) = \sum_{i=1}^{n} Yji/n,$$

(6) calculating functional value Z(j,n) of Y(j,n);

$$Z(j,n) = -\log(Y(j,n)),$$

(7) repeating the steps (3') to (6) sequentially and determining the Z(j,n) value of each amino-acid residue Aj at position j ($n \leq j \leq L-n+1$) in the amino acid sequence of length (L),

(8) sequentially repeating the steps (3) to (7) for the entire proteins of the organism *"a"*, thereby determining the distribution of the Z(j,n) value of each amino-acid residue in the entire proteins, and the Z(j,n) values are classified into twenty according to the twenty amino acids, and then calculating mean Av(Aa) of the Z(j,n) values for each amino acid Aa and the standard deviation Sd (Aa) of the distribution thereof, on the basis of the distribution, to determine function g to the j-th amino-acid residue Aj of a protein for normalizing the difference in distribution due to the species of amino-acid residues;

$$g= (Z(j,n), Aj) = [Z(j,n) - Av(Aa)]/Sd(Aa)$$

(wherein, Aj = Aa),

(9) calculating value D(j,n) of the function g of each Aj of all the amino-acid residues at the j-th position (n≤j≤L-n+1) of a protein in the entire proteins as recovered in the step (8) ;

$$D(j,n) = g(Z(j,n) , Aj) ,$$

(10) defining the representative value of the function of the j-th amino-acid residue in the amino acid sequence of length (L) as functional value Wj of the Z(j,n) and D(j,n);

$$Wj=h(Z(j,l),Z(j,2) ,..,Z(j,M),D(j,l) ,D(j,2),..,D(j,M))$$

(11) sequentially repeating the steps (3) to (10), to determine the individual Wj values of all the amino-acid residues at the position (n+1≤j≤L-n) in the amino acid sequence of length (L),
(12) selecting at least one amino-acid residue to be subjected to mutation on the basis of the alignment data carried out in the step (1) from the amino acid sequence of length (L) of the protein "A", and sequentially repeating the steps (3) to (10) for variant amino-acid residues in various mutated amino acid sequences where the selected amino-acid residue has been mutated into another amino-acid residue, to determine the Wj value of the variant amino-acid residue,
(13) selecting a mutated amino acid sequence wherein the Wj value of the variant amino-acid residue as determined in the step (12) is larger or smaller than the Wj value of the wild type amino-acid residue as determined in the step (10), and
(14) preparing a modified gene encoding the modified amino acid sequence from the protein "A" gene, and producing the modified protein as the expression product of the gene.

**Patentansprüche**

1. System zur automatischen Durchführung eines Verfahrens zur Voraussage einer funktionellen Stelle eines Proteins, abgeleitet von den gesamten mutmaßlichen Proteinen eines Organismus "a", von dem Genom-Daten oder cDNA-Daten bekannt sind, welches Verfahren die Schritte umfasst:

(1) Berechnen der Häufigkeit des Auftretens einer jeden Aminosäure und der Häufigkeit des Auftretens individueller Oligopeptide, gebildet durch Permutationen von 20 Aminosäuren, in den Aminosäuresequenzen der gesamten Proteine des Organismus "a" und Feststellen der kleinsten Länge (n) von Oligopeptiden, welche die folgenden Kriterien erfüllen:
unter den Oligopeptiden der Länge (n) ist die Anzahl der Oligopeptide, welche einmal in den gesamten Proteinen vorkommen, kleiner als die Anzahl der Oligopeptide, welche zweimal in den gesamten Proteinen vorkommen;
unter den Oligopeptiden der Länge (n+1) ist die Anzahl der Oligopeptide, welche einmal in den gesamten Proteinen vorkommen, größer als die Anzahl der Oligopeptide, welche zweimal in den gesamten Proteinen vorkommen;
(2) Berechnen der Häufigkeit des Auftretens des folgenden Aj-Oligopeptids der Länge (n+1), welches ein Teil der Aminosäuresequenz der Länge (L) des Proteins als Gegenstand zur Voraussage einer funktionellen Stelle ist und einen Aminosäurerest Aj (n+1 ≤j≤ L-1) an der j-ten Position vom N-Terminus der Aminosäuresequenz des Proteins enthält, in den gesamten Proteinen des Organismus "a";
Aj-Oligopeptid: aj1 aj2....Aji....ajnaj(n+1 )
(worin 1≤ i≤ n+1; Aj = Aji; und Aj der i-te Rest des Oligopeptids ist);
und Berechnen in den gesamten Proteinen des Organismus "a" die Häufigkeit des Auftretens des folgenden Xi-Oligopeptids der Länge (n+1);
Xi-Oligopeptid: aj1aj2....Xi....ajnaj(n+1)
(worin der i-te Rest Xi irgendeine Aminosäure ist);
(3) Berechnen des Verhältnisses Yji der Häufigkeit des Auftretens des Aj-Oligopeptids zu derjenigen des Xi-Oligopeptids;
(4) Berechnen des Mittelwerts Yj des Yji;

$$Yj = \sum_{i=1}^{n+1} Yji/(n+1),$$

(5) Berechnen des Funktionswerts Zj von Yj;

$$Zj = f(Yj)$$

(worin die Funktion f eine monoton abnehmende Funktion oder eine monoton zunehmende Funktion ist), und Definieren des Zj-Werts als repräsentativen Wert der Funktion des j-ten Aminosäurerests Aj der Aminosäuresequenz der Länge (L) und

(6) Wiederholen der Schritte (2) bis (5) nacheinander und Bestimmen des Zj-Werts eines jeden Aj aller Aminosäurereste an den Positionen zwischen n+1 ≤ j ≤ L-n in der Aminosäuresequenz der Länge (L), wodurch der Grad der Beteiligung eines jeden Aminosäurerests an der Funktion des Proteins vorausgesagt wird durch Verwendung der Dimension des Zj-Werts als Indikator, wobei das System mindestens die folgenden Einheiten (a) bis (g) umfasst;

(a) eine äußere Speichereinheit, welche die Aminosäuresequenzdaten der gesamten mutmaßlichen Proteine des Organismus *"a",* von dem Genom-Daten oder cDNA-Daten bekannt sind, speichert, sowie eine existierende Proteindatenbank;

(b) eine Berechnungs/Speichereinheit, aufgebaut aus einer CPU (Zentralen Prozessierungseinheit), welche die Häufigkeit des Auftretens einer jeden Aminosäure und die Häufigkeit des Auftretens individueller Oligopeptide, gebildet von Permutationen von 20 Aminosäuren, in den Aminosäuresequenzen der gesamten Proteine des Organismus "a" berechnet, und einer Speichereinheit mit der Speicherung der Berechnungsergebnisse;

(c) eine Berechnungs/Speichereinheit, aufgebaut aus einer CPU, welche die kleinste Länge (n) von Oligopeptiden berechnet, welche unter den individuellen Oligopeptiden, von denen die Häufigkeiten des Auftretens in der Einheit (b) gespeichert sind, die folgenden Kriterien erfüllen:

unter den Oligopeptiden der Länge (n) ist die Anzahl der Oligopeptide, welche einmal in den gesamten Proteinen auftreten, kleiner als die Anzahl der Oligopeptide, welche zweimal in den gesamten Proteinen auftreten; unter den Oligopeptiden der Länge (n+1) ist die Anzahl der Oligopeptide, welche einmal in den gesamten Proteinen auftreten, größer ist als die Anzahl der Oligopeptide, welche zweimal in den gesamten Proteinen auftreten;

und einer Speichereinheit mit der Speicherung der (n);

(d) eine Berechnungs/Speichereinheit, aufgebaut aus einer CPU, welche in den gesamten Proteinen des Organismus "a" die Häufigkeit des Auftretens des folgenden Aj-Oligopeptids der Länge (n+1) berechnet, das ein Teil der Aminosäuresequenz der Länge (L) des Proteins als Gegenstand zur Voraussage einer funktionellen Stelle ist und einen Aminosäurerest Aj (n+1 ≤ j ≤ L-n) an der j-ten Position vom N-Terminus der Aminosäuresequenz des Proteins enthält;

Aj-Oligopeptid: aj1aj2....Aji....ajnaj(n+1)

(worin 1 ≤ i ≤ n+1; Aj = Aji; und Aj der i-te Rest des Oligopeptids ist);

und in den gesamten Proteinen des Organismus "a" die Häufigkeit des Auftretens des folgenden Xi-Oligopeptids der Länge (n+1) berechnet;

Xi-Oligopeptid: aj 1 aj2....Xji....ajnaj(n+1)

(worin der i-te Rest Xji irgendeine Aminosäure ist);

und einer Speichereinheit mit der Speicherung der Berechnungsergebnisse;

(e) eine Berechnungs/Speichereinheit, aufgebaut aus einer CPU, welche das Verhältnis Yji der Häufigkeit des Auftretens des Aj-Oligopeptids zu derjenigen des Xi-Oligopeptids berechnet, und einer Speichereinheit mit der Speicherung von Yji;

(f) eine Berechnungs/Speichereinheit, aufgebaut aus einer CPU, welche den Mittelwert Yj des Yji berechnet;

$$Y_j = \sum_{i=1}^{n+1} Y_{ji}/(n+1)$$

und einer Speichereinheit mit der Speicherung von Yj; und

(g) eine Berechnungs/Speichereinheit, aufgebaut aus einer CPU, welche den Funktionswert Zj von Yj berechnet,

$$Z_j = f(Y_j)$$

(worin die Funktion f eine monoton abnehmende Funktion oder eine monoton zunehmende Funktion ist), und einer Speichereinheit mit der Speicherung von Zj.

2. System nach Anspruch 1, wobei in dem mit dem System durchgeführten Verfahren der Zj-Wert ($n+1 \le j \le L-n$) eines jeden Aminosäurerests in der Aminosäuresequenz der Länge (L) in einer Verteilungskarte dargestellt wird.

3. System nach Anspruch 1, welches ferner eine Anzeigeeinheit umfasst, welche den Zj-Wert ($n+1 \le j \le L-n$) eines jeden Aminosäurerests in der Aminosäuresequenz der Länge (L) in einer Verteilungskarte darstellt.

4. System zur automatischen Durchführung eines Verfahrens zur Voraussage einer funktionellen Stelle eines Proteins, abgeleitet von den gesamten mutmaßlichen Proteinen eines Organismus "a", von dem Genom-Daten oder cDNA-Daten bekannt sind, welches Verfahren die Schritte umfasst:

(1) Berechnen der Häufigkeit des Auftretens einer jeden Aminosäure und der Häufigkeit des Auftretens individueller Oligopeptide, gebildet durch Permutationen von 20 Aminosäuren, in den Aminosäuresequenzen der gesamten Proteine des Organismus "a";

(2) bezüglich eines Proteins des Organismus "a",

(2') Berechnen in den gesamten Proteinen des Organismus "a" die Häufigkeit des Auftretens des folgenden Aj-Oligopeptids einer gegebenen Länge (n) ($1 \le n \le M$, mit der Maßgabe, dass M die kleinste Länge von Oligopeptiden ist, welche das Kriterium erfüllen, dass alle Oligopeptide der Länge M mit einer Häufigkeit 1 des Auftretens vorliegen), welches Aj-Oligopeptid ein Teil der Aminosäuresequenz der Länge (L) des Proteins ist und einen Aminosäurerest Aj ($n \le j \le L-n+1$) an der j-ten Position vom N-Terminus der Aminosäuresequenz des Proteins enthält;

Aj-Oligopeptid: aj1aj2....aji....ajn

(worin $1 \le i \le n+1$, Aj = aji und Aj der i-te Rest des Oligopeptids ist);

und Berechnen in den gesamten Proteinen des Organismus "a" die Häufigkeit des Auftretens des folgenden Xi-Oligopeptids der Länge (n) entsprechend der Länge des Aj-Oligopeptids;

Xi-Oligopeptid: aj1aj2....Xi....ajn

(worin der i-te Rest Xi irgendeine Aminosäure ist),

(3) Berechnen des Verhältnisses Yji der Häufigkeit des Auftretens des Aj-Oligopeptids zu derjenigen des Xi-Oligopeptids;

(4) Berechnen des Mittelwerts Y(j,n) des Yji;

$$Y(j, n) = \sum_{i=1}^{n} Y_{ji}/n,$$

(5) Berechnen des Funktionswerts Z(j,n) von Y(j,n);

(6) Wiederholen der Schritte (2') bis (5) nacheinander und Bestimmen des Z(j,n)-Werts eines jeden Aminosäurerests Aj an der j-ten Position ($n \le j \le L-n+1$) in der Aminosäuresequenz der Länge (L);

(7) Wiederholen der Schritte (2) bis (6) nacheinander für die gesamten Proteine des Organismus "a", wodurch die Verteilung des Z(j,n)-Werts eines jeden Aminosäurerests in den gesamten Proteinen bestimmt wird und die Z(j,n)-Werte in 20 gemäß den 20 Aminosäuren klassifiziert werden, und dann Berechnen des Mittelwerts Av (As) der Z(j,n)-Werte für jede Aminosäure As und der Standardabweichung Sd (As) von deren Verteilung, um auf der Basis der Verteilung eine Funktion g für den j-ten Aminosäurerest Aj eines Proteins für die Normalisierung der Differenz in der Verteilung aufgrund der Spezies der Aminosäurereste zu berechnen;

$$g = (Z(j,n), Aj) = [Z(j,n) - Av(As)]/Sd(As)$$

(worin Aj = As ist);

(8) Berechnen eines Werts D(j,n) der Funktion g eines jeden Aj aller Aminosäurereste an der j-ten Position (n ≤ j ≤ L-n+1) eines Proteins in den gesamten Proteinen, wie in Schritt (7) gewonnen;

$$D(j,n) = g(Z(j,n), Aj),$$

und

(9) Definieren des repräsentativen Werts der Funktion des j-ten Aminosäurerests in der Aminosäuresequenz der Länge (L) als Funktionswert Wj von Z(j,n) und D(j,n);

$$Wj = h(Z(j,1),Z(j,2),....,Z(j,M),D(j,1),D(j,2),....,D(j,M))$$

und **dadurch** Voraussagen des Grades der Beteiligung eines jeden Aminosäurerests an der Funktion des Proteins durch Verwendung der Dimension des Wj-Werts als Indikator, wobei das System mindestens die folgenden Einheiten (a) bis (i) umfasst;

(a) eine äußere Speichereinheit, welche die Aminosäuresequenzdaten der gesamten mutmaßlichen Proteine des Organismus "a", von dem Genom-Daten oder cDNA-Daten bekannt sind, speichert, sowie eine existierende Proteindatenbank;

(b) eine Berechnungs/Speichereinheit, aufgebaut aus einer CPU, welche die Häufigkeit des Auftretens einer jeden Aminosäure und die Häufigkeit des Auftretens individueller Oligopeptide, gebildet durch Permutationen von 20 Aminosäuren, in den Aminosäuresequenzen der gesamten Proteine des Organismus "a" berechnet, und einer Speichereinheit mit der Speicherung der Berechnungsergebnisse;

(c) eine Berechnungs/Speichereinheit, aufgebaut aus einer CPU, welche in den gesamten Proteinen des Organismus "a" die Häufigkeit des Auftretens des folgenden Aj-Oligopeptids einer gegebenen Länge (n) (1 ≤ n ≤ M, mit der Maßgabe, dass M die kleinste Länge von Oligopeptiden ist, welche das Kriterium erfüllen, dass alle Oligopeptide der Länge M eine Häufigkeit des Auftretens 1 aufweisen) berechnet, welches Aj-Oligopeptid ein Teil der Aminosäuresequenz der Länge (L) eines gegebenen Proteins des Organismus "a" ist und einen Aminosäurerest Aj (n ≤ j ≤ L-n+1) an der j-ten Position vom N-Terminus der Aminosäuresequenz des Proteins enthält;

Aj-Oligopeptid: aj1aj2....aji....ajn

(worin 1 ≤ i ≤ n+1, Aj = aji und Aj der i-te Rest des Oligopeptids ist),

und in den gesamten Proteinen des Organismus "a" die Häufigkeit des Auftretens des folgenden Xi-Oligopeptids der Länge (n) entsprechend der Länge des Aj-Oligopeptids berechnet;

Xi-Oligopeptid: aj1aj2....Xji....ajn

(worin der i-te Rest Xji irgendeine Aminosäure ist),

und einer Speichereinheit, welche die Berechnungsergebnisse speichert;

(d) eine Berechnungs/Speichereinheit, aufgebaut aus einer CPU, welche das Verhältnis Yji der Häufigkeit des Auftretens des Aj-Oligopeptids zu derjenigen des Xi-Oligopeptids berechnet, und einer Speichereinheit mit der Speicherung des Yji;;

(e) eine Berechnungs/Speichereinheit, aufgebaut aus einer CPU, welche den Mittelwert Y(j,n) des Yji berechnet;

$$Y(j,n) = \sum_{i=1}^{n} Yji/n,$$

und einer Speichereinheit mit der Speicherung von Y(j,n);
(f) eine Berechnungs/Speichereinheit, aufgebaut aus einer CPU, welche den Funktionswert Z(j,n) von Y(j, n) berechnet;

$$Z(j,n) = -log(Y(j,n)),$$

und einer Speichereinheit mit der Speicherung von Z(j,n);
(g) eine Berechnungs/Speichereinheit, aufgebaut aus einer CPU, welche den Z(j,n)-Wert eines jeden Aminosäurerests in den Aminosäuresequenzen der gesamten Proteine des Organismus "a" berechnet, die Verteilung des Z(j,n)-Werts eines jeden Aminosäurerests in den gesamten Proteinen berechnet und die Z(j,n)-Werte entsprechend den 20 Aminosäureresten in 20 klassifiziert, und dann den Mittelwert Av(As) der Z(j,n)-Werte für jede Aminosäure As und die Standardabweichung Sd(As) von deren Verteilung berechnet, um auf Basis der Verteilung eine Funktion g zur Normalisierung der Differenz in der Verteilung aufgrund der Spezies von Aminosäureresten zu bestimmen;

$$g = (Z(j,n), Aj) = [Z(j,n) - Av(As)]/Sd(As) \text{ (worin } Aj = As)$$

und einer Speichereinheit mit der Speicherung von g;
(h) eine Berechnungs/Speichereinheit, aufgebaut aus einer CPU, welche den Wert (D(j,n) der Funktion g, die in der Einheit (g) gespeichert ist, bezüglich eines jeden der Aminosäurereste Aj an der j-ten Position $(n \leq j \leq L-n+1)$ in der Aminosäuresequenz der Länge (L) berechnet;

$$D(j,n) = g(Z(j,n), Aj)$$

und einer Speichereinheit mit der Speicherung des D(j,n)-Werts; und
(i) eine Berechnungs/Speichereinheit, aufgebaut aus einer Berechnungseinheit, welche einen angemessenen Funktionswert Wj der Z(j,n) und D(j,n) eines jeden Aminosäurerests in der Aminosäuresequenz berechnet;

$$Wj = h(Z(j,1),Z(j,2),....,Z(j,M),D(j,1),D(j,2),....,D(j,M))$$

und einer Speichereinheit mit der Speicherung des Wj-Werts.

5. System nach Anspruch 4, wobei in dem mit dem System durchgeführten Verfahren der Wj-Wert eines jeden Aminosäurerests in einer zweidimensionalen Verteilungskarte dargestellt wird.

6. System nach Anspruch 4, wobei in dem mit dem System durchgeführtem Verfahren der Wj-Wert eines jeden Aminosäurerests in einem dreidimensionalen Strukturmodell des Proteins dargestellt wird.

7. System nach Anspruch 4, welches ferner eine Anzeigeeinheit umfasst, welche den Wj-Wert eines jeden Aminosäurerests in einer zweidimensionalen Verteilungskarte darstellt.

8. System nach Anspruch 4, welches ferner eine Berechnungs/Speichereinheit umfasst, welche eine existierende Datenbank der dreidimensionalen Struktur von Proteinen speichert oder ein dreidimensionales Strukturmodell auf der Basis einer Aminosäuresequenz nach einem bekannten Verfahren erstellt und das dreidimensionale Strukturmodell speichert, und eine Anzeigeeinheit umfasst, welche den Wj-Wert eines jeden Aminosäurerests in der Aminosäuresequenz in einer Verteilungskarte bezüglich der dreidimensionalen Struktur, die in der Datenbank gespeichert ist, oder des dreidimensionalen Strukturmodells, das in der Berechnungs/Speichereinheit gespeichert ist, darstellt.

9. Verfahren zur Modifizierung der bekannten Funktion eines Proteins "A", abgeleitet von den gesamten Proteinen eines Organismus "a", von dem Genom-Daten oder cDNA-Daten bekannt waren, welches Verfahren die Schritte umfasst:

(1) Extrahieren eines Proteins, das eng mit dem Protein "A" verwandt ist, aus einer existierenden Protein-Datenbank und Unterwerfen der Proteine einer Zuordnung;

(2) Berechnen der Häufigkeit des Auftretens einer jeden Aminosäure und der Häufigkeit des Auftretens individueller Oligopeptide, gebildet durch Permutationen von 20 Aminosäuren, in den Aminosäuresequenzen der gesamten Proteine des Organismus "a", und Bestimmen der kleinsten Länge (n) von Oligopeptiden, welche die folgenden Kriterien erfüllen:

unter den Oligopeptiden der Länge (n) ist die Anzahl der Oligopeptide, welche einmal in den gesamten Proteinen vorkommen, kleiner als die Anzahl der Oligopeptide, welche zweimal in den gesamten Proteinen vorkommen;

unter den Oligopeptiden der Länge (n+1) ist die Anzahl der Oligopeptide, welche einmal in den gesamten Proteinen vorkommen, größer als die Anzahl der Oligopeptide, die zweimal in den gesamten Proteinen vorkommen;

(3) Berechnen in den gesamten Proteinen des Organismus "a" die Häufigkeit des Auftretens des folgenden Aj-Oligopeptids der Länge (n+1), welches ein Teil der Aminosäuresequenz der Länge (L) des Proteins "A" ist und einen Aminosäurerest Aj ($n+1 \leq j \leq L-n$) an der j-ten Position vom N-Terminus der Aminosäuresequenz des Proteins enthält;

Aj-Oligopeptid: aj1aj2....Aji....ajnaj(n+1)

(worin $1 \leq i \leq n+1$, Aj = Aji, und Aj der i-te Rest des Oligopeptids ist),

und Berechnen in den gesamten Proteinen des Organismus "a" die Häufigkeit des Auftretens des folgenden Xi-Oligopeptids der Länge (n+1);

Xi-Oligopeptid: aj1aj2....Xji....ajnaj(n+1)

(worin der Rest Xji irgendeine Aminosäure ist);

(4) Berechnen des Verhältnisses Yji der Häufigkeit des Auftretens des Aj-Oligopeptids zu derjenigen des Xi-Oligopeptids;

(5) Berechnen des Mittelwerts Yj des Yji;

$$Yj = \sum_{i=1}^{n+1} Yji/(n+1),$$

(6) Berechnen des Funktionswerts Zj von Yj;

$$Zj = f(Yj)$$

(worin die Funktion f eine monoton abnehmende Funktion oder monoton zunehmende Funktion ist),

und Definieren des Zj-Werts als repräsentativen Wert der Funktion des j-ten Aminosäurerests der Aminosäuresequenz der Länge (L) des Proteins "A";

(7) Wiederholen der Schritte (3) bis (6) nacheinander und Bestimmen des Zj-Werts eines jeden Aminosäurerests an der Position zwischen $n+1 \leq j \leq L-n$ in der Aminosäuresequenz der Länge (L);

(8) Auswählen mindestens eines Aminosäurerests, der einer Mutation unterworfen werden soll, auf Basis der in Schritt (1) erhaltenen Zuordnungsdaten aus der Aminosäuresequenz der Länge (L) des Proteins "A", Wiederholen der Schritte (3) bis (6) nacheinander für Varianten-Aminosäurereste in verschiedenen mutierten Aminosäuresequenzen, worin der ausgewählte Aminosäurerest zu einem anderen Aminosäurerest mutiert wurde, um den Zj-Wert der Varianten-Aminosäurereste zu bestimmen;

(9) Auswählen einer mutierten Aminosäuresequenz, wobei der Zj-Wert des Varianten-Aminosäurerests wie in Schritt (8) bestimmt größer oder kleiner ist als der Zj-Wert des Wildtyp-Aminosäurerests wie in Schritt (7) bestimmt, und

(10) Herstellen eines modifizierten Gens, welches für die modifizierte Aminosäuresequenz von dem Protein *"A"*-Gen codiert, und Herstellen des modifizierten Proteins als Expressionsprodukt des Gens.

10. Verfahren zur Modifizierung der Funktion eines Proteins "B", abgeleitet von einem Organismus "b", von dem Genom-Daten oder cDNA-Daten unbekannt waren, welches Verfahren die Schritte umfasst:

(1) Extrahieren eines Proteins *"A",* welches mit dem Protein *"B"* am engsten verwandt ist, aus den gesamten Proteinen des Organismus "a", von dem genomische Daten oder cDNA-Daten bekannt sind, und Unterwerfen des Proteins einer Zuordnung oder Extrahieren eines Proteins, das mit dem Protein *"B"* eng verwandt ist, aus einer existierenden Proteindatenbank, um das Protein einer Zuordnung zu unterwerfen;

(2) Berechnen in den Aminosäuresequenzen der gesamten Proteine des Organismus "a" die Häufigkeit des Auftretens einer jeden Aminosäure und die Häufigkeit des Auftretens individueller Oligopeptide, gebildet durch Permutationen von 20 Aminosäuren, und Bestimmen der kleinsten Länge (n) von Oligopeptiden, welche die folgenden Kriterien erfüllen:

unter den Oligopeptiden der Länge (n) ist die Anzahl der Oligopeptide, welche einmal in den gesamten Proteinen auftreten, kleiner als die Anzahl der Oligopeptide, welche zweimal in den gesamten Proteinen auftreten; unter den Oligopeptiden der Länge (n+1) ist die Anzahl der Oligopeptide, welche einmal in den gesamten Proteinen auftreten, größer als die Anzahl der Oligopeptide, welche zweimal in den gesamten Proteinen auftreten;

(3) Berechnen in den gesamten Proteinen des Organismus "a" die Häufigkeit des Auftretens des folgenden Aj-Oligopeptids der Länge (n+1), welches ein Teil der Aminosäuresequenz der Länge (L) des Proteins *"A"* ist und einen Aminosäurerest Aj ($n+1 \leq j \leq L-n$) an der j-ten Position vom N-Terminus der Aminosäuresequenz des Proteins enthält;

Ai-Oligopeptid: aj1aj2....Aji....ajnaj(n+1)

(worin $1 \leq i \leq n+1$, Aj = Aji, und Aj der i-te Rest des Oligopeptids ist),

und Berechnen in den gesamten Proteinen des Organismus "a" die Häufigkeit des Auftretens des folgenden Xi-Oligopeptids der Länge (n+1);

Xi-Oligopeptid: aj1aj2....Xji....ajnaj(n+1)

(worin der i-te Rest Xji irgendeine Aminosäure ist);

(4) Berechnen des Verhältnisses Yji der Häufigkeit des Auftretens des Aj-Oligopeptids zu derjenigen des Xi-Oligopeptids;

(5) Berechnen des Mittelwerts Yj des Yji;

$$Yj = \sum_{i=1}^{n+1} Yji/(n+1),$$

(6) Berechnen des Funktionswerts Zj von Yj;

$$Zj = f(Yj)$$

(worin die Funktion f eine monoton abnehmende Funktion oder eine monoton zunehmende Funktion ist), und Definieren des Zj-Werts als repräsentativen Wert der Funktion des j-ten Aminosäurerests Aj der Aminosäuresequenz der Länge (L) des Proteins "A";

(7) Wiederholen der Schritte (3) bis (6) nacheinander und Bestimmen des Zj-Werts eines jeden der Aminosäurereste an der Position zwischen $n+1 \leq j \leq L-n$ in der Aminosäuresequenz der Länge (L);

(8) Auswählen mindestens eines Aminosäurerests, der einer Mutation unterworfen werden soll, auf Grundlage der in Schritt (1) erhaltenen Zuordnungsdaten aus der Aminosäuresequenz der Länge (L) des Proteins "A", Wiederholen der Schritte (3) bis (6) nacheinander für Varianten-Aminosäurereste in verschiedenen mutierten Aminosäuresequenzen, worin der ausgewählte Aminosäurerest zu anderen Aminosäureresten mutiert wurde, um den Zj-Wert der Varianten-Aminosäurereste festzustellen;

(9) Auswählen der Mutationsposition und des mutierten Aminosäurestes, wobei der Zj-Wert des Varianten-Aminosäurerests wie in Schritt (8) bestimmt größer oder kleiner ist als der Zj-Wert des Wildtyp-Aminosäurerests wie in Schritt (7) bestimmt; und

(10) Herstellen eines modifizierten Gens, welches für die modifizierte Aminosäuresequenz mit dem mutierten Aminosäurerest an der Position von dem Protein "B"-Gen codiert, und Herstellen des modifizierten Proteins als Expressionsprodukt des Gens.

**11.** Verfahren zur Modifizierung der bekannten Funktion eines Proteins "A", abgeleitet von den gesamten Proteinen des Organismus "a", von dem Genom-Daten oder cDNA-Daten bekannt waren, welches Verfahren die Schritte umfasst:

(1) Extrahieren von Proteinen, die eng mit dem Protein "A" verwandt sind, aus einer existierenden Proteindatenbank und Unterwerfen der Proteine einer Zuordnung;

(2) Berechnen der Häufigkeit des Auftretens einer jeden Aminosäure und der Häufigkeit des Auftretens individueller Oligopeptide, gebildet durch Permutationen von 20 Aminosäuren, in den Aminosäuresequenzen der gesamten Proteine des Organismus "a";

(3) bezüglich eines Proteins des Organismus "a",

(3') Berechnen in den gesamten Proteinen des Organismus "a" die Häufigkeit des Auftretens des folgenden Aj-Oligopeptids einer gegebenen Länge (n) ($1 \leq n \leq M$, mit der Maßgabe, dass M die kleinste Länge von Oligopeptiden ist, welche das Kriterium erfüllen, dass alle Oligopeptide der Länge M eine Häufigkeit 1 des Auftretens haben), welches Aj-Oligopeptid ein Teil der Aminosäuresequenz des Proteins ist und einen Aminosäurerest Aj ($n \leq j \leq$ L-n+1) an der j-ten Position vom N-Terminus des Proteins enthält;

Aj-Oligopeptid: aj1aj2....aji....ajn

(worin $1 \leq i \leq n$, Aj = aji und Aj der i-te Rest des Oligopeptids ist);

und Berechnen in den gesamten Proteinen des Organismus "a" die Häufigkeit des Auftretens des folgenden Xi-Oligopeptids der Länge (n) entsprechend der Länge des Aj-Oligopeptids;

Xi-Oligopeptid: aj1aj2....Xji....ajn

(worin der i-te Rest Xji irgendeine Aminosäure ist);

(4) Berechnen des Verhältnisses Yji der Häufigkeit des Auftretens des Aj-Oligopeptids zu derjenigen des Xi-Oligopeptids;

(5) Berechnen des Mittelwerts Y(j,n) des Yji;

$$Y(j,n) = \sum_{i=1}^{n} Yji/n,$$

(6) Berechnen des Funktionswerts Z(j,n) von Y(j,n)

$$Z(j,n) = -\log(Y(j,n));$$

(7) Wiederholen der Schritte (3') bis (6) nacheinander und Bestimmen des Z(j,n)-Werts eines jeden Aminosäurerests Aj an der Position j ($n \leq j \leq$ L-n+1) in der Aminosäuresequenz der Länge (L);

(8) Wiederholen der Schritte (3) bis (7) nacheinander für die gesamten Proteine des Organismus *"a",* wodurch die Verteilung des Z(j,n)-Werts eines jeden Aminosäurerests in den gesamten Proteinen bestimmt und die Z(j, n)-Werte in 20, entsprechend den 20 Aminosäuren, klassifiziert werden, und dann Berechnen des Mittelwerts Av(As) der Z(j,n)-Werte für jede Aminosäure As und der Standardabweichung Sd(As) von deren Verteilung, um auf Basis der Verteilung die Funktion g für den j-ten Aminosäurerest Aj eines Proteins zu bestimmen, um die Differenz in der Verteilung aufgrund der Spezies von Aminosäureresten zu normalisieren;

$$g = Z(j,n),Aj) = [Z(j,n) - Av(As)]/Sd(As)$$

(worin Aj = As ist);

(9) Berechnen des Werts D(j.n) der Funktion g eines jeden Aj aller Aminosäurereste an der j-ten Position ($n \leq j \leq$

L-n+1) eines Proteins in den gesamten Proteinen wie in Schritt (8) gewonnen;

$$D(j,n) = g(Z(j,n),Aj);$$

(10) Definieren des repräsentativen Werts der Funktion des j-ten Aminosäurerests in der Aminosäuresequenz der Länge (L) als Funktionswert Wj von Z(j,n) und D(j,n)

$$Wj = h(Z(j,1),Z(j,2)....,Z(j,M),D(j,1),D(j,2)....,D(j,M));$$

(11) Wiederholen der Schritte (3) bis (10) nacheinander, um die individuellen Wj-Werte aller Aminosäurereste an der Position (n+1 $\leq$j$\leq$ L-n) in der Aminosäuresequenz der Länge (L) zu bestimmen;

(12) Auswählen mindestens eines Aminosäurerests, der einer Mutation unterworfen werden soll, auf Grundlage der in Schritt (1) erhaltenen Zuordnungsdaten aus der Aminosäuresequenz der Länge (L) des Proteins "A" und Wiederholen der Schritte (3) bis

(10) nacheinander für Varianten-Aminosäurereste in verschiedenen mutierten Aminosäuresequenzen, worin der ausgewählte Aminosäurerest zu einem anderen Aminosäurerest mutiert wurde, um den Wj-Wert des Varianten-Aminosäurerests zu bestimmen;

(13) Auswählen einer mutierten Aminosäuresequenz, wobei der Wj-Wert des Varianten-Aminosäurerests wie in Schritt (12) bestimmt größer oder kleiner ist als der Wj-Wert des Wildtyp-Aminosäurerests wie in Schritt (10) bestimmt; und

(14) Herstellen eines modifizierten Gens, welches für die modifizierte Aminosäuresequenz des Protein "*A*"-Gens codiert, und Herstellen des modifizierten Proteins als Expressionsprodukt des Gens.

## Revendications

1. Système pour effectuer automatiquement un procédé pour prédire un site fonctionnel d'une protéine dérivée de la totalité des protéines supposées d'un organisme *"a"* dont on connaît des données sur le génome ou des données sur l'ADNc, ce procédé comprenant les étapes consistant à :

(1) calculer la fréquence d'occurrence de chaque aminoacide, et la fréquence d'occurrence des oligopeptides individuels produits par les permutations de 20 aminoacides dans les séquences d'aminoacides de la totalité des protéines de l'organisme *"a",* et déterminer la plus courte longueur (n) des oligopeptides satisfaisant aux critères suivants :

parmi les oligopeptides de longueur (n), le nombre d'oligopeptides qui apparaissent une fois dans la totalité des protéines, est inférieur au nombre des oligopeptides qui apparaissent deux fois dans la totalité des protéines ; parmi les oligopeptides de longueur (n+1), le nombre des oligopeptides qui apparaissent une fois dans la totalité des protéines, est supérieur au nombre des oligopeptides qui apparaissent deux fois dans la totalité des protéines,

(2) calculer dans la totalité des protéines de l'organisme *"a",* la fréquence d'occurrence du Aj-oligopeptide suivant de longueur (n+1), qui est une partie de la séquence d'aminoacides de longueur (L) de la protéine, en tant que sujet pour prédire un site fonctionnel, et contient un résidu d'aminoacide Aj (n+1 $\leq$j$\leq$ L-1) à la j$^{\text{ième}}$ position à partir de l'extrémité N-terminale de la séquence d'aminoacides de la protéine ;
Aj-oligopeptide: ajlaj2....Aji....ajnaj(n+1)
(dans laquelle 1 $\leq$ i $\leq$ n+1 ; Aj = Aji ; et Aj est le i$^{\text{ième}}$ résidu de l'oligopeptide), et
calculer dans la totalité des protéines de l'organisme "a", la fréquence d'occurrence du Xi-oligopeptide suivant de longueur (n+1) ;
Xi-oligopeptide: ajlaj2....Xi....ajnaj(n+1)
(dans lequel le i$^{\text{ième}}$ résidu Xi est n'importe quel aminoacide),
(3) calculer le rapport Yji de la fréquence d'occurrence du Aj-oligopeptide à celle du Xi-oligopeptide,
(4) calculer la moyenne Yj des valeurs Yji;

$$Yj = \sum_{i=1}^{n+1} Yji/(n+1),$$

(5) calculer la valeur fonctionnelle Zj de Yj :

$$Zj = f(Yj)$$

(dans laquelle la fonction f est une fonction monotone décroissante ou une fonction monotone croissante), et définir la valeur Zj en tant que valeur représentative de la fonction du j$^{ième}$ résidu d'aminoacide Aj de la séquence d'aminoacides de longueur (L), et

(6) répéter séquentiellement les étapes (2) à (5), et déterminer la valeur Zj de chaque Aj de tous les résidus d'aminoacide aux positions de n+1 $\leq$j$\leq$ L-n dans la séquence d'aminoacides de longueur L, de façon à prédire le degré d'implication de chaque résidu d'aminoacide dans la fonction de la protéine, en employant la dimension de la valeur Zj comme indicateur, ledit système comprenant au moins les unités (a) à (g) suivantes ;

(a) une unité de mémoire externe, mémorisant les données sur la séquence d'aminoacides de la totalité des protéines supposées de l'organisme *"a"* dont on connaît des données sur le génome ou des données sur l'ADNc, de même qu'une base de données sur les protéines existantes,

(b) une unité de calcul/mémoire composée d'une unité de traitement centrale (CPU) calculant la fréquence d'occurrence de chaque aminoacide et la fréquence d'occurrence des oligopeptides individuels produits par les permutations de vingt aminoacides, dans les séquences d'aminoacides de la totalité des protéines de l'organisme "*a*", et une unité de mémoire ayant la mémoire des résultats de calcul,

(c) une unité de calcul/mémoire composée d'une CPU calculant la plus petite longueur (n) des oligopeptides satisfaisant aux critères suivants, parmi les oligopeptides individuels dont les fréquences des occurrences, sont mémorisées dans l'unité (b) ;

parmi les oligopeptides de longueur (n), le nombre des oligopeptides qui apparaissent une fois dans la totalité des protéines est inférieur au nombre des oligopeptides qui apparaissent deux fois dans la totalité des protéines ; parmi les oligopeptides de longueur (n+1), le nombre des oligopeptides qui apparaissent une fois dans la totalité des protéines est supérieur au nombre des oligopeptides qui apparaissent deux fois dans la totalité des protéines,

et une unité de mémoire ayant la mémoire de (n),

(d) une unité de calcul/mémoire composée d'une CPU calculant dans la totalité des protéines de l'organisme *"a"*, la fréquence d'occurrence du Aj-oligopeptide suivant de longueur (n+1), qui est une partie de la séquence d'aminoacides de longueur (L) de la protéine, en tant que sujet pour prédire un site fonctionnel, et contient un résidu d'aminoacide Aj (n+1 $\leq$j$\leq$ L-n) à la j$^{ième}$ position de l'extrémité N-terminale de la séquence d'aminoacides de la protéine;

Aj-oligopeptide: aj1aj2....Aji....ajnaj(n+1)

(dans laquelle 1$\leq$ i$\leq$ n+1 ; Aj = Aji ; et Aj est le i$^{ième}$ résidu de l'oligopeptide),

et calculer dans la totalité des protéines de l'organisme *"a",* la fréquence d'occurrence du Xi-oligopeptide suivant de longueur (n+1);

Xi-oligopeptide: aj1aj2....Xji....ajnaj(n+1)

(dans lequel le i$^{ième}$ résidu Xji est n'importe quel aminoacide), et une unité de mémoire ayant la mémoire des résultats de calcul,

(e) une unité de calcul/mémoire composée d'une CPU calculant le rapport Yji de la fréquence d'occurrence du Aj-oligopeptide, à celle du Xi-oligopeptide, et une unité de mémoire ayant la mémoire de Yji,

(f) une unité de calcul/mémoire composée d'une CPU calculant la moyenne Yj des valeurs Yji ;

$$Yj = \sum_{i=1}^{n+1} Yji/(n+1),$$

et une unité de mémoire ayant la mémoire de Yj, et
(g) une unité de calcul/mémoire composée d'une CPU calculant la valeur fonctionnelle Zj de Yj ;

$$Zj = f(Yj)$$

(dans laquelle la fonction f est une fonction monotone décroissante ou une fonction monotone croissante),
et une unité de mémoire ayant la mémoire de Zj.

2. Système selon la revendication 1, dans lequel, dans le procédé effectué avec le système, la valeur Zj ($n+1 \leq j \leq$ L-n) de chaque résidu d'aminoacide dans la séquence d'aminoacides de longueur L, est représentée sous la forme d'un diagramme de distribution.

3. Système selon la revendication 1, qui comprend en outre une unité d'affichage affichant la valeur Zj ($n+1 \leq j \leq$ L-n) de chaque résidu d'aminoacide dans la séquence d'aminoacides de longueur L, sous la forme d'un diagramme de distribution.

4. Système pour effectuer automatiquement un procédé de prédiction d'un site fonctionnel d'une protéine dérivée de la totalité des protéines supposées d'un organisme "*a*" dont on connaît des données sur le génome ou des données sur l'ADNc, ce procédé comprenant les étapes consistant à :

(1) calculer la fréquence d'occurrence de chaque aminoacide, et la fréquence d'occurrence des oligopeptides individuels produits par les permutations de 20 aminoacides dans les séquences d'aminoacides de la totalité des protéines de l'organisme "*a*",
(2) quant à une protéine de l'organisme "*a*",
(2') calculer dans la totalité des protéines de l'organisme "*a*", la fréquence d'occurrence du Aj-oligopeptide suivant de longueur (n) donnée ($1 \leq n \leq$ M, à condition que M soit la plus petite longueur des oligopeptides satisfaisant au critère consistant en ce que la totalité des oligopeptides de longueur M aient une fréquence d'occurrence de 1), cet Aj-oligopeptide étant une partie de la séquence d'aminoacides de longueur (L) de la protéine, et contenant un résidu d'aminoacide Aj ($n \leq j \leq$ L-n+1) à la $j^{\text{ième}}$ position à partir de l'extrémité N-terminale de la séquence d'aminoacides de la protéine ;
Aj-oligopeptide: ajlaj2....aji....ajn (dans laquelle $1 \leq i \leq n+1$ ; Aj = aji, et Aj est le $i^{\text{ième}}$ résidu de l'oligopeptide),
et calculer dans la totalité des protéines de l'organisme "*a*", la fréquence d'occurrence du Xi-oligopeptide suivant de longueur (n), correspondant à la longueur du Aj-oligopeptide ;
Xi-oligopeptide: ajlaj2....Xi....ajn
(dans lequel le $i^{\text{ième}}$ résidu Xi est n'importe quel aminoacide),
(3) calculer le rapport Yji de la fréquence d'occurrence du Aj-oligopeptide, à celle du Xi-oligopeptide,
(4) calculer la moyenne Y(j,n) des valeurs Yji ;

$$Y(j, n) = \sum_{i=1}^{n} Yji/n ,$$

(5) calculer une valeur fonctionnelle Z(j,n) de Y(j,n) ;
(6) répéter successivement les étapes (2') à (5) et déterminer la valeur Z(j,n) de chaque résidu d'aminoacide Aj à la $j^{\text{ième}}$ position ($n \leq j \leq$ L-n+1) dans la séquence d'aminoacides de longueur (L),
(7) répéter séquentiellement les étapes (2) à (6) pour la totalité des protéines de l'organisme "*a*", de façon à déterminer la distribution de la valeur Z(j,n) de chaque résidu d'aminoacide dans la totalité des protéines, et les valeurs Z(j,n) sont réparties en vingt classes en fonction des vingt aminoacides, et calculer ensuite la moyenne Av(Aa) des valeurs Z(j,n) pour chaque aminoacide Aa, et l'écart-type Sd (Aa) de sa distribution, d'après la distribution, pour calculer une fonction g pour le $j^{ième}$ résidu d'aminoacide Aj d'une protéine, afin de normaliser la différence de distribution due aux espèces des résidus d'aminoacide ;

$$g = (Z(j,n), Aj) = [Z(j,n) - Av(Aa)]/Sd(Aa)$$

(dans laquelle Aj = Aa),
(8) calculer une valeur D(j,n) de la fonction g de chaque Aj de tous les résidus d'aminoacide à la $j^{ième}$ position ($n \leq j \leq L-n+1$) d'une protéine dans la totalité des protéines, comme obtenue dans l'étape (7) ;

$$D(j,n) = g(Z(j,n), Aj),$$

et
(9) définir la valeur représentative de la fonction du $j^{ème}$ résidu d'aminoacide dans la séquence d'aminoacides de longueur (L), en tant que valeur fonctionnelle Wj des Z(j,n) et D(j,n) ;

$$Wj = h(Z(j,l), Z(j,2),..,Z(j,M),D(j,l),D(j,2),…,D(j,M))$$

de façon à prédire le degré de l'implication de chaque résidu d'aminoacide dans la fonction de la protéine, en employant la dimension de la valeur Wj en tant qu'indicateur, ledit système comprenant au moins les unités (a) à (i) suivantes :

(a) une unité de mémoire externe mémorisant les données sur la séquence d'aminoacides de la totalité des protéines supposées d'un organisme *"a"* dont on connaît des données sur le génome ou des données sur l'ADNc, de même qu'une base de données existante sur les protéines,
(b) une unité de calcul/mémoire composée d'une CPU calculant la fréquence d'occurrence de chaque aminoacide, et la fréquence d'occurrence des oligopeptides individuels produits par les permutations de vingt aminoacides dans les séquences d'aminoacides de la totalité des protéines de l'organisme *"a"*, et une unité de mémoire ayant la mémoire des résultats de calcul,
(c) une unité de calcul/mémoire composée d'une CPU calculant dans la totalité des protéines de l'organisme *"a"*, la fréquence d'occurrence du Aj-oligopeptide suivant de longueur (n) donnée ($1 \leq n \leq M$, à condition que M soit la plus petite longueur des oligopeptides satisfaisant au critère consistant en ce que la totalité des oligopeptides de longueur M aient une fréquence d'occurrence de 1), cet Aj-oligopeptide étant une partie de la séquence d'aminoacides de longueur (L) d'une protéine donnée de l'organisme *"a"*, et contient un résidu d'aminoacide Aj ($n \leq j \leq L-n+1$) à la $j^{ième}$ position à partir de l'extrémité N-terminale de la séquence d'aminoacides de la protéine :
Aj-oligopeptide: ajlaj2....aji....ajn
(dans lequel $1 \leq i \leq n+1$ ; Aj = aji, et Aj est le $i^{ième}$ résidu de l'oligopeptide),
et calculer dans la totalité des protéines de l'organisme *"a"*, la fréquence d'occurrence du Xi-oligopeptide suivant de longueur (n) correspondant à la longueur du Aj-oligopeptide ;
Xi-oligopeptide: aj1aj2....Xji....ajn
(dans lequel le $i^{ième}$ résidu Xji est n'importe quel aminoacide), et une unité de mémoire mémorisant les résultats de calcul,
(d) une unité de calcul/ mémoire composée d'une CPU calculant le rapport Yji de la fréquence d'occurrence du Aj-oligopeptide, à celle du Xi-oligopeptide, et une unité de mémoire ayant la mémoire des Yji,
(e) une unité de calcul/ mémoire composée d'une CPU calculant la moyenne Yjn des valeurs Yji ;

$$Y(j,n) = \sum_{i=1}^{n} Yji/n,$$

et une unité de mémoire ayant la mémoire de Y(j,n),
(f) une unité de calcul/ mémoire composée d'une CPU calculant une valeur fonctionnelle Z(j,n) de Y(j,n) ;

$$Z(j,n) = -\log(Y(j,n)),$$

et une unité de mémoire ayant la mémoire de Z(j,n),
(g) une unité de calcul/mémoire composée d'une CPU calculant la valeur Z(j,n) de chaque résidu d'aminoacide dans les séquences d'aminoacides de la totalité des protéines de l'organisme *"a"*, calculant la distribution de la valeur Z(j,n) de chaque résidu d'aminoacide dans la totalité des protéines, et les valeurs Z(j,n) sont réparties en vingt classes en fonction des vingt aminoacides, et calculant ensuite la moyenne Av(Aa) des valeurs Z(j,n) pour chaque aminoacide Aa, et l'écart-type Sd (Aa) de sa distribution, d'après la distribution, pour déterminer une fonction g afin de normaliser la différence de distribution due aux espèces des résidus d'aminoacide ;

$$g = (Z(j,n), Aj) = [Z(j,n) - Av(Aa)]/Sd(Aa)$$

(dans laquelle Aj = Aa)
et une unité de mémoire ayant la mémoire de g,
(h) une unité de calcul/ mémoire composée d'une CPU calculant une valeur D(j,n) de la fonction g mémorisée dans l'unité (g), pour chacun de tous les résidus d'aminoacide Aj à la $j^{\text{ième}}$ position ($n \leq j \leq L\text{-}n+1$) dans la séquence d'aminoacides de longueur (L) ;

$$D(j,n) = g(Z(j,n), Aj)$$

et une unité de mémoire ayant la mémoire de la valeur D(j, n), et
(i) une unité de calcul/mémoire composée d'une unité de calcul calculant une valeur fonctionnelle appropriée Wj des Z(j,n) et D(j,n) de chaque résidu d'aminoacide dans la séquence d'aminoacides ;

$$Wj=h(Z(j,l),Z(j,2),..,Z(j,M),D(j,l),D(j,2),..,D(j,M))$$

et une unité de mémoire ayant la mémoire de la valeur Wj.

5. Système selon la revendication 4, dans lequel, dans le procédé effectué avec le système, la valeur Wj de chaque résidu d'aminoacide, est représentée sous la forme d'un diagramme de distribution bidimensionnel.

6. Système selon la revendication 4, dans lequel, dans le procédé effectué avec le système, la valeur Wj de chaque résidu d'aminoacide est représentée par un modèle structural tridimensionnel de la protéine.

7. Système selon la revendication 4, qui comprend en outre une unité d'affichage affichant la valeur Wj de chaque résidu d'aminoacide sous la forme d'un diagramme de distribution bidimensionnel.

8. Système selon la revendication 4, qui comprend en outre une unité de calcul/mémoire mémorisant une base de données existante sur la structure tridimensionnelle de protéines, ou préparant un modèle structural tridimensionnel basé sur une séquence d'aminoacides selon un procédé connu et mémorisant le modèle structural tridimensionnel, et une unité d'affichage affichant la valeur Wj de chaque résidu d'aminoacide dans la séquence d'aminoacides, sous la forme d'un diagramme de distribution sur la structure tridimensionnelle conservée dans la base de données, ou le modèle structural tridimensionnel mémorisé dans l'unité de calcul/mémoire.

9. Procédé de modification de la fonction connue d'une protéine *"A"* dérivée de la totalité des protéines d'un organisme *"a"* dont on connaît des données sur le génome ou des données sur l'ADNc, ce procédé comprenant les étapes consistant à :

(1) extraire une protéine étroitement apparentée à la protéine *"A"* d'après une base de données existante sur les protéines, et soumettre les protéines à un alignement,

(2) calculer la fréquence d'occurrence de chaque aminoacide et la fréquence d'occurrence des oligopeptides individuels produits par les permutations de vingt aminoacides dans les séquences d'aminoacides de la totalité des protéines de l'organisme *"a",* et déterminer la plus courte longueur (n) des oligopeptides satisfaisant aux critères suivants :

parmi les oligopeptides de longueur (n), le nombre des oligopeptides qui apparaissent une fois dans la totalité des protéines est inférieur au nombre des oligopeptides qui apparaissent deux fois dans la totalité des protéines ;

parmi les oligopeptides de longueur (n+1), le nombre des oligopeptides qui apparaissent une fois dans la totalité des protéines est supérieur au nombre des oligopeptides qui apparaissent deux fois dans la totalité des protéines,

(3) calculer dans la totalité des protéines de l'organisme *"a"*, la fréquence d'occurrence du Aj-oligopeptide suivant de longueur (n+1) qui est une partie de la séquence d'aminoacides de longueur (L) de la protéine *"A"*, et contient un résidu d'aminoacide Aj (n+1 $\leq$ j $\leq$ L-n) à la j$^{ième}$ position à partir de l'extrémité N-terminale de la séquence d'aminoacides de la protéine ;

Aj-oligopeptide: aj1aj2....Aji....ajnaj(n+1)

(dans lequel 1$\leq$ i$\leq$ n+1 ; Aj = Aji ; et Aj est le i$^{ième}$ résidu de l'oligopeptide),

et calculer dans la totalité des protéines de l'organisme *"a",* la fréquence d'occurrence du Xi-oligopeptide suivant de longueur (n+1) ;

Xi-oligopeptide: aj1aj2....Xji....ajnaj(n+1)

(dans lequel le résidu Xji est n'importe quel aminoacide),

(4) calculer le rapport Yji de la fréquence d'occurrence du Aj-oligopeptide, à celle du Xi-oligopeptide,

(5) calculer la moyenne Yj des valeurs Yji ;

$$Yj = \sum_{i=1}^{n+1} Yji/(n+1),$$

(6) calculer une valeur fonctionnelle Zj de Yj ;

$$Zj = f(Yj)$$

(dans laquelle la fonction f est une fonction monotone décroissante ou une fonction monotone croissante), et définir la valeur Zj en tant que valeur représentative de la fonction du j$^{ième}$ résidu d'aminoacide de la séquence d'aminoacides de longueur (L) de la protéine *"A",*

(7) répéter séquentiellement les étapes (3) à (6) et déterminer la valeur Zj de chacun de la totalité des résidus d'aminoacide à une position entre n+1 $\leq$ j $\leq$ L-n dans la séquence d'aminoacides de longueur (L),

(8) sélectionner au moins un résidu d'aminoacide destiné à être soumis à une mutation, d'après les données d'alignement obtenues dans l'étape (1) à partir de la séquence d'aminoacides de longueur (L) de la protéine *"A",* répéter séquentiellement les étapes (3) à (6) pour différents résidus d'aminoacide dans diverses séquences d'aminoacides mutées dans lesquelles le résidu d'aminoacide sélectionné a été muté en un autre résidu d'aminoacide, pour déterminer la valeur Zj des résidus d'aminoacide modifiés,

(9) sélectionner une séquence d'aminoacides mutée dans laquelle la valeur Zj du résidu d'aminoacide modifié, déterminée dans l'étape (8) est plus grande ou moins grande que la valeur Zj du résidu d'aminoacide de type sauvage, déterminée dans l'étape (7), et

(10) préparer un gène modifié codant pour la séquence d'aminoacides modifiée à partir du gène de la protéine "A", et produire la protéine modifiée en tant que produit d'expression du gène.

**10.** Procédé de modification de la fonction d'une protéine *"B"* dérivée d'un organisme "b" dont on ne connaît pas de données sur le génome ou de données sur l'ADNc, ce procédé comprenant les étapes consistant à :

(1) extraire la protéine "A" la plus étroitement apparentée à la protéine "B" à partir de la totalité des protéines d'un organisme *"a"* dont on connaît des données sur le génome ou des données sur l'ADNc, et soumettre la protéine à un alignement, ou extraire une protéine étroitement apparentée à la protéine *"B"* à partir d'une base de données existante sur les protéines, pour soumettre la protéine à un alignement,

(2) calculer dans les séquences d'aminoacides de la totalité des protéines de l'organisme "*a*", la fréquence d'occurrence de chaque aminoacide, et la fréquence d'occurrence des oligopeptides individuels produits par les permutations de vingt aminoacides, et déterminer la plus courte longueur (n) des oligopeptides satisfaisants aux critères suivants:

parmi les oligopeptides de longueur (n), le nombre des oligopeptides qui apparaissent une fois dans la totalité des protéines, est inférieur au nombre des oligopeptides qui apparaissent deux fois dans la totalité des protéines ; parmi les oligopeptides de longueur (n+1), le nombre des oligopeptides qui apparaissent une fois dans la totalité des protéines, est supérieur au nombre des oligopeptides qui apparaissent deux fois dans la totalité des protéines,

(3) calculer dans la totalité des protéines de l'organisme *"a"*, la fréquence d'occurrence du Aj-oligopeptide suivant de longueur (n+1), qui est une partie de la séquence d'aminoacides de longueur (L) de la protéine *"A"*, et contient un résidu d'aminoacide Aj ($n+1 \leq j \leq L-n$) à la j$^{ième}$ position à partir de l'extrémité N-terminale de la séquence d'aminoacides de la protéine ;

Ai-oligopeptide: aj1aj2....Aji....ajnaj(n+1)

(dans lequel $1 \leq i \leq n+1$ ; Aj = Aji ; et Aj est le i$^{ième}$ résidu de l'oligopeptide),

et calculer dans la totalité des protéines de l'organisme *"a"*, la fréquence d'occurrence du Xi-oligopeptide suivant de longueur (n+1) ;

Xi-oligopeptide: aj1aj2....Xji....ajnaj(n+1)

(dans lequel le i$^{ième}$ résidu Xji est n'importe quel aminoacide),

(4) calculer le rapport Yji de la fréquence d'occurrence du Aj-oligopeptide, à celle du Xi-oligopeptide,

(5) calculer la moyenne Yj des valeurs Yji ;

$$Yj = \sum_{i=1}^{n+1} Yji/(n+1),$$

(6) calculer une valeur fonctionnelle Zj de Yj ;

$$Zj = f(Yj)$$

(dans laquelle la fonction f est une fonction monotone décroissante ou une fonction monotone croissante), et définir la valeur Zj en tant que valeur représentative de la fonction du j$^{ième}$ résidu d'aminoacide Aj de la séquence d'aminoacides de longueur (L) de la protéine *"A"*,

(7) répéter séquentiellement les étapes (3) à (6) et déterminer la valeur Zj de chacun de la totalité des résidus d'aminoacide à une position entre $n+1 \leq j \leq L-n$ dans la séquence d'aminoacides de longueur (L),

(8) sélectionner au moins un résidu d'aminoacide destiné à être soumis à une mutation d'après les données d'alignement obtenues dans l'étape (1) à partir de la séquence d'aminoacides de longueur (L) de la protéine *"A"*, répéter séquentiellement les étapes (3) à (6) pour différent résidu d'aminoacide dans diverses séquences d'aminoacides mutées dans lesquelles le résidu d'aminoacide choisi a été muté en un autre résidu d'aminoacide, pour déterminer la valeur Zj des résidus d'aminoacide modifiés,

(9) sélectionner la position de mutation et le résidu d'aminoacide muté dans lesquels la valeur Zj du résidu d'aminoacide modifié, déterminée dans l'étape (8), est plus grande ou moins grande que la valeur Zj du résidu d'aminoacide de type sauvage, déterminée dans l'étape (7), et

(10) préparer un gène modifié codant pour la séquence d'aminoacides modifiée à partir du gène de la protéine *"B"* ayant le résidu d'aminoacide muté à la position, et produire la protéine modifiée en tant que produit d'expression du gène.

**11.** Procédé pour modifier la fonction connue d'une protéine *"A"* dérivée de la totalité des protéines d'un organisme *"a"* dont on connaît des données sur le génome ou des données sur l'ADNc, ce procédé comprenant les étapes consistant à :

(1) extraire les protéines étroitement apparentées à la protéine *"A"*, d'après une base de données existante sur les protéines, et soumettre les protéines à un alignement,

(2) calculer la fréquence d'occurrence de chaque aminoacide, et la fréquence d'occurrence des oligopeptides individuels produits par les permutations de vingt aminoacides, dans les séquences d'aminoacides de la totalité des protéines de l'organisme *"a"*,

(3) quant à une protéine de l'organisme "*a*",

(3') calculer dans la totalité des protéines de l'organisme *"a"*, la fréquence d'occurrence du Aj-oligopeptide suivant de longueur (n) donnée ($1 \le n \le M$, à condition que M soit la plus petite longueur des oligopeptides satisfaisant au critère consistant en ce que la totalité des oligopeptides de longueur M ont une fréquence d'occurrence de 1), cet Aj-oligopeptide étant une partie de la séquence d'aminoacides de la protéine, et contient un résidu d'aminoacide Aj ($n \le j \le L-n+1$) à la j$^{ième}$ position à partir de l'extrémité N-terminale de la protéine ;

Aj-oligopeptide: ajlaj2....aji....ajn

(dans laquelle $1 \le i \le n$ ; Aj = aji, et Aj est le i$^{ième}$ résidu de l'oligopeptide),

et calculer dans la totalité des protéines de l'organisme "*a*", la fréquence d'occurrence du Xi-oligopeptide suivant de longueur (n) correspondant à la longueur du Aj-oligopeptide ;

Xi-oligopeptide: aj1aj2....Xji....ajn

(dans lequel le i$^{ième}$ résidu Xji est n'importe quel aminoacide),

(4) calculer le rapport Yji de la fréquence d'occurrence du Aj-oligopeptide, à celle du Xi-oligopeptide,

(5) calculer la moyenne Y(j,n) des valeurs Yji ;

$$Y(j, n) = \sum_{i=1}^{n} Yji/n ,$$

(6) calculer une valeur fonctionnelle Z(j,n) de Y(j,n);

$$Z(j,n) = -\log(Y(j,n)),$$

(7) répéter successivement les étapes (3') à (6) et déterminer la valeur Z(j,n) de chaque résidu d'aminoacide Aj à une position j ($n \le j \le L-n+1$) dans la séquence d'aminoacides de longueur (L),

(8) répéter séquentiellement les étapes (3) à (7) pour la totalité des protéines de l'organisme *"a"*, de façon à déterminer la distribution de la valeur Z(j,n) de chaque résidu d'aminoacide dans la totalité des protéines, et les valeurs Z(j,n) sont réparties en vingt classes en fonction des vingt aminoacides, et calculer ensuite la moyenne Av(Aa) des valeurs Z(j,n) pour chaque aminoacide Aa, et l'écart-type Sd (Aa) de sa distribution, d'après la distribution, pour déterminer une fonction g pour le j$^{ième}$ résidu d'aminoacide Aj d'une protéine, afin de normaliser la différence de distribution due aux espèces des résidus d'aminoacide ;

$$g = (Z(j,n), Aj) = [Z(j,n) - Av(Aa)]/Sd(Aa)$$

(dans laquelle Aj = Aa),

(9) calculer une valeur D(j,n) de la fonction g de chaque Aj de la totalité des résidus d'aminoacide à la j$^{ième}$ position ($n \le j \le L-n+1$) d'une protéine dans la totalité des protéines, obtenue dans l'étape (8) ;

$$D(j,n) = g(Z(j,n), Aj),$$

(10) définir la valeur représentative de la fonction du j$^{ème}$ résidu d'aminoacide dans la séquence d'aminoacides de longueur (L), en tant que valeur fonctionnelle Wj des valeurs Z(j,n) et D(j,n) ;

$$Wj=h(Z(j,l),Z(j,2),..,Z(j,M),D(j,l),D(j,2),…,D(j,M))$$

(11) répéter séquentiellement les étapes (3) à (10) pour déterminer les valeurs Wj individuelles de tous les résidus d'aminoacide à la position (n+1 ≤j≤ L-n) dans la séquence d'aminoacides de longueur (L),

(12) sélectionner au moins un résidu d'aminoacide destiné à être soumis à une mutation d'après les données d'alignement obtenues dans l'étape (1) à partir de la séquence d'aminoacides de longueur (L) de la protéine "A", et répéter séquentiellement les étapes (3) à (10) pour différents résidus d'aminoacide dans diverses séquences d'aminoacides mutées dans lesquelles le résidu d'aminoacide sélectionné a été muté en un autre résidu d'aminoacide, afin de déterminer la valeur Wj du résidu d'aminoacide modifié,

(13) sélectionner une séquence d'aminoacides mutée dans laquelle la valeur Wj du résidu d'aminoacide modifié, déterminée dans l'étape (12) est plus grande ou moins grande que la valeur Wj du résidu d'aminoacide acide type sauvage, déterminée dans l'étape (10), et

(14) préparer un gène modifié codant pour la séquence d'aminoacides modifiées, à partir du gène de la protéine "A", et produire la protéine modifiée en tant que produit d'expression du gène.

# Fig. 1

*Fig. 2*

```
         1               20
{1}      12345678901234567890
         LGMSMGKIKIDALIDNTYKT      Sequence (1)

              GMSM  ◄────────  Aj-oligopeptide (j=5, i=4) of length  4,
              MSMG              containing the 5-th Met of the Sequence (1)
{2}           SMGK
              MGKI ◄───────  Aj-oligopeptide (j=5, i=1)


          {3}     {4}     {5}     {6}

          GMSN    MSNG    SNGK    NGKI
          GMSD    MSDG    SDGK    DGKI
          GMSA    MSAG    SAGK    AGKI
          GMSR    MSRG    SRGK    RGKI
          GMSI    MSIG    SIGK    IGKI
          GMSG    MSGG    SGGK    GGKI
          GMSQ    MSQG    SQGK    QGKI
          GMSE    MSEG    SEGR    EGKI
          GMSC    MSCG    SCGK    CGKI
          GMSS    MSSG    SSGK    SGKI
          GMSY    MSYG    SYGK    YGKI
          GMSW    MSWG    SWGK    WGKI
          GMST    MSTG    STGK    TGKI
          GMSV    MSVG    SVGK    VGKI
          GMSH    MSHG    SHGK    HGKI
          GMSF    MSFG    SFGK    FGKI
          GMSP    MSPG    SPGK    PGKI
          GMSM    MSMG    SMGK    MGKI
          GMSK    MSKG    SKGK    KGKI
          GMSL    MSLG    SLGK    LGKI
```

Xi-oligopeptide (i=4) for
the oligopeptide GMSM
of length  4, containing
the 5-th Met of the Sequence (1)

Xi-oligopeptide (i=1) for
the oligopeptide MGKI
of length  4, containing
the 5-th Met of the Sequence (1)

# Fig. 3

# Fig. 4

```
                    ┌─────────┐
                    │  START  │
                    └────┬────┘
                         ▼
                 ┌───────────────┐
                 │  freq ()←0    │
                 └───────┬───────┘
                         ▼
                 ┌───────────────┐
                 │     p←1       │
                 └───────┬───────┘
                         ▼
                    ╱─────────╲      No
                   ⟨   p≦N    ⟩──────────►  END
                    ╲─────────╱
                         │ Yes
                         ▼
           ┌───────────────────────────────┐
           │  L←length of the p-th protein │
           └───────────────┬───────────────┘
                           ▼
   ┌────────────────────────────────────────────────┐
   │  A₁A₂---A_L ← amino acid sequence of the p-th protein │
   └───────────────────────┬────────────────────────┘
                           ▼
                   ┌───────────────┐
                   │     i←1       │
                   └───────┬───────┘
                           ▼
   ┌──────────────────────────────────────────────────┐
   │ freq (AᵢAᵢ₊₁---Aᵢ₊ₙ₋₁)←freq (AᵢAᵢ₊₁---Aᵢ₊ₙ₋₁)+1 │
   └───────────────────────┬──────────────────────────┘
                           ▼
                   ┌───────────────┐
                   │    i←i+1      │
                   └───────┬───────┘
                           ▼
                      ╱─────────╲      Yes
                     ⟨ i≦L-n+1  ⟩──────────►
                      ╲─────────╱
                           │ No
                           ▼
                   ┌───────────────┐
                   │    p←p+1      │
                   └───────────────┘
```

$$A_1A_2\text{---}A_L \leftarrow \text{amino acid sequence of the p-th protein}$$

$$\text{freq}(A_iA_{i+1}\text{---}A_{i+n-1}) \leftarrow \text{freq}(A_iA_{i+1}\text{---}A_{i+n-1})+1$$

*Fig. 5*

```
                    ┌─────────┐
                    │  START  │
                    └────┬────┘
                         ▼
                  ┌────────────┐
                  │   i←1      │
                  └─────┬──────┘
                        ▼
                  ┌────────────┐◄───────────────────────────┐
                  │   j←1      │                            │
                  └─────┬──────┘                            │
                        ▼                                    │
                  ┌────────────┐◄──────────────────────┐    │
                  │   S←0      │                        │    │
                  └─────┬──────┘                        │    │
                        ▼                                │    │
                  ┌────────────┐                         │    │
                  │   Aa←1     │                         │    │
                  └─────┬──────┘                         │    │
                        ▼                                 │    │
  ┌──────────────────────────────────────────────────┐◄─┐   │    │
  │ S←S+freq (substituting the j-th residue in        │  │   │    │
  │           AᵢAᵢ₊₁---Aᵢ₊ₙ₋₁ with Aa)                 │  │   │    │
  └─────────────────────┬────────────────────────────┘  │   │    │
                        ▼                                 │   │    │
                  ┌────────────┐                          │   │    │
                  │  Aa←Aa+1   │                          │   │    │
                  └─────┬──────┘                          │   │    │
                        ▼                         Yes      │   │    │
                  ╱──────────────╲  ───────────────────────┘   │    │
                  ╲   Aa≦20      ╱                             │    │
                   ╲────┬───────╱                              │    │
                        │ No                                    │    │
                        ▼                                        │    │
  ┌──────────────────────────────────────────────┐             │    │
  │ Yᵢ₊ⱼ₋₁,ᵢ←freq (AᵢAᵢ₊₁---Aᵢ₊ₙ₋₁)/S             │             │    │
  └─────────────────────┬────────────────────────┘             │    │
                        ▼                                        │    │
                  ┌────────────┐                                 │    │
                  │   j←j+1    │                                 │    │
                  └─────┬──────┘                                 │    │
                        ▼                        Yes             │    │
                  ╱──────────────╲  ──────────────────────────────┘    │
                  ╲    j≦n       ╱                                     │
                   ╲────┬───────╱                                      │
                        │ No                                            │
                        ▼                                                │
                  ┌────────────┐                                         │
                  │   i←i+1    │                                         │
                  └─────┬──────┘                                         │
                        ▼                        Yes                     │
                  ╱──────────────╲  ──────────────────────────────────────┘
                  ╲   i≦L-n+1    ╱
                   ╲────┬───────╱
                        │ No
                        ▼
                    ┌─────────┐
                    │   END   │
                    └─────────┘
```

*Fig. 6*

## Fig. 7

*Fig. 8*

START

Aa←1

c←0

S←0

V←0

p←1

L←length of the p-th protein

$A_1 A_2 --- A_L$←sequence of the p-th protein

j←n

Aj=Aa —No→

Yes

c←c+1

S←S+Z(j,n)

V←V+Z(j,n)^2

j←j+1

j≦L-n+1 —Yes

No

p←p+1

p≦N —Yes

No

Av(Aa) ←S/c

Sd(Aa) ←$\sqrt{}$(V/c-Av(Aa)^2)

Aa←Aa+1

Aa≦20 —Yes

No

END

## Fig. 9

START

j ← n

$D(j, n) \leftarrow (Z(j, n) - Av(Aj))/Sd(Aj)$

j ← j+1

j ≦ L·n+1    Yes

No

END

Fig. 10

START

$j \leftarrow 1$

$Wj \leftarrow h(Z(j,1), Z(j,2) \cdots Z(j,M), D(j,1), D(j,2), \cdots, D(j,M))$

$j \leftarrow j+1$

$j \leqq L$  —— Yes

No

END

## Fig. 11

```
┌─────────────────────────────┐
│ Outer Memory Unit (a):      │
│  memorizing protein data    │
└─────────────────────────────┘
```

```
┌──────────────────────────────────────────────────────────────┐
│  ┌──────────────────────────────────────────┐    ┌─────────┐ │
│  │ Calculation/Memory Unit (b):             │    │         │ │
│  │  process of the frequency of occurrence  │    │         │ │
│  │  of oligopeptide                         │    │         │ │
│  └──────────────────────────────────────────┘    │         │ │
│  ┌──────────────────────────────────────────┐    │         │ │
│  │ Calculation/Memory Unit (c):             │    │         │ │
│  │  process of the smallest n               │    │         │ │
│  └──────────────────────────────────────────┘    │    ╭─╮  │ │
│  ┌──────────────────────────────────────────┐    │    c    │ │
│  │ Calculation/Memory Unit (d):             │    │    Control Unit (n) │
│  │  process of the frequency of occurrences of │  │         │ │
│  │  Aj- and Xi-oligopeptides                │    │         │ │
│  └──────────────────────────────────────────┘    │         │ │
│  ┌──────────────────────────────────────────┐    │         │ │
│  │ Calculation/Memory Unit (e):             │    │         │ │
│  │  process of Yji                          │    │         │ │
│  └──────────────────────────────────────────┘    │         │ │
│  ┌──────────────────────────────────────────┐    │         │ │
│  │ Calculation/Memory Unit (f):             │    │         │ │
│  │  process of Yi, a mean value of Yji      │    │         │ │
│  └──────────────────────────────────────────┘    │         │ │
│  ┌──────────────────────────────────────────┐    │         │ │
│  │ Calculation/Memory Unit (g):             │    │         │ │
│  │  process of Zj, a functional value of Yi │    │         │ │
│  └──────────────────────────────────────────┘    └─────────┘ │
└──────────────────────────────────────────────────────────────┘
```

```
┌──────────────────────────┐      ┌──────────────────────────┐
│    Display Unit (h)       │      │     Keyboard (i)         │
└──────────────────────────┘      └──────────────────────────┘
```

## Fig. 12

**Outer Memory Unit (a):**
memorizing protein data

**Calculation/Memory Unit (b):**
process of the frequency of occurrence
of oligopeptide

**Calculation/Memory Unit (c):**
process of the frequency of occurrences of
Aj- and Xi-oligopeptides

**Calculation/Memory Unit (d):**
process of Yji

**Calculation/Memory Unit (e):**
process of Y(j,n), a mean value of Yji

**Calculation/Memory Unit (f):**
process of Z(j,n), a logarithmic value of Y(j,n)

**Calculation/Memory Unit (g):**
process of statistic values of Z(j,n)

**Calculation/Memory Unit (h):**
process of D(j,n), a standard score of Z(j,n)

**Calculation/Memory Unit (i):**
process of Wj, a functional value of Z(j,n) and D(j,n)

**Calculation/Memory Unit (k):**
process of a three-dimensional structure

**Control Unit (n)**

**Display Unit (l)**   **Display Unit (j)**   **Keyboard (m)**

EP 1 013 759 B1

# Fig. 13

**Pfu Pol.**  **KOD Pol.**

0.5 1.0 3.0   0.5 1.0 3.0   min

base

1353-
1098-
872-
603-

310-
281-
271-

234-

194-

118-

72-

# Fig. 14

*Fig. 15*

motif A

MJ0558 Position 325..346

motif C

MJ0558 Position 550..570

*Fig. 16*

Frequency

Zj

*Fig. 17*

motif C

```
          550                    570
MJ    AEKFGFKVLYIDTDGFYAIWK
KOD   EEKYGFKVIYSDTDGFFATIP
Pfu   EEKFGFKVLYIDTDGLYATIP
```

Fig. 18

*Fig. 19*

*Fig. 20*

*Fig. 21*

Fig. 22

*Fig. 23*

*Fig. 24*

base

1353-
1098-
872-
603-

310-
281-
271-

234-

194-

118-

Wild-type

Wild-type

Modified-type (pDP5b17)

Wild-type

Modified-type (pDP5C4)

SEQUENCE LISTING

<110> Japan Science and Technology Corporation

<120> Method and Apparatus for Predicting Protein Function Site, Method for Improving Protein Function and Function-Improved Protein

<130> 31.17.70591

<140> 98901095.4
<141> 1998-02-02

<150> JP 1924897
<151> 1997-01-31

<150> JP 1924997
<151> 1997-01-31

<150> JP 33210097
<151> 1997-12-02

<150> JP 1869998
<151> 1998-01-30

<160> 11

<170> PatentIn Ver. 2.1

<210> 1
<211> 775
<212> PRT
<213> Artificial Sequence

<220>
<223> Description of Artificial Sequence: modified DNA polymerase I

<400> 1

```
Met Val Leu Asp Val Asp Tyr Ile Thr Glu Glu Gly Lys Pro Val Ile
 1               5                  10                  15

Arg Leu Phe Lys Lys Glu Asn Gly Lys Phe Lys Ile Glu His Asp Arg
            20                  25                  30

Thr Phe Arg Pro Tyr Ile Tyr Ala Leu Leu Arg Asp Asp Ser Lys Ile
        35                  40                  45
```

```
Glu Glu Val Lys Lys Ile Thr Gly Glu Arg His Gly Lys Ile Val Arg
    50                      55                  60

Ile Val Asp Val Glu Lys Val Glu Lys Lys Phe Leu Gly Lys Pro Ile
    65                  70                  75                  80

Thr Val Trp Lys Leu Tyr Leu Glu His Pro Gln Asp Val Pro Thr Ile
                    85                  90                  95

Arg Glu Lys Val Arg Glu His Pro Ala Val Val Asp Ile Phe Glu Tyr
            100                 105                 110

Asp Ile Pro Phe Ala Lys Arg Tyr Leu Ile Asp Lys Gly Leu Ile Pro
            115                 120                 125

Met Glu Gly Glu Glu Glu Leu Lys Ile Leu Ala Phe Asp Ile Glu Thr
        130                 135                 140

Leu Tyr His Glu Gly Glu Glu Phe Gly Lys Gly Pro Ile Ile Met Ile
145                 150                 155                 160

Ser Tyr Ala Asp Glu Asn Glu Ala Lys Val Ile Thr Trp Lys Asn Ile
                165                 170                 175

Asp Leu Pro Tyr Val Glu Val Val Ser Ser Glu Arg Glu Met Ile Lys
            180                 185                 190

Arg Phe Leu Arg Ile Ile Arg Glu Lys Asp Pro Asp Ile Ile Val Thr
            195                 200                 205

Tyr Asn Gly Asp Ser Phe Asp Phe Pro Tyr Leu Ala Lys Arg Ala Glu
    210                 215                 220

Lys Leu Gly Ile Lys Leu Thr Ile Gly Arg Asp Gly Ser Glu Pro Lys
225                 230                 235                 240

Met Gln Arg Ile Gly Asp Met Thr Ala Val Glu Val Lys Gly Arg Ile
                245                 250                 255

His Phe Asp Leu Tyr His Val Ile Thr Arg Thr Ile Asn Leu Pro Thr
            260                 265                 270

Tyr Thr Leu Glu Ala Val Tyr Glu Ala Ile Phe Gly Lys Pro Lys Glu
            275                 280                 285

Lys Val Tyr Ala Asp Glu Ile Ala Lys Ala Trp Glu Ser Gly Glu Asn
    290                 295                 300
```

```
Leu Glu Arg Val Ala Lys Tyr Ser Met Glu Asp Ala Lys Ala Thr Tyr
305                 310             315                 320

Glu Leu Gly Lys Glu Phe Leu Pro Met Glu Ile Gln Leu Ser Arg Leu
                325             330                 335

Val Gly Gln Pro Leu Trp Asp Val Ser Arg Ser Ser Thr Gly Asn Leu
            340             345             350

Val Glu Trp Phe Leu Leu Arg Lys Ala Tyr Glu Arg Asn Glu Val Ala
            355             360             365

Pro Asn Lys Pro Ser Glu Glu Glu Tyr Gln Arg Arg Leu Arg Glu Ser
    370             375             380

Tyr Thr Gly Gly Phe Val Lys Glu Pro Glu Lys Gly Leu Trp Glu Asn
385             390             395                 400

Ile Val Tyr Leu Asp Phe Arg Ala Leu Tyr Pro Ser Ile Ile Ile Thr
            405             410             415

His Asn Val Ser Pro Asp Thr Leu Asn Leu Glu Gly Cys Lys Asn Tyr
            420             425             430

Asp Ile Ala Pro Gln Val Gly His Lys Phe Cys Lys Asp Ile Pro Gly
    435             440             445

Phe Ile Pro Ser Leu Leu Gly His Leu Leu Glu Glu Arg Gln Lys Ile
    450             455             460

Lys Thr Lys Met Lys Glu Thr Gln Asp Pro Ile Glu Lys Ile Leu Leu
465             470             475                 480

Asp Tyr Arg Gln Lys Ala Ile Lys Leu Leu Ala Asn Ser Phe Tyr Gly
            485             490             495

Tyr Tyr Gly Tyr Ala Lys Ala Arg Trp Tyr Cys Lys Glu Cys Ala Glu
            500             505             510

Ser Val Thr Ala Trp Gly Arg Lys Tyr Ile Glu Leu Val Trp Lys Glu
            515             520             525

Leu Glu Glu Lys Tyr Gly Phe Lys Val Ile Tyr Ser Asp Thr Asp Gly
    530             535             540

Phe Phe Ala Thr Ile Pro Gly Gly Glu Ser Glu Glu Ile Lys Lys Lys
545             550             555                 560
```

Ala Leu Glu Phe Val Lys Tyr Ile Asn Ser Lys Leu Pro Gly Leu Leu
565 570 575

Glu Leu Glu Tyr Glu Gly Phe Tyr Lys Arg Gly Phe Phe Val Thr Lys
580 585 590

Lys Arg Tyr Ala Val Ile Asp Glu Glu Gly Lys Val Ile Thr Arg Gly
595 600 605

Leu Glu Ile Val Arg Arg Asp Trp Ser Glu Ile Ala Lys Glu Thr Gln
610 615 620

Ala Arg Val Leu Glu Thr Ile Leu Lys His Gly Asp Val Glu Glu Ala
625 630 635 640

Val Arg Ile Val Lys Glu Val Ile Gln Lys Leu Ala Asn Tyr Glu Ile
645 650 655

Pro Pro Glu Lys Leu Ala Ile Tyr Glu Gln Ile Thr Arg Pro Leu His
660 665 670

Glu Tyr Lys Ala Ile Gly Pro His Val Ala Val Ala Lys Lys Leu Ala
675 680 685

Ala Lys Gly Val Lys Ile Lys Pro Gly Met Val Ile Gly Tyr Ile Val
690 695 700

Leu Arg Gly Asp Gly Pro Ile Ser Asn Arg Ala Ile Leu Ala Glu Glu
705 710 715 720

Tyr Asp Pro Lys Lys His Lys Tyr Asp Ala Glu Tyr Tyr Ile Glu Asn
725 730 735

Gln Val Leu Pro Ala Val Leu Arg Ile Leu Glu Gly Phe Gly Tyr Arg
740 745 750

Lys Glu Asp Leu Arg Tyr Gln Lys Thr Arg Gln Val Gly Leu Thr Ser
755 760 765

Trp Leu Asn Ile Lys Lys Ser
770 775


<210> 2
<211> 35
<212> DNA

<213> Artificial Sequence

<220>
<223> Description of Artificial Sequence: synthetic DNA

<400> 2
gtggggagca ccatggtttt agatgtggat tacat          35

<210> 3
<211> 35
<212> DNA
<213> Artificial Sequence

<220>
<223> Description of Artificial Sequence: synthetic DNA

<400> 3
gcatgcagat agaccatttc taacgaaggc gtttg          35

<210> 4
<211> 66
<212> DNA
<213> Artificial Sequence

<220>
<223> Description of Artificial Sequence: synthetic DNA

<400> 4

```
ctcgaagaaa agtatggatt taaagtcatc tacagtgaca ctgatggttt ctttgcaact 60
atccca                                                            66
```

<210> 5
<211> 66
<212> DNA
<213> Artificial Sequence

<220>
<223> Description of Artificial Sequence: synthetic DNA

<400> 5

```
tgggatagtt gcaaagaaac catcagtgtc actgtagatg actttaaatc catacttttc 60
ttcgag                                                            66
```

<210> 6
<211> 775
<212> PRT
<213> Artificial Sequence

<220>
<223> Description of Artificial Sequence: modified DNA polymerase II

<400> 6

```
Met Val Leu Asp Val Asp Tyr Ile Thr Glu Glu Gly Lys Pro Val Ile
 1               5                  10                  15

Arg Leu Phe Lys Lys Glu Asn Gly Lys Phe Lys Ile Glu His Asp Arg
            20                  25                  30

Thr Phe Arg Pro Tyr Ile Tyr Ala Leu Leu Arg Asp Asp Ser Lys Ile
        35                  40                  45

Glu Glu Val Lys Lys Ile Thr Gly Glu Arg His Gly Lys Ile Val Arg
    50                  55                  60

Ile Val Asp Val Glu Lys Val Glu Lys Lys Phe Leu Gly Lys Pro Ile
65                  70                  75                  80

Thr Val Trp Lys Leu Tyr Leu Glu His Pro Gln Asp Val Pro Thr Ile
            85                  90                  95

Arg Glu Lys Val Arg Glu His Pro Ala Val Val Asp Ile Phe Glu Tyr
            100                 105                 110

Asp Ile Pro Phe Ala Lys Arg Tyr Leu Ile Asp Lys Gly Leu Ile Pro
        115                 120                 125

Met Glu Gly Glu Glu Glu Leu Lys Ile Leu Ala Phe Asp Ile Glu Thr
    130                 135                 140

Leu Tyr His Glu Gly Glu Glu Phe Gly Lys Gly Pro Ile Ile Met Ile
145                 150                 155                 160

Ser Tyr Ala Asp Glu Asn Glu Ala Lys Val Ile Thr Trp Lys Asn Ile
            165                 170                 175

Asp Leu Pro Tyr Val Glu Val Val Ser Ser Glu Arg Glu Met Ile Lys
        180                 185                 190

Arg Phe Leu Arg Ile Ile Arg Glu Lys Asp Pro Asp Ile Ile Val Thr
        195                 200                 205

Tyr Asn Gly Asp Ser Phe Asp Phe Pro Tyr Leu Ala Lys Arg Ala Glu
    210                 215                 220

Lys Leu Gly Ile Lys Leu Thr Ile Gly Arg Asp Gly Ser Glu Pro Lys
225                 230                 235                 240
```

```
Met Gln Arg Ile Gly Asp Met Thr Ala Val Glu Val Lys Gly Arg Ile
              245             250             255

His Phe Asp Leu Tyr His Val Ile Thr Arg Thr Ile Asn Leu Pro Thr
              260             265             270

Tyr Thr Leu Glu Ala Val Tyr Glu Ala Ile Phe Gly Lys Pro Lys Glu
              275             280             285

Lys Val Tyr Ala Asp Glu Ile Ala Lys Ala Trp Glu Ser Gly Glu Asn
    290             295             300

Leu Glu Arg Val Ala Lys Tyr Ser Met Glu Asp Ala Lys Ala Thr Tyr
305             310             315             320

Glu Leu Gly Lys Glu Phe Leu Pro Met Glu Ile Gln Leu Ser Arg Leu
              325             330             335

Val Gly Gln Pro Leu Trp Asp Val Ser Arg Ser Ser Thr Gly Asn Leu
              340             345             350

Val Glu Trp Phe Leu Leu Arg Lys Ala Tyr Glu Arg Asn Glu Val Ala
              355             360             365

Pro Asn Lys Pro Ser Glu Glu Glu Tyr Gln Arg Arg Leu Arg Glu Ser
    370             375             380

Tyr Thr Gly Gly Phe Val Lys Glu Pro Glu Lys Gly Leu Trp Glu Asn
385             390             395             400

Ile Val Tyr Leu Asp Phe Arg Ala Leu Tyr Pro Ser Ile Ile Ile Thr
              405             410             415

His Asn Val Ser Pro Asp Thr Leu Asn Leu Glu Gly Cys Lys Asn Tyr
              420             425             430

Asp Ile Ala Pro Gln Val Gly His Lys Phe Cys Lys Asp Ile Pro Gly
    435             440             445

Phe Ile Pro Ser Leu Leu Gly His Leu Leu Glu Glu Arg Gln Lys Ile
    450             455             460

Lys Thr Lys Met Lys Glu Thr Gln Asp Pro Ile Glu Lys Ile Leu Leu
465             470             475             480

Asp Tyr Arg Gln Lys Ala Ile Lys Leu Leu Ala Asn Ser Phe Tyr Gly
              485             490             495
```

Tyr Tyr Gly Tyr Ala Lys Ala Arg Trp Tyr Cys Lys Glu Cys Ala Glu
500                    505                    510

Ser Val Thr Ala Trp Gly Arg Lys Tyr Ile Glu Leu Val Trp Lys Glu
515                    520                    525

Leu Glu Glu Lys Phe Gly Phe Lys Val Leu Tyr Ile Asp Thr Asp Gly
530                    535                    540

Leu Tyr Ala Thr Ile Pro Gly Gly Glu Ser Glu Glu Ile Lys Lys Lys
545                    550                    555                    560

Ala Leu Glu Phe Val Lys Tyr Ile Asn Ser Lys Leu Pro Gly Leu Leu
565                    570                    575

Glu Leu Glu Tyr Glu Gly Phe Tyr Lys Arg Gly Phe Phe Val Thr Lys
580                    585                    590

Lys Arg Tyr Ala Val Ile Asp Glu Glu Gly Lys Val Ile Thr Arg Gly
595                    600                    605

Leu Glu Ile Val Arg Arg Asp Trp Ser Glu Ile Ala Lys Glu Thr Gln
610                    615                    620

Ala Arg Val Leu Glu Thr Ile Leu Lys His Gly Asp Val Glu Glu Ala
625                    630                    635                    640

Val Arg Ile Val Lys Glu Val Ile Gln Lys Leu Ala Asn Tyr Glu Ile
645                    650                    655

Pro Pro Glu Lys Leu Ala Ile Tyr Glu Gln Ile Thr Arg Pro Leu His
660                    665                    670

Glu Tyr Lys Ala Ile Gly Pro His Val Ala Val Ala Lys Lys Leu Ala
675                    680                    685

Ala Lys Gly Val Lys Ile Lys Pro Gly Met Val Ile Gly Tyr Ile Val
690                    695                    700

Leu Arg Gly Asp Gly Arg Ile Arg Asp Arg Ala Ile Pro Ala Glu Glu
705                    710                    715                    720

Tyr Asp Pro Lys Lys His Lys Tyr Asp Ala Glu Tyr Tyr Ile Glu Asn
725                    730                    735

Gln Val Leu Pro Ala Val Leu Arg Ile Leu Glu Gly Phe Gly Tyr Arg
740                    745                    750

```
        Lys Glu Asp Leu Arg Tyr Gln Lys Thr Arg Gln Val Gly Leu Thr Ser
            755                 760                 765

        Trp Leu Asn Ile Lys Lys Ser
            770                 775
```

<210> 7
<211> 775
<212> PRT
<213> Artificial Sequence

<220>
<223> Description of Artificial Sequence: modified DNA polymerase III

<400> 7

```
        Met Val Leu Asp Val Asp Tyr Ile Thr Glu Glu Gly Lys Pro Val Ile
          1                 5                  10                  15

        Arg Leu Phe Lys Lys Glu Asn Gly Lys Phe Lys Ile Glu His Asp Arg
                        20                  25                  30

        Thr Phe Arg Pro Tyr Ile Tyr Ala Leu Leu Arg Asp Asp Ser Lys Ile
                    35                  40                  45

        Glu Glu Val Lys Lys Ile Thr Gly Glu Arg His Gly Lys Ile Val Arg
              50                  55                  60

        Ile Val Asp Val Glu Lys Val Glu Lys Lys Phe Leu Gly Lys Pro Ile
          65                  70                  75                  80

        Thr Val Trp Lys Leu Tyr Leu Glu His Pro Gln Asp Val Pro Thr Ile
                        85                  90                  95

        Arg Glu Lys Val Arg Glu His Pro Ala Val Val Asp Ile Phe Glu Tyr
                       100                 105                 110

        Asp Ile Pro Phe Ala Lys Arg Tyr Leu Ile Asp Lys Gly Leu Ile Pro
                       115                 120                 125

        Met Glu Gly Glu Glu Glu Leu Lys Ile Leu Ala Phe Asp Ile Glu Thr
                       130                 135                 140

        Leu Tyr His Glu Gly Glu Glu Phe Gly Lys Gly Pro Ile Ile Met Ile
            145                 150                 155                 160

        Ser Tyr Ala Asp Glu Asn Glu Ala Lys Val Ile Thr Trp Lys Asn Ile
```

                                    165                          170                          175

Asp Leu Pro Tyr Val Glu Val Val Ser Ser Glu Arg Glu Met Ile Lys
            180                     185                     190

Arg Phe Leu Arg Ile Ile Arg Glu Lys Asp Pro Asp Ile Ile Val Thr
            195                     200                     205

Tyr Asn Gly Asp Ser Phe Asp Phe Pro Tyr Leu Ala Lys Arg Ala Glu
    210                     215                     220

Lys Leu Gly Ile Lys Leu Thr Ile Gly Arg Asp Gly Ser Glu Pro Lys
225                     230                     235                     240

Met Gln Arg Ile Gly Asp Met Thr Ala Val Glu Val Lys Gly Arg Ile
            245                     250                     255

His Phe Asp Leu Tyr His Val Ile Thr Arg Thr Ile Asn Leu Pro Thr
            260                     265                     270

Tyr Thr Leu Glu Ala Val Tyr Glu Ala Ile Phe Gly Lys Pro Lys Glu
            275                     280                     285

Lys Val Tyr Ala Asp Glu Ile Ala Lys Ala Trp Glu Ser Gly Glu Asn
    290                     295                     300

Leu Glu Arg Val Ala Lys Tyr Ser Met Glu Asp Ala Lys Ala Thr Tyr
305                     310                     315                     320

Glu Leu Gly Lys Glu Phe Leu Pro Met Glu Ile Gln Leu Ser Arg Leu
            325                     330                     335

Val Gly Gln Pro Leu Trp Asp Val Ser Arg Ser Ser Thr Gly Asn Leu
            340                     345                     350

Val Glu Trp Phe Leu Leu Arg Lys Ala Tyr Glu Arg Asn Glu Val Ala
            355                     360                     365

Pro Asn Lys Pro Ser Glu Glu Glu Tyr Gln Arg Arg Leu Arg Glu Ser
    370                     375                     380

Tyr Thr Gly Gly Phe Val Lys Glu Pro Glu Lys Gly Leu Trp Glu Asn
385                     390                     395                     400

Ile Val Tyr Leu Asp Phe Arg Ala Leu Tyr Pro Ser Ile Ile Ile Thr
            405                     410                     415

His Asn Val Ser Pro Asp Thr Leu Asn Leu Glu Gly Cys Lys Asn Tyr

```
                    420                    425                    430

        Asp Ile Ala Pro Gln Val Gly His Lys Phe Cys Lys Asp Ile Pro Gly
                435                440                445

        Phe Ile Pro Ser Leu Leu Gly His Leu Leu Glu Glu Arg Gln Lys Ile
            450                455                460

        Lys Thr Lys Met Lys Glu Thr Gln Asp Pro Ile Glu Lys Ile Leu Leu
        465                470                475                480

        Asp Tyr Arg Gln Lys Ala Ile Lys Leu Leu Ala Asn Ser Phe Tyr Gly
                485                490                495

        Tyr Tyr Gly Tyr Ala Lys Ala Arg Trp Tyr Cys Lys Glu Cys Ala Glu
                500                505                510

        Ser Val Thr Ala Trp Gly Arg Lys Tyr Ile Glu Leu Val Trp Lys Glu
            515                520                525

        Leu Glu Glu Lys Phe Gly Phe Lys Val Leu Tyr Ile Asp Thr Asp Gly
            530                535                540

        Leu Tyr Ala Thr Ile Pro Gly Gly Glu Ser Glu Glu Ile Lys Lys Lys
        545                550                555                560

        Ala Leu Glu Phe Val Lys Tyr Ile Asn Ser Lys Leu Pro Gly Leu Leu
                565                570                575

        Glu Leu Glu Tyr Glu Gly Phe Tyr Lys Arg Gly Phe Phe Val Thr Lys
                580                585                590

        Lys Arg Tyr Ala Val Ile Asp Glu Glu Gly Lys Val Ile Thr Arg Gly
                595                600                605

        Leu Glu Ile Val Arg Arg Asp Trp Ser Glu Ile Ala Lys Glu Thr Gln
            610                615                620

        Ala Arg Val Leu Glu Thr Ile Leu Lys His Gly Asp Val Glu Glu Ala
        625                630                635                640

        Val Arg Ile Val Lys Glu Val Ile Gln Lys Leu Ala Asn Tyr Glu Ile
                645                650                655

        Pro Pro Glu Lys Leu Ala Ile Tyr Glu Gln Ile Thr Arg Pro Leu His
                660                665                670

        Glu Tyr Lys Ala Ile Gly Pro His Val Ala Val Ala Lys Lys Leu Ala
```

675 680 685

Ala Lys Gly Val Lys Ile Lys Pro Gly Met Val Ile Gly Tyr Ile Val
690 695 700

Leu Arg Gly Asp Gly Pro Ile Ser Asn Arg Ala Ile Pro Ala Glu Glu
705 710 715 720

Tyr Asp Pro Lys Lys His Lys Tyr Asp Ala Glu Tyr Tyr Ile Glu Asn
725 730 735

Gln Val Leu Pro Ala Val Leu Arg Ile Leu Glu Gly Phe Gly Tyr Arg
740 745 750

Lys Glu Asp Leu Arg Tyr Gln Lys Thr Arg Gln Val Gly Leu Thr Ser
755 760 765

Trp Leu Asn Ile Lys Lys Ser
770 775

<210> 8
<211> 49
<212> DNA
<213> Artificial Sequence

<220>
<223> Description of Artificial Sequence: synthetic DNA

<400> 8
agaggcgatg gtcgaattcg cgatagggca attccagctg aggaatacg          49

<210> 9
<211> 49
<212> DNA
<213> Artificial Sequence

<220>
<223> Description of Artificial Sequence: synthetic DNA

<400> 9
cgtattcctc agctggaatt gccctatcgc gaattcgacc atcgcctct          49

<210> 10
<211> 40
<212> DNA
<213> Artificial Sequence

<220>
<223> Description of Artificial Sequence: synthetic DNA

<400> 10
ccaattagca atagggcaat tccagctgag gaatacgatc          40

<210> 11
<211> 40
<212> DNA
<213> Artificial Sequence

<220>
<223> Description of Artificial Sequence: synthetic DNA

<400> 11
gatcgtattc ctcagctgga attgccctat tgctaattgg        40